(19) Europäisches Patentamt
European Patent Office
Office européen des brevets

(11) EP 3 285 776 B1

(12) EUROPEAN PATENT SPECIFICATION

(45) Date of publication and mention of the grant of the patent:
**21.12.2022 Bulletin 2022/51**

(21) Application number: **16783764.0**

(22) Date of filing: **20.04.2016**

(51) International Patent Classification (IPC):
***A61K 31/69** (2006.01)* ***A61K 31/407** (2006.01)*
***A61P 31/04** (2006.01)* ***A61K 9/00** (2006.01)*
***A61K 45/06** (2006.01)*

(52) Cooperative Patent Classification (CPC):
(C-Sets available)
**A61K 31/69; A61K 31/407; A61P 31/04;**
A61K 45/06 (Cont.)

(86) International application number:
**PCT/US2016/028437**

(87) International publication number:
**WO 2016/172208 (27.10.2016 Gazette 2016/43)**

(54) **DOSAGE REGIMEN OF VABORBACTAM AND MEROPENEM FOR TREATING BACTERIAL INFECTIONS IN SUBJECTS WITH REDUCED RENAL FUNCTION**

DOSIERUNGSREGIME VON VABORBACTAM UND MEROPENEM ZUR BEHANDLUNG BAKTERIELLER INFEKTIONEN BEI PERSONEN MIT REDUZIERTER NIERENFUNKTION

POSOLOGIE DU VABORBACTAM ET DU MEROPENEM POUR LE TRAITEMENT DES INFECTIONS BACTÉRIENNES CHEZ LES SUJETS AYANT UNE FONCTION RÉNALE RÉDUITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2015 US 201562152668 P**

(43) Date of publication of application:
**28.02.2018 Bulletin 2018/09**

(73) Proprietor: **Melinta Therapeutics, Inc.**
**Morristown, NJ 07960 (US)**

(72) Inventors:
- **GRIFFITH, David, C.**
  **San Marcos, CA 92069 (US)**
- **DUDLEY, Michael, N.**
  **San Diego, CA 92127 (US)**
- **LOUTIT, Jeffrey, S.**
  **Los Altos, CA 94024 (US)**
- **LOMOVSKAYA, Olga**
  **Mountain View, CA 94040 (US)**

(74) Representative: **Bassil, Nicholas Charles et al**
**Kilburn & Strode LLP**
**Lacon London**
**84 Theobalds Road**
**London WC1X 8NL (GB)**

(56) References cited:
**WO-A1-2013/184845**

- **D Griffith D ET AL: "Abstract: A Phase 1 Study of the Safety, Tolerability, and Pharmacokinetics of the Beta-lactamase inhibitor RPX7009 Alone, Meropenem Alone, and both in Combination (Carbavance) TID for 7 days in Healthy Adult Subjects (IDWeek 2014)", , 9 October 2014 (2014-10-09), pages F-560, XP055534351, 54th Interscience Conference on Antimicrobial Agents and Chemotherapy (ICAAC); Washington, DC, USA; September 05 -09, 2014 Retrieved from the Internet: URL:https://idsa.confex.com/idsa/2014/webprogram/Paper48056.html [retrieved on 2018-12-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 285 776 B1

- **KUTI J L ET AL: "Optimal cefepime and meropenem dosing for ventilator-associated pneumonia patients with reduced renal function: An update to our clinical pathway", JOURNAL OF CRITICAL CARE, GRUNE AND STRATTON, ORLANDO, FL, US, vol. 25, no. 1, 1 March 2010 (2010-03-01), pages 155-156, XP026923027, ISSN: 0883-9441, DOI: 10.1016/J.JCRC.2010.01.001 [retrieved on 2010-02-25]**
- **YAGI YUSUKE ET AL: "Outcome evaluation of an intervention to improve the effective and safe use of meropenem", INTERNATIONAL JOURNAL OF CLINICAL PHARMACY, SPRINGER NETHERLANDS, DORDRECHT, vol. 36, no. 3, 20 April 2014 (2014-04-20) , pages 648-656, XP035315830, ISSN: 2210-7703, DOI: 10.1007/S11096-014-9949-2 [retrieved on 2014-04-20]**
- **'A Phase 3, Multi-Center, Randomized, Open-Label Study of Carbavance (Meropenem/RPX7009) Versus Best Available Therapy in Subjects With Selected Serious Infections Due to Carbapenem-Resistant Enterobacteriaceae' CLINICAL TRIAL NCT02168946, [Online] 06 October 2014, XP055325228 Retrieved from the Internet: <URL:https://clinicaltrials.gov/archive/NCT 02168946/2014_10_06> [retrieved on 2016-05-20]**
- **HECKER, S.J. ET AL.: 'Discovery of a Cyclic Boronic Acid beta-Lactamase Inhibitor (RPX7009) with Utility vs Class A Serine Carbapenemases' JOURNAL OF MEDICINAL CHEMISTRY vol. 58, no. 9, 2015, pages 3682 - 3692, XP055325230**
- **SABET, M. ET AL.: 'In Vivo Efficacy of Carbavance (meropenem/RPX7009) against KPC-producing Enterobacteriaceae' ABSTRACTS OF THE INTERSCIENCE CONFERENCE ON ANTIMICROBIAL AGENTS AND CHEMOTHERAPY vol. 54, 2014, page F-958, XP009506892**
- **LAPUEBLA, A. ET AL.: 'Activity of Meropenem Combined with RPX7009, a Novel beta-Lactamase Inhibitor, against Gram-Negative Clinical Isolates in New York City' ANTIMICROBIAL AGENTS AND CHEMOTHERAPY vol. 59, no. 8, August 2015, pages 4856 - 4860, XP055325282**


(52) Cooperative Patent Classification (CPC): (Cont.)

C-Sets
**A61K 31/407, A61K 2300/00;**
**A61K 31/69, A61K 2300/00**

## Description

BACKGROUND

Field

Embodiments of the present application relate to antimicrobial compounds, compositions, their use and preparation as therapeutic agents.

Antibiotics have been effective tools in the treatment of infectious diseases during the last half-century. From the development of antibiotic therapy to the late 1980s there was almost complete control over bacterial infections in developed countries. However, in response to the pressure of antibiotic usage, multiple resistance mechanisms have become widespread and are threatening the clinical utility of anti-bacterial therapy. The increase in antibiotic resistant strains has been particularly common in major hospitals and care centers. The consequences of the increase in resistant strains include higher morbidity and mortality, longer patient hospitalization, and an increase in treatment costs.

Various bacteria have evolved β-lactam deactivating enzymes, namely, β-lactamases, that counter the efficacy of the various β-lactams. β-lactamases can be grouped into 4 classes based on their amino acid sequences, namely, Ambler classes A, B, C, and D. Enzymes in classes A, C, and D include active-site serine β-lactamases, and class B enzymes, which are encountered less frequently, are Zn-dependent. These enzymes catalyze the chemical degradation of β-lactam antibiotics, rendering them inactive. Some β-lactamases can be transferred within and between various bacterial strains and species. The rapid spread of bacterial resistance and the evolution of multi-resistant strains severely limits β-lactam treatment options available.

The increase of class D β-lactamase-expressing bacterium strains such as *Acinetobacter baumannii* has become an emerging multidrug-resistant threat. *A. baumannii* strains express A, C, and D class β-lactamases. The class D β-lactamases such as the OXA families are particularly effective at destroying carbapenem type β-lactam antibiotics, e.g., imipenem, the active carbapenems component of Merck's Primaxin® (Montefour, K.; et al. Crit. Care Nurse 2008, 28, 15; Perez, F. et al. Expert Rev. Anti Infect. Ther. 2008, 6, 269; Bou, G.; Martinez-Beltran, J. Antimicrob. Agents Chemother. 2000, 40, 428. 2006, 50, 2280; Bou, G. et al, J. Antimicrob. Agents Chemother. 2000, 44, 1556). This has imposed a pressing threat to the effective use of drugs in that category to treat and prevent bacterial infections. Indeed the number of catalogued serine-based β-lactamases has exploded from less than ten in the 1970s to over 300 variants. These issues fostered the development of five "generations" of cephalosporins. When initially released into clinical practice, extended-spectrum cephalosporins resisted hydrolysis by the prevalent class A β-lactamases, TEM-1 and SHV-1. However, the development of resistant strains by the evolution of single amino acid substitutions in TEM-1 and SHV-1 resulted in the emergence of the extended-spectrum β-lactamase (ESBL) phenotype.

New β-lactamases have recently evolved that hydrolyze the carbapenem class of antimicrobials, including imipenem, biapenem, doripenem, meropenem, and ertapenem, as well as other β-lactam antibiotics. These carbapenemases belong to molecular classes A, B, and D. Class A carbapenemases of the KPC-type predominantly in *Klebsiella pneumoniae* but now also reported in other *Enterobacteriaceae, Pseudomonas aeruginosa* and *Acinetobacter baumannii.* The KPC carbapenemase was first described in 1996 in North Carolina, but since then has disseminated widely in the US. It has been particularly problematic in the New York City area, where several reports of spread within major hospitals and patient morbidity have been reported. These enzymes have also been recently reported in France, Greece, Sweden, United Kingdom, and an outbreak in Germany has recently been reported. Treatment of resistant strains with carbapenems can be associated with poor outcomes.

Another mechanism of β-lactamase mediated resistance to carbapenems involves combination of permeability or efflux mechanisms combined with hyper production of beta-lactamases. One example is the loss of a porin combined in hyperproduction of ampC beta-lactamase results in resistance to imipenem in *Pseudomonas aeruginosa.* Efflux pump over expression combined with hyperproduction of the ampC β-lactamase can also result in resistance to a carbapenem such as meropenem.

Because there are three major molecular classes of serine-based β-lactamases, and each of these classes contains significant numbers of β-lactamase variants, inhibition of one or a small number of β-lactamases is unlikely to be of therapeutic value. Legacy β-lactamase inhibitors are largely ineffective against at least Class A carbapenemases, against the chromosomal and plasmid-mediated Class C cephalosporinases and against many of the Class D oxacillinases. Therefore, there is a need for improved β-lactamase inhibitors combination therapy.

Cited Documents

Griffith D et al, 54th Interscience Conference on Antimicrobial Agents and Chemotherapy (ICAAC); Washington, DC, USA (2014) discloses "A Phase 1 Study of the Safety, Tolerability, and Pharmacokinetics of the Beta-lactamase inhibitor RPX7009 Alone, Meropenem Alone, and both in Combination (Carbavance) TID for 7 days in Healthy Adult

Subjects".

Kuti J L et al, J. of Critical Care (2010) 25, 155-156 discloses "Optimal cefepime and meropenem dosing for ventilator-associated pneumonia patients with reduced renal function: An update to our clinical pathway".

Yagi Yusuke et al, J. Clin. Pharm. (2014) 36, 648-656 discloses "Outcome evaluation of an intervention to improve the effective and safe use of meropenem".

SUMMARY

The present invention is defined in the appended claims.

Some embodiments described herein relate to Compound I or a pharmaceutically acceptable salt thereof for use in a method for treating or ameliorating a bacterial infection in a subject suffering from reduced renal function in combination with meropenem:

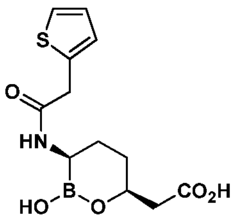
(Compound I)

wherein Compound I or the pharmaceutically acceptable salt thereof and meropenem are administered after a dialysis session and wherein:

(i) the subject has a creatinine clearance level of equal or greater than 30 ml/min and less than 50 ml/min, and wherein the amount of Compound I or the pharmaceutically acceptable salt thereof is about 1.0 g and the amount of meropenem is about 1.0 g, and wherein Compound I or the pharmaceutically acceptable salt thereof and meropenem are administered every 8 hours (q8h); or,
(ii) the subject has a creatinine clearance level of equal or greater than 20 ml/min and less than 30 ml/min, and wherein the amount of Compound I or the pharmaceutically acceptable salt thereof is about 1.0 g and the amount of meropenem is about 1.0 g, and wherein Compound I or the pharmaceutically acceptable salt thereof and meropenem are administered every 12 hours (q12h); or
(iii) the subject has a creatinine clearance of equal or greater than 10 ml/min and less than 20 ml/min, and wherein the amount of Compound I or the pharmaceutically acceptable salt thereof is about 0.5 g and the amount of meropenem is about 0.5 g, and wherein Compound I or the pharmaceutically acceptable salt thereof and meropenem are administered every 12 hours (q12h); or
(iv) the subject has a creatinine clearance of less than 10 ml/min, and wherein the amount of Compound I or the pharmaceutically acceptable salt thereof is about 0.5 g and the amount of meropenem is about 0.5 g, and wherein Compound I or the pharmaceutically acceptable salt thereof and meropenem are administered every 24 hours (q24h).

In some embodiments the subject is suffering from a lower respiratory tract infection.

In some embodiments, the method further comprises administering an additional medicament selected from an antibacterial agent, antifungal agent, an antiviral agent, an anti-inflammatory agent, or an anti-allergic agent.

In some embodiments, the subject treated by the method described above is a mammal. In some further embodiments, the subject is a human.

BRIEF DESCRIPTION OF THE DRAWINGS

**FIG. 1** is a graph depicting the plasma concentration profile (mg/L) of various doses of Compound I as a function of time following a single IV infusion in healthy subjects in the study disclosed in Example 1.
**FIG. 2** is a graph depicting Compound I dose versus AUC (hr*mg/L) following single or multiple doses in healthy subjects in the study disclosed in Example 1.
**FIG. 3** is a graph depicting the plasma concentration profile (mg/L) of Compound I alone and in combination with meropenem after 3-hour infusions in healthy adult subjects in the study disclosed in Example 2.
**FIG. 4** is a graph depicting the plasma concentration profile (mg/L) of meropenem alone and in combination with

Compound I after 3-hour infusions in healthy adult subjects in the study disclosed in Example 2.

**FIG. 5** is a graph depicting the plasma concentration profile (mg/L) of Compound I alone and in combination with meropenem after single and 7 days of TID (i.e., three times a day) dosing by 3-hour infusions in healthy adult subjects in the study disclosed in Example 3.

**FIG. 6** is a graph depicting the plasma concentration profile (mg/L) of meropenem alone and in combination with Compound I after single and 7 days of TID (i.e., three times a day) dosing by 3-hour infusions in healthy adult subjects in the study disclosed in Example 3.

**FIG. 7** is a graph depicting the plasma concentration profile (mg/L) of 2 g Compound I alone and in combination with 2 g meropenem following single and multiple doses by 3-hour infusion in healthy subjects in the study disclosed in Example 4.

**FIG. 8** is a graph depicting the plasma concentration profile (mg/L) of 2 g Compound I alone and in combination with 2 g meropenem following single and multiple doses by 1-hour infusion in healthy subjects in the study disclosed in Example 4.

**FIG. 9** is a graph depicting the mean plasma concentration profile (mg/L) of Compound I after 1-hour or 3-hour infusions of 2 g Compound I in combination with 2 g meropenem in healthy subjects in the study disclosed in Example 4.

**FIG. 10** is a graph depicting the plasma concentration profile (mg/L) of 2 g meropenem alone and in combination with 2 g Compound I following single and multiple doses by 3-hour infusion in healthy subjects in the study disclosed in Example 4.

**FIG. 11** is a graph depicting the plasma concentration profile (mg/L) of 2 g meropenem alone and in combination with 2 g Compound I following single and multiple doses by 1-hour infusion in healthy subjects in the study disclosed in Example 4.

**FIG. 12** is a graph depicting the mean plasma concentration profile (mg/L) of meropenem I after 1-hour or 3-hour infusions of 2 g Compound I in combination with 2 g meropenem in healthy subjects in the study disclosed in Example 4.

**FIG. 13** is a graph depicting the plasma concentration profile (mg/L) of meropenem open-lactam after 1-hour infusion of 2 g meropenem alone and in combination with 2 g Compound I in healthy subjects in the study disclosed in Example 4.

**FIG. 14** is a graph depicting the mean plasma concentration profile (mg/L) of meropenem open-lactam after 1-hour or 3-hour infusions of 2 g meropenem in combination with 2 g Compound I in healthy subjects in the study disclosed in Example 4.

**FIG. 15** is a graph depicting the combination of 1g Compound I and 1 g meropenem clearance according to creatinine clearance in subjects with varying degrees of renal impairment in the study disclosed in Example 5.

**FIG. 16** is a graph depicting the mean plasma concentration profile (μg/mL) of meropenem before and after the start of the third meropenem 2g infusion over 3 hours in the study disclosed in Example 6.

**FIG. 17** is a graph depicting the mean plasma concentration profile (μg/mL) of Compound I before and after the start of the third Compound I 2g infusion over 3 hours in the study disclosed in Example 6.

**FIG. 18** is a graph depicting the mean plasma and epithelial lining fluid (ELF) concentration profile (μg/mL) of meropenem at time of bronchoscopy with bronchoalveolar lavage (BAL) (meropenem 2 g dose infused over 3 hours) in the study disclose in Example 6.

**FIG. 19** is a graph depicting the mean plasma and epithelial lining fluid (ELF) concentration profile (μg/mL) of Compound I at time of bronchoscopy with bronchoalveolar lavage (BAL) (Compound I 2 g dose infused over 3 hours) in the study disclosed in Example 6.

**FIG. 20** is a graph depicting the mean plasma concentration profile (μg/mL) of Compound I and meropenem before and after the start of the third meropenem 2g/Compound I 2g infusion over 3 hours in the study disclosed in Example 6.

**FIG. 21** is a graph depicting the mean epithelial lining fluid (ELF) concentration profile (μg/mL) of Compound I and meropenem at time of bronchoscopy with bronchoalveolar lavage (BAL) (meropenem 2 g dose infused over 3 hours) in the study disclosed in Example 6.

**FIG. 22** is a graph depicting the activity of 1g meropenem /1g Compound I administered by 3-hour infusion every 8 hours against certain strains of Carbapenem Resistant *K. pneumoniae* in an *in vitro* Hollow Fiber Model.

**FIG. 23** is a graph depicting the activity of 1g meropenem /1g Compound I administered by 3-hour infusion every 8 hours against certain strains of Carbapenem Resistant *K. pneumoniae* in an *in vitro* Hollow Fiber Model.

**FIG. 24** is a graph depicting the activity of 2g meropenem /2g Compound I administered by 3-hour infusion every 8 hours against certain strains of Carbapenem Resistant *K. pneumoniae* in an *in vitro* Hollow Fiber Model.

**FIG. 25** is a graph depicting the activity of 1g meropenem /1g Compound I administered by 3-hour infusion every 8 hours against certain *P. aeruginosa* strains in an *in vitro* Hollow Fiber Model.

**FIG. 26** is a graph depicting the representative pharmacokinetic profiles of 2g meropenem and 2g Compound I administered every 8 hours by 3-hour infusion in an *in vitro* Hollow Fiber Model.

**FIG. 27** is a graph depicting the activity of 2g meropenem administered every 8 hours by 3-hour infusion against certain *P. aeruginosa* strains in an *in vitro* Hollow Fiber Model.

**FIG. 28** is a graph depicting the activity of 2g meropenem /2g Compound I administered by 3-hour infusion every 8 hours against certain *P. aeruginosa* strains in an *in vitro* Hollow Fiber Model.

## DETAILED DESCRIPTION OF EMBODIMENTS

### Definitions

The term "agent" or "test agent" includes any substance, molecule, element, compound, entity, or a combination thereof. It includes, but is not limited to, e.g., protein, polypeptide, peptide or mimetic, small organic molecule, polysaccharide, polynucleotide, and the like. It can be a natural product, a synthetic compound, or a chemical compound, or a combination of two or more substances. Unless otherwise specified, the terms "agent", "substance", and "compound" are used interchangeably herein.

The term "mammal" is used in its usual biological sense. Thus, it specifically includes humans, cattle, horses, dogs, cats, rats and mice but also includes many other species.

The term "microbial infection" refers to the invasion of the host organism, whether the organism is a vertebrate, invertebrate, fish, plant, bird, or mammal, by pathogenic microbes. This includes the excessive growth of microbes that are normally present in or on the body of a mammal or other organism. More generally, a microbial infection can be any situation in which the presence of a microbial population(s) is damaging to a host mammal. Thus, a mammal is "suffering" from a microbial infection when excessive numbers of a microbial population are present in or on a mammal's body, or when the effects of the presence of a microbial population(s) is damaging the cells or other tissue of a mammal. Specifically, this description applies to a bacterial infection. Note that the compounds of preferred embodiments are also useful in treating microbial growth or contamination of cell cultures or other media, or inanimate surfaces or objects, and nothing herein should limit the preferred embodiments only to treatment of higher organisms, except when explicitly so specified in the claims.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions. In addition, various adjuvants such as are commonly used in the art may be included. These and other such compounds are described in the literature, e.g., in the Merck Index, Merck & Company, Rahway, NJ. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

The term "pharmaceutically acceptable salt" refers to salts that retain the biological effectiveness and properties of the compounds of the preferred embodiments and, which are not biologically or otherwise undesirable. In many cases, the compounds of the preferred embodiments are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in WO 87/05297, Johnston et al., published September 11, 1987.

"Solvate" refers to the compound formed by the interaction of a solvent and an EPI, a metabolite, or salt thereof. Suitable solvates are pharmaceutically acceptable solvates including hydrates.

"Subject" as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate.

A therapeutic effect relieves, to some extent, one or more of the symptoms of the infection, and includes curing an infection. "Curing" means that the symptoms of active infection are eliminated, including the elimination of excessive members of viable microbe of those involved in the infection. However, certain long-term or permanent effects of the

infection may exist even after a cure is obtained (such as extensive tissue damage).

"Treat," "treatment," or "treating," as used herein refers to administering a pharmaceutical composition for prophylactic and/or therapeutic purposes. The term "prophylactic treatment" refers to treating a patient who is not yet infected, but who is susceptible to, or otherwise at risk of, a particular infection, whereby the treatment reduces the likelihood that the patient will develop an infection. The term "therapeutic treatment" refers to administering treatment to a patient already suffering from an infection.

Methods of Treatment

The technical information set out below may in some respects go beyond the scope of the presently claimed invention, which is defined by the appended claims. The additional technical information is provided to place the actual invention in a broader technical context and to illustrate possible related technical developments.

Some embodiments described herein relate to Compound I or a pharmaceutically acceptable salt thereof for use in a method for treating a bacterial infection in combination with meropenem, comprising administering an effective amount of Compound I or a pharmaceutically acceptable salt thereof and meropenem to a subject in need thereof:

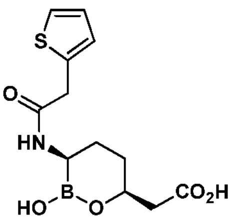
(Compound I)

wherein the amount of Compound I or the pharmaceutically acceptable salt thereof is from about 1.0 g to about 3.0 g and the amount of meropenem is from about 1.0 g to about 3.0 g.

In some embodiments, the amount of Compound I or the pharmaceutically acceptable salt thereof is about 2.0 g. In some embodiments, the amount of meropenem is about 2.0 g. In some embodiments, the amount of both Compound I or the pharmaceutically acceptable salt thereof and meropenem are about 2.0 g.

In some embodiments, Compound I or the pharmaceutically acceptable salt thereof and meropenem are administered at least once per day. In some embodiments, Compound I or the pharmaceutically acceptable salt thereof and meropenem are administered 3 times per day. In some further embodiments, the daily dose of Compound I or the pharmaceutically acceptable salt thereof is about 6.0 g and wherein the daily dose of meropenem is about 6.0 g.

In some embodiments, the administration is by intravenous infusion. In some such embodiments, the intravenous infusion is completed in about 1 to about 5 hours. In some further embodiments, the intravenous infusion is completed is about 3 hours.

In some embodiments, Compound I or the pharmaceutically acceptable salt thereof is administered prior or subsequent to meropenem. In some other embodiments, Compound I or the pharmaceutically acceptable salt thereof and meropenem are in a single dosage form. In some embodiments, the single dosage form further comprises a pharmaceutically acceptable excipient, diluent, or carrier.

Subjects with Reduced Renal Function

Some embodiments described herein relate to compound I or a pharmaceutically acceptable salt thereof for use in a method for treating a bacterial infection in combination with meropenem, comprising selecting for treatment a subject in need for treatment of a bacterial infection who is suffering from reduced renal function; administering an effective amount of compound I or a pharmaceutically acceptable salt thereof and meropenem to said subject. In some embodiments, said subject has a creatinine clearance of ≥ 30 ml/min and < 50 ml/min. In some embodiments, said subject has a creatinine clearance of ≥ 20 ml/min and < 30 ml/min. In some embodiments, said subject has a creatinine clearance of ≥ 10 ml/min and < 20 ml/min. In some embodiments, said subject has a creatinine clearance of < 10 ml/min. In some embodiments, the bacterial infection is lower respiratory tract infection.

In some embodiments, Compound I or the pharmaceutically acceptable salt thereof is administered in a dose range from about 250 mg to about 2.0 g. In some further embodiments, Compound I or the pharmaceutically acceptable salt thereof is administered in a dose of about 500 mg to about 1.0 g. In some such embodiments, Compound I or the pharmaceutically acceptable salt thereof is administered in a dose of about 1.0 g. In some other embodiments, Compound I or the pharmaceutically acceptable salt thereof is administered in a dose of about 500 mg. In some embodiments, meropenem is administered in a dose range from about 250 mg to about 2.0 g. In some further embodiments, meropenem

is administered in a dose of about 500 mg to about 1.0 g. In some such embodiments, meropenem is administered in a dose of about 1.0 g. In some other embodiments, meropenem is administered in a dose of about 500 mg. In some further embodiments, both Compound I or the pharmaceutically acceptable salt thereof and meropenem are administered in a dose of about 1.0 g. In some other embodiments, both Compound I or the pharmaceutically acceptable salt thereof and meropenem are administered in a dose of about 500 mg.

In some embodiments, Compound I or the pharmaceutically acceptable salt thereof and meropenem are administered at least once per day (every 24 hours). In some embodiments, Compound I or the pharmaceutically acceptable salt thereof and meropenem are administered 2 times per day (every 12 hours). In some embodiments, Compound I or the pharmaceutically acceptable salt thereof and meropenem are administered 3 times per day (every 8 hours). In some embodiments, the daily dose of Compound I or the pharmaceutically acceptable salt thereof is about 3.0 g and wherein the daily dose of meropenem is about 3.0 g. In some embodiments, the daily dose of Compound I or the pharmaceutically acceptable salt thereof is about 2.0 g and wherein the daily dose of meropenem is about 2.0 g. In some embodiments, the daily dose of Compound I or the pharmaceutically acceptable salt thereof is about 1.0 g and wherein the daily dose of meropenem is about 1.0 g. In some further embodiments, the daily dose of Compound I or the pharmaceutically acceptable salt thereof is about 500 mg and wherein the daily dose of meropenem is about 500 mg.

In some embodiments, the administration is by intravenous infusion. In some such embodiments, the intravenous infusion is completed in about 1 to about 5 hours. In some further embodiments, the intravenous infusion is completed is about 3 hours.

In some embodiments, Compound I or the pharmaceutically acceptable salt thereof is administered prior or subsequent to meropenem. In some other embodiments, Compound I or the pharmaceutically acceptable salt thereof and meropenem are in a single dosage form. In some embodiments, the single dosage form further comprises a pharmaceutically acceptable excipient, diluent, or carrier.

Subjects with Lower Respiratory Tract Infection

Some embodiments described herein relate to Compound I or a pharmaceutically acceptable salt thereof for use in a method of treating or ameliorating a lower respiratory tract infection in combination with meropenem, comprising administering an effective amount of Compound I or a pharmaceutically acceptable salt thereof and meropenem to a subject in need thereof.

In some embodiments, Compound I or the pharmaceutically acceptable salt thereof is administered in a dose range from about 250 mg to about 5.0 g. In some further embodiments, Compound I or the pharmaceutically acceptable salt thereof is administered in a dose range from about 1.0 g to about 3.0 g. In still some further embodiments, the amount of Compound I is about 2.0 g. In some embodiments, meropenem is administered in a dose range from about 250 mg to about 5.0 g. In some further embodiments, meropenem is administered in a dose range from about 1.0 g to about 3.0 g. In still some further embodiments, the amount of meropenem is about 2.0 g. In some embodiments, both Compound I or the pharmaceutically acceptable salt thereof and meropenem are administered in a dose of about 2.0 g.

In some embodiments, Compound I or the pharmaceutically acceptable salt thereof and meropenem are administered at least once per day. In some embodiments, Compound I or the pharmaceutically acceptable salt thereof and meropenem are administered 3 times per day. In some embodiments, the daily dose of Compound I or the pharmaceutically acceptable salt thereof is from about 3.0 g to about 6.0 g and wherein the daily dose of meropenem is from about 3.0 g to about 6.0 g. In some further embodiments, the daily dose of Compound I or the pharmaceutically acceptable salt thereof is about 6.0 g and wherein the daily dose of meropenem is about 6.0 g.

In some embodiments, the administration is by intravenous infusion. In some such embodiments, the intravenous infusion is completed in about 1 to about 5 hours. In some further embodiments, the intravenous infusion is completed is about 3 hours.

In some embodiments, Compound I or the pharmaceutically acceptable salt thereof is administered prior or subsequent to meropenem. In some other embodiments, Compound I or the pharmaceutically acceptable salt thereof and meropenem are in a single dosage form. In some embodiments, the single dosage form further comprises a pharmaceutically acceptable excipient, diluent, or carrier.

In any embodiments of the methods described herein, the method may further comprise administering an additional medicament selected from an antibacterial agent, antifungal agent, an antiviral agent, an anti-inflammatory agent, or an anti-allergic agent.

In some embodiments, the subject treated by the method described above is a mammal. In some further embodiments, the subject is a human.

In any embodiments of the methods described herein, the treatment is for infection caused by carbapenem-resistant *Enterobacteriaceae.*

Indications

The compositions comprising Compound I and a carbapenem compound meropenem described herein can be used to treat bacterial infections. Bacterial infections that can be treated with a combination of Compound I and meropenem can comprise a wide spectrum of bacteria. Example organisms include gram-positive bacteria, gram-negative bacteria, aerobic and anaerobic bacteria, such as *Staphylococcus, Lactobacillus, Streptococcus, Sarcina, Escherichia, Enterobacter, Klebsiella, Pseudomonas, Acinetobacter, Mycobacterium, Proteus, Campylobacter, Citrobacter, Nisseria, Baccillus, Bacteroides, Peptococcus, Clostridium, Salmonella, Shigella, Serratia, Haemophilus, Brucella* and other organisms.

More examples of bacterial infections include *Pseudomonas aeruginosa, Pseudomonas fluorescens, Pseudomonas acidovorans, Pseudomonas alcaligenes, Pseudomonas putida, Stenotrophomonas maltophilia, Burkholderia cepacia, Aeromonas hydrophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Francisella tularensis, Morganella morganii, Proteus mirabilis, Proteus vulgaris, Providencia alcalifaciens, Providencia rettgeri, Providencia stuartii, Acinetobacter baumannii, Acinetobacter calcoaceticus, Acinetobacter haemolyticus, Yersinia enterocolitica, Yersinia pestis, Yersinia pseudotuberculosis, Yersinia intermedia, Bordetella pertussis, Bordetella parapertussis, Bordetella bronchiseptica, Haemophilus influenzae, Haemophilus parainfluenzae, Haemophilus haemolyticus, Haemophilus parahaemolyticus, Haemophilus ducreyi, Pasteurella multocida, Pasteurella haemolytica, Branhamella catarrhalis, Helicobacter pylori, Campylobacter fetus, Campylobacter jejuni, Campylobacter coli, Borrelia burgdorferi, Vibrio cholerae, Vibrio parahaemolyticus, Legionella pneumophila, Listeria monocytogenes, Neisseria gonorrhoeae, Neisseria meningitidis, Kingella, Moraxella, Gardnerella vaginalis, Bacteroides fragilis, Bacteroides distasonis, Bacteroides 3452A homology group, Bacteroides vulgatus, Bacteroides ovalus, Bacteroides thetaiotaomicron, Bacteroides uniformis, Bacteroides eggerthii, Bacteroides splanchnicus, Clostridium difficile, Mycobacterium tuberculosis, Mycobacterium avium, Mycobacterium intracellulare, Mycobacterium leprae, Corynebacterium diphtheriae, Corynebacterium ulcerans, Streptococcus pneumoniae, Streptococcus agalactiae, Streptococcus pyogenes, Enterococcus faecalis, Enterococcus faecium, Staphylococcus aureus, Staphylococcus epidermidis, Staphylococcus saprophyticus, Staphylococcus intermedius, Staphylococcus hyicus subsp. hyicus, Staphylococcus haemolyticus, Staphylococcus hominis,* or *Staphylococcus saccharolyticus.*

In some embodiments, the infection is caused by a bacteria selected from *Pseudomonas aeruginosa, Pseudomonas fluorescens, Stenotrophomonas maltophilia, Escherichia coli, Citrobacter freundii, Salmonella typhimurium, Salmonella typhi, Salmonella paratyphi, Salmonella enteritidis, Shigella dysenteriae, Shigella flexneri, Shigella sonnei, Enterobacter cloacae, Enterobacter aerogenes, Klebsiella pneumoniae, Klebsiella oxytoca, Serratia marcescens, Acinetobacter calcoaceticus, Acinetobacter haemolyticus, Yersinia enterocolitica, Yersinia pestis, Yersinia pseudotuberculosis, Yersinia intermedia, Haemophilus influenzae, Haemophilus parainfluenzae, Haemophilus haemolyticus, Haemophilus parahaemolyticus, Helicobacter pylori, Campylobacter fetus, Campylobacter jejuni, Campylobacter coli, Vibrio cholerae, Vibrio parahaemolyticus, Legionella pneumophila, Listeria monocytogenes, Neisseria gonorrhoeae, Neisseria meningitidis, Moraxella, Bacteroides fragilis, Bacteroides vulgatus, Bacteroides ovalus, Bacteroides thetaiotaomicron, Bacteroides uniformis, Bacteroides eggerthii, or Bacteroides splanchnicus.*

In some embodiments, the bacterial infection is gram-negative infection. In some embodiments, the bacterial infection is lower respiratory tract infection. In some embodiments, the bacterial infection is caused by *Pseudomonas aeruginosa.* In some embodiments, the bacterial infection is caused by *Klebsiella pneumonia.*

Antibacterial Compounds

Compound I has the structures shown as follows:

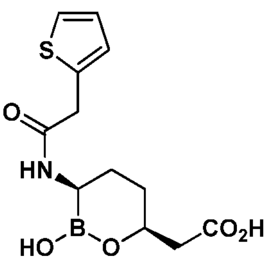

In some embodiments, due to the facile exchange of boron esters, Compound I may convert to or exist in

equilibrium with alternate forms. Accordingly, in some embodiments, Compound I may exist in combination with one or more of these forms. For example, Compound I may exist in combination with one or more open-chain form (Formula Ia), dimeric form (Formula Ib), cyclic dimeric form (Formula Ic), trimeric form (Formula Id), cyclic trimeric form (Formula Ie), and the like. Compound I and its enantiomer, diastereoisomer or tautomer, or pharmaceutically acceptable salt is described in U.S. Patent No. 8,680,136.

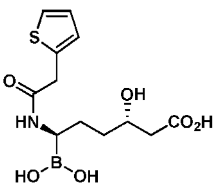

Ia

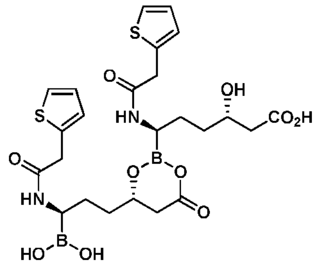

Ib

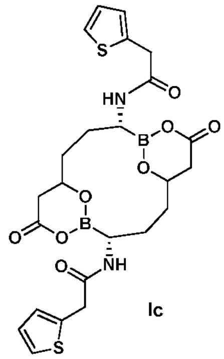

Ic

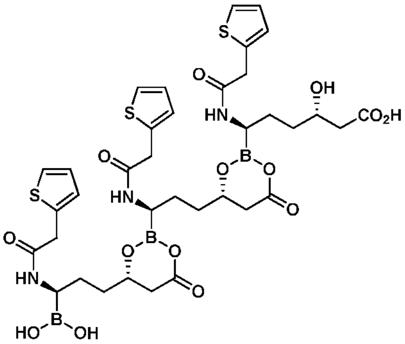

Id

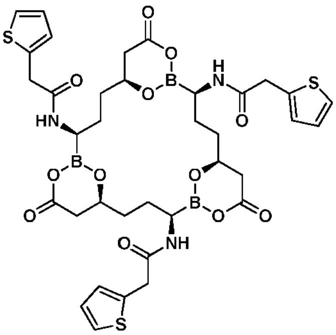

Ie

Meropenem is an ultra-broad-spectrum injectable antibiotic used to treat a wide variety of infections. It is a β-lactam and belongs to the subgroup of carbapenem. It has the structure shown as follows:

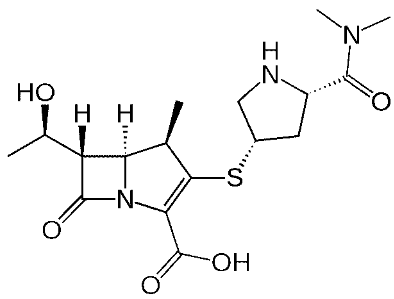

Some embodiments include Compound I for use in methods for treating or preventing a bacterial infection comprising administering to a subject in need thereof, an effective amount of Compound I and meropenem, wherein Compound I can be in any one of the forms described above or a combination thereof.

Some embodiments further comprise administering an additional medicament, either is a separate composition or in the same composition. In some embodiments, the additional medicament includes an antibacterial agent, antifungal agent, an antiviral agent, an anti-inflammatory agent or an anti-allergic agent. In some embodiments, the additional medicament comprises an antibacterial agent such as an additional (3-lactam.

In some embodiments, the additional β-lactam includes Amoxicillin, Ampicillin (Pivampicillin, Hetacillin, Bacampicillin, Metampicillin, Talampicillin), Epicillin, Carbenicillin (Carindacillin), Ticarcillin, Temocillin, Azlocillin, Piperacillin, Mezlocillin, Mecillinam (Pivmecillinam), Sulbenicillin, Benzylpenicillin (G), Clometocillin, Benzathine benzylpenicillin, Procaine benzylpenicillin, Azidocillin, Penamecillin, Phenoxymethylpenicillin (V), Propicillin, Benzathine phenoxymethylpenicillin, Pheneticillin, Cloxacillin (Dicloxacillin, Flucloxacillin), Oxacillin, Meticillin, Nafcillin, Faropenem, Biapenem,

Doripenem, Ertapenem, Imipenem, Panipenem, Tomopenem, Razupenem, Tebipenem, Sulopenem, Cefazolin, Cefacetrile, Cefadroxil, Cefalexin, Cefaloglycin, Cefalonium, Cefaloridine, Cefalotin, Cefapirin, Cefatrizine, Cefazedone, Cefazaflur, Cefradine, Cefroxadine, Ceftezole, Cefaclor, Cefamandole, Cefminox, Cefonicid, Ceforanide, Cefotiam, Cefprozil, Cefbuperazone, Cefuroxime, Cefuzonam, Cefoxitin, Cefotetan, Cefmetazole, Loracarbef, Cefixime, Ceftazidime, Ceftriaxone, Cefcapene, Cefdaloxime, Cefdinir, Cefditoren, Cefetamet, Cefmenoxime, Cefodizime, Cefoperazone, Cefotaxime, Cefpimizole, Cefpiramide, Cefpodoxime, Cefsulodin, Cefteram, Ceftibuten, Ceftiolene, Ceftizoxime, Flomoxef, Latamoxef, Cefepime, Cefozopran, Cefpirome, Cefquinome, Ceftobiprole, Ceftaroline, Ceftolozane, CXA-101, RWJ-54428, MC-04,546, ME1036, BAL30072, SYN 2416, Ceftiofur, Cefquinome, Cefovecin, Aztreonam, Tigemonam, Carumonam, RWJ-442831, RWJ-333441, RWJ-333442, S649266, GSK3342830, and AIC 499.

In some embodiments, the additional β-lactam includes Ceftazidime, Doripenem, Ertapenem, Imipenem, or Panipenem.

Some embodiments include a pharmaceutical composition comprising a therapeutically effective amount of any one of the foregoing compounds and a pharmaceutically acceptable excipient.

Administration and Pharmaceutical Compositions

Some embodiments include pharmaceutical compositions comprising: (a) a safe and therapeutically effective amount of compound I, or its corresponding enantiomer, diastereoisomer or tautomer, or pharmaceutically acceptable salt; (b) meropenem, and (c) a pharmaceutically acceptable carrier.

Compound I and meropenem are administered at a therapeutically effective dosage, e.g., a dosage sufficient to provide treatment for the disease states previously described. In some embodiments, a single dose of Compound I and meropenem may range from about 250 mg to about 5000 mg or from about 1000 mg to about 3000 mg. In some embodiments, Compound I and meropenem can be administered at least once a day, for example 1 to 5 times a day.

Administration of the combination comprising Compound I or its corresponding enantiomer, diastereoisomer, tautomer, or the pharmaceutically acceptable salt thereof and meropenem can be via any of the accepted modes of administration for agents that serve similar utilities including, but not limited to, orally, subcutaneously, intravenously, intranasally, topically, transdermally, intraperitoneally, intramuscularly, intrapulmonarilly, vaginally, rectally, or intraocularly. Intravenous, oral and parenteral administrations are customary in treating the indications that are the subject of the preferred embodiments.

Compound I and meropenem can be formulated into pharmaceutical compositions for use in treatment of these conditions. Standard pharmaceutical formulation techniques are used, such as those disclosed in Remington's The Science and Practice of Pharmacy, 21st Ed., Lippincott Williams & Wilkins (2005).

In addition to Compound I and meropenem, some embodiments include compositions containing a pharmaceutically-acceptable carrier. The term "pharmaceutically-acceptable carrier", as used herein, means one or more compatible solid or liquid filler diluents or encapsulating substances, which are suitable for administration to a mammal. The term "compatible", as used herein, means that the components of the composition are capable of being commingled with the subject compound, and with each other, in a manner such that there is no interaction, which would substantially reduce the pharmaceutical efficacy of the composition under ordinary use situations. Pharmaceutically-acceptable carriers must, of course, be of sufficiently high purity and sufficiently low toxicity to render them suitable for administration preferably to an animal, preferably mammal being treated.

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The choice of a pharmaceutically-acceptable carrier to be used in conjunction with the combination is basically determined by the way the combination is to be administered.

The compositions described herein are preferably provided in unit dosage form. As used herein, a "unit dosage form" is a composition containing an amount of a compound that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, though a single administration is not specifically excluded. The skilled artisan will recognize that the formulation does not specifically contemplate the entire course of therapy and

such decisions are left for those skilled in the art of treatment rather than formulation.

The compositions useful as described above may be in any of a variety of suitable forms for a variety of routes for administration, for example, for oral, nasal, rectal, topical (including transdermal), ocular, intracerebral, intracranial, intrathecal, intra-arterial, intravenous, intramuscular, or other parental routes of administration. The skilled artisan will appreciate that oral and nasal compositions comprise compositions that are administered by inhalation, and made using available methodologies. Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials may be included, which do not substantially interfere with the inhibitory activity of the compound. The amount of carrier employed in conjunction with the compound is sufficient to provide a practical quantity of material for administration per unit dose of the compound. Techniques and compositions for making dosage forms useful in the methods described herein are described in the following references: Modern Pharmaceutics, 4th Ed., Chapters 9 and 10 (Banker & Rhodes, editors, 2002); Lieberman et al., Pharmaceutical Dosage Forms: Tablets (1989); and Ansel, Introduction to Pharmaceutical Dosage Forms 8th Edition (2004). In some embodiments, the pharmaceutical compositions are administered intravenously. In some embodiments, the pharmaceutical compositions are administered orally. In some other embodiments, the pharmaceutical compositions are administered intraperitoneally.

Various oral dosage forms can be used, including such solid forms as tablets, capsules, granules and bulk powders. These oral forms comprise a safe and effective amount, usually at least about 5%, with a maximum of about 90%, of the compound. Tablets can be compressed, tablet triturates, enteric-coated, sugar-coated, film-coated, or multiple-compressed, containing suitable binders, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The pharmaceutically-acceptable carrier suitable for the preparation of unit dosage forms for peroral administration is well-known in the art. Tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Such compositions may also be coated by conventional methods, typically with pH or time-dependent coatings, such that the subject compound is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, Eudragit coatings, waxes and shellac.

Compositions described herein may optionally include other drug actives.

Other compositions useful for attaining systemic delivery of the subject compounds include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

A liquid composition, which is formulated for topical ophthalmic use, is formulated such that it can be administered topically to the eye. The comfort should be maximized as much as possible, although sometimes formulation considerations (e.g. drug stability) may necessitate less than optimal comfort. In the case that comfort cannot be maximized, the liquid should be formulated such that the liquid is tolerable to the patient for topical ophthalmic use. Additionally, an ophthalmically acceptable liquid should either be packaged for single use, or contain a preservative to prevent contamination over multiple uses.

For ophthalmic application, solutions or medicaments are often prepared using a physiological saline solution as a major vehicle. Ophthalmic solutions should preferably be maintained at a comfortable pH with an appropriate buffer system. The formulations may also contain conventional, pharmaceutically acceptable preservatives, stabilizers and surfactants.

Preservatives that may be used in the pharmaceutical compositions disclosed herein include, but are not limited to, benzalkonium chloride, PHMB, chlorobutanol, thimerosal, phenylmercuric, acetate and phenylmercuric nitrate. A useful surfactant is, for example, Tween 80. Likewise, various useful vehicles may be used in the ophthalmic preparations disclosed herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. For many compositions, the pH will be between 4 and 9. Accordingly, buffers include acetate buffers, citrate buffers, phosphate buffers and borate buffers. Acids or bases may be used to adjust the pH of these formulations as needed.

In a similar vein, an ophthalmically acceptable antioxidant includes, but is not limited to, sodium metabisulfite, sodium thiosulfate, acetylcysteine, butylated hydroxyanisole and butylated hydroxytoluene.

Other excipient components, which may be included in the ophthalmic preparations, are chelating agents. A useful chelating agent is edetate disodium, although other chelating agents may also be used in place or in conjunction with it.

For topical use, creams, ointments, gels, solutions or suspensions, etc., containing the compound disclosed herein are employed. Topical formulations may generally be comprised of a pharmaceutical carrier, co-solvent, emulsifier, penetration enhancer, preservative system, and emollient.

For intravenous administration, the compounds and compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HCl, and citric acid. In various embodiments, the pH of the final composition ranges from 2 to 8, or preferably from 4 to 7. Antioxidant excipients may include sodium bisulfite, acetone sodium bisulfite, sodium formaldehyde, sulfoxylate, thiourea, and EDTA. Other non-limiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol.

The resulting composition may be infused into the patient over a period of time. In various embodiments, the infusion time ranges from 5 minutes to continuous infusion, from 10 minutes to 8 hours, from 30 minutes to 4 hours, and from 1 hour to 3 hours. In one embodiment, the drug is infused over a 3 hour period. The infusion may be repeated at the desired dose interval, which may include, for example, 6 hours, 8 hours, 12 hours, or 24 hours.

The compositions for intravenous administration may be provided to caregivers in the form of one more solids that are reconstituted with a suitable diluent such as sterile water, saline or dextrose in water shortly prior to administration. Reconstituted concentrated solutions may be further diluted into a parenteral solutions having a volume of from about 25 to about 1000 ml, from about 30 ml to about 500 ml, or from about 50 ml to about 250 ml. In other embodiments, the compositions are provided in solution ready to administer parenterally. In still other embodiments, the compositions are provided in a solution that is further diluted prior to administration. In embodiments that include administering a combination of a compound described herein and another agent, the combination may be provided to caregivers as a mixture, or the caregivers may mix the two agents prior to administration, or the two agents may be administered separately.

The actual dose of the active compounds described herein depends on the specific compound, and on the condition to be treated; the selection of the appropriate dose is well within the knowledge of the skilled artisan.

## Kits for Intravenous Administration

Some embodiments include a kit comprising Compound I and a carbapenem antibacterial agent Meropenem. In some embodiments, the kits are used for intravenous administration.

In one embodiment, both components are provided in a single sterile container. In the case of solids for reconstitution, the agents may be pre-blended and added to the container simultaneously or may be dry-powder filled into the container in two separate steps. In some embodiments, the solids are sterile crystalline products. In other embodiment,

the solids are lyophiles. In one embodiment, both components are lyophilized together. Non-limiting examples of agents to aid in lyophilization include sodium or potassium phosphates, citric acid, tartaric acid, gelatin, and carbohydrates such as dextrose, mannitol, and dextran. One embodiment includes non-sterile solids that are irradiated either before or after introduction into the container.

In the case of a liquid, the agents may be dissolved or dispersed in a diluent ready for administration. In another embodiment, the solution or dispersion may be further diluted prior to administration. Some embodiments include providing the liquid in an IV bag. The liquid may be frozen to improve stability.

In one embodiment, the container includes other ingredients such as a pH adjuster, a solubilizing agent, or a dispersing agent. Non-limiting examples of pH adjusters include NaOH, sodium carbonate, sodium acetate, HCl, and citric acid.

In an alternative embodiment, the two components may be provided in separate containers. Each container may include a solid, solution, or dispersion. In such embodiments, the two containers may be provided in a single package or may be provided separately. In one embodiment, the compound described herein is provided as a solution while the additional agent (e.g., antibacterial agent) is provided as a solid ready for reconstitution. In one such embodiment, the solution of the compound described herein is used as the diluent to reconstitute the other agent.

In some embodiments, the kit may comprise comprises one or more additional medicaments selected from an antibacterial agent, antifungal agent, an antiviral agent, an anti-inflammatory agent, or an anti-allergic agent. The additional medicaments can be prepared in the same way as described above.

## EXAMPLES

The following examples, including experiments and results achieved, are provided for illustrative purposes only and are not to be construed as limiting the present application.

### Example 1

Example 1 provides a summary of a clinical study of the safety, tolerability and pharmacokinetics of the beta-lactamase inhibitor Compound I in healthy adult subjects.

Methods: 56 healthy subjects were enrolled into one of 7 cohorts of 8 subjects each in the single ascending dose phase (250 mg, 500 mg, 750 mg, 1000 mg, 1250 mg, 1500 mg and 2000 mg). Thirty-two additional subjects were then enrolled into one of 4 cohorts in the multiple-dose phase (250 mg, 1000 mg, 1500 mg, and 2000 mg, given q8h for 7 days). Within each cohort, subjects were randomly assigned to Compound I (n = 6) or normal saline placebo (n = 2). All infusions were administered over 3 hours. Plasma and urine samples were obtained after single or multiple doses and assayed for Compound I content using a validated HPLC/MS method.

Results: Table 1 summarizes mean pharmacokinetics of Compound I in different doses. Compound I concentration profile as a function of time following a single IV fusion and Compound I AUC profile as a function of dose are illustrated in **FIGs. 1** and **2** respectively.

**TABLE 1.**

**Compound I Dose, mg**

| Parameters (Mean ± SD) | 250 | 250 | 500 | 750 | 1000 | 1000 | 1250 | 1500 | 1500 | 2000 | 2000 |
|---|---|---|---|---|---|---|---|---|---|---|---|
| | SD | MD | SD | SD | SD | MD | SD | SD | MD | SD | MD |
| $C_{max}$, μg/mL | 5.03 ±0.86 | 4.81 ±1.04 | 9.97 ±0.95 | 15.30 ±2.76 | 21.80 ±3.83 | 21.30 ±6.63 | 27.80 ±3.67 | 32.90 ±5.77 | 33.40 ±4.48 | 41.60 ±4.75 | 40.90 ±4.68 |
| $T_{max}$, h | 3.02 | 2.25 | 3 | 3 | 3.01 | 3 | 3 | 3.01 | 3 | 3.02 | 2.25 |
| $T_{1/2}$, h ±0.15 | 1.17 | 1.17 ±0.13 | 1.35 ±0.22 | 1.24 ±0.33 | 1.41 ±0.28 | 1.43 ±0.36 | 1.32 ±0.47 | 1.40 ±0.31 | 1.65 ±0.26 | 1.51 ±0.08 | 1.66 ±0.10 |
| h/mL $AUC_{(0-t)}$,μg | 16.2 ±3.17 | 16.30 ±3.56 | 34.50 ±4.87 | 50.60 ±7.51 | 76.70 ±13.20 | 74.60 ±17.90 | 97.20 ±14.80 | 110.00 ±18.90 | 118.00 ±15.30 | 140.00 ±13.50 | 145.00 ±15.80 |
| $AUC_{(0-inf)}$ μg·h/mL | 16.6 ±3.24 | | 35.60 ±5.21 | 51.80 ±8.03 | 79.30 ±14.20 | | 100.00 ±17.40 | 114.00 ±20.00 | | 144.00 ±13.90 | |
| CL, L/h | 15.6 ±2.63 | 15.20 ±2.56 | 14.30 ±2.28 | 14.80 ±2.24 | ±± 13.10 ±2.59 | 14.10 ±3.42 | 12.80 ±2.36 | 13.50 ±2.17 | 12.90 ±1.71 | 14.00 ±1.40 | 14.00 ±1.78 |
| $V_{ss}$, L | 24.5 ±5.81 | | 25.40 ±2.96 | 23.20 ±3.97 | 21.00 ±3.03 | | 20.20 ±2.43 | 23.00± 4.76 | | 21.80 ±2.26 | |
| $V_d$, L | 26.2 ±5.30 | 25.70 ±5.57 | 27.70 ±4.64 | 23.20 ±3.97 | 25.90 ±3.80 | 28.00 ±5.66 | 20.20 ±2.43 | 26.90 ±5.39 | 30.30 ±3.48 | 30.60 ±4.45 | 33.40 ±4.52 |
| CL, L/h/kg | 0.20 ±0.03 | 0.20 ±0.02 | 0.19 ±0.02 | 0.19 ±0.04 | 0.17 ±0.02 | 0.18 ±0.02 | 0.18 ±0.04 | 0.16 ±0.02 | 0.15 ±0.01 | 0.17 ±0.03 | 0.17 ±0.02 |
| $V_{ss}$ , L/kg | 0.31 ±0.06 | | 0.33 ±0.04 | 0.30 ±0.09 | 0.28 ±0.02 | | 0.29± 0.05 | 0.28 ±0.05 | | 0.27 ±0.04 | |
| $V_d$, L/kg | 0.33 ±0.06 | 0.34 ±0.06 | 0.37 ±0.07 | 0.34 ±0.10 | 0.33 ±0.06 | 0.36 ±0.05 | 0.33 ±0.10 | 0.33 ±0.07 | 0.37 ±0.07 | 0.38 ±0.07 | 0.41 ±0.05 |
| $CL_R$, L/h | 12.70 ±2.71 | 12.70 ±3.68 | 11.80 ±1.63 | 13.00 ±2.08 | 12.10± 2.43 | 11.70 ±3.75 | 11.50 ±2.58 | 11.80 ±1.88 | 11.20 ±1.72 | 15.10 ±2.55 | 12.80 ±2.05 |
| Urinary Recovery% | 81.30 ±16.60 | 79.90 ±16.30 | 80.30 ±9.94 | 86.40 ±5.05 | 89.90 ±6.97 | 82.80 ±10.30 | 86.90 ±9.71 | 86.60 ±7.22 | 86.80 ±2.48 | 105.00 ±15.10 | 91.60 ±5.36 |
| $CL_{Non-R}$, L/h | 2.85 ±3.11 | 2.49 ±3.27 | 2.55 ±1.92 | 1.72 ±0.80 | 0.97 ±0.92 | 2.31 ±1.21 | 1.33 ±1.31 | 1.42 ±1.17 | 1.68 ±0.13 | -1.07 ±2.13 | 1.15 ±0.68 |

Maximum concentrations for Compound I were achieved at the end of the 3-hour infusion. Compound I exposure (Cmax and AUC) increased in a dose-proportional manner following single and multiple doses (See **FIGs. 1** and **2**). There was no evidence of accumulation with multiple doses, consistent with the observed terminal half-life (<2 hours). Both the volume of distribution and plasma clearance were independent of dose. High concentrations of **FIGs. 1** and **2** were measured in the urine. Urinary recovery was 80% or greater over 48 hours across all dose groups.

No subjects discontinued the study due to adverse events (AEs) and no serious adverse events (SAEs) were observed. AEs were similar between Compound I and placebo-treated subjects, with no evidence of increasing incidence or severity of AEs with increasing dose, and all AEs were mild or moderate.

Conclusion: Compound I was safe and well tolerated at all doses tested. AUC and Cmax increased proportionally independent of dose.

Example 2

Example 2 provides a summary of a clinical study of the safety, tolerability and pharmacokinetics of the beta-lactamase inhibitor Compound I alone, meropenem alone, and the combination of both in healthy adult subjects.

Methods: Eighty healthy subjects were enrolled into 1 of 5 cohorts in the single ascending dose phase (250 mg, 1000 mg, 1500 mg and 2000 mg Compound I in combination with 1 or 2 g of meropenem). Within each cohort, subjects were administered single doses of either Compound I or meropenem day 1, and Compound I or meropenem day 3. The combination of both drugs was administered on day 7. All drugs were infused over 3 hours. Plasma and urine samples were obtained and assayed using validated HPLC/MS methods. Pharmacokinetics of Compound I alone and in combination with meropenem after 3-hour infusions in healthy adult subjects and pharmacokinetics of meropenem alone and in combination with Compound I after 3-hour infusions in healthy adult subjects are illustrated in **FIGs. 3** and **4** respectively.

Results: Pharmacokinetic parameters, derived using non-compartmental methods, for each drug alone and in combination of Compound I and meropenem are shown below in Table 2 and Table 3. Table 2 summarizes Compound I pharmacokinetic parameters following single dose of Compound I administered alone or in combination with meropenem as 3-hour infusions to healthy volunteers (data are mean ±standard deviation). Table 3 summarizes meropenem pharmacokinetic parameters following single dose of meropenem administered alone or in combination with Compound I as 3-hour infusions to healthy volunteers (data are mean ±standard deviation).

**TABLE 2.**

| Parameter | Compound I 250 mg | | Compound I 1000 mg | | Compound I 1500 mg | | Compound I 2000 mg | | Compound I 2000 mg | |
|---|---|---|---|---|---|---|---|---|---|---|
| | Alone (N=24) | Meropenem 1g (N=8) | Alone (N=5) | Meropenem 1g (N=5) | Alone (N=8) | Meropenem 1g (N=7) | Alone (N=8) | Meropenem 1g (N=8) | Alone (N=8) | Meropenem 2g (N=8) |
| $C_{max}$(mg/L) | 5.20 ±0.92 | 5.34 ±0.78 | 21.98 ±3.54 | 23.68 ±4.38 | 37.23 ±5.33 | 37.14 ±4.70 | 39.20 ±4.29 | 41.44 ±4.38 | 51.44 ±16.16 | 51.66 ±7.26 |
| $AUC_{(0-\infty)}$(mg·h/L) | 17.48 ±3.02 | 17.40 ±2.22 | 77.56 ±15.87 | 81.18 ±15.38 | 123.66 ±18.03 | 127.07 ±20.99 | 133.26 ±20.89 | 141.02 ±21.35 | 159.21 ±44.58 | 170.44 ±31.99 |
| Half-Life (h) | 1.18 ±0.35 | 1.08 ±0.21 | 1.56 ±0.67 | 1.53 ±0.32 | 1.21 ±0.24 | 1.35 ±0.22 | 1.31 ±0.32 | 1.43 ±0.22 | 1.39 ±0.20 | 1.98 ±0.81 |
| Vss (L) | 23.15 ±6.00 | 22.25 ±3.02 | 21.44 ±5.22 | 20.25 ±3.20 | 19.37 ±5.14 | 19.83 ±2.84 | 22.02 ±2.24 | 22.43 ±2.00 | 21.37 ±3.33 | 21.84 ±3.50 |
| Plasma Clearance (L/h) ±2.38 | 14.69 | 14.56 ±1.76 | 13.35 ±2.83 | 12.70 ±2.50 | 12.35 ±1.75 | 12.04 ±1.70 | 15.32 ±2.33 | 14.44 ±1.97 | 13.43 ±3.23 | 12.08 ±2.09 |

$C_{max}$ =maximum observed drug concentration; AUC(0-Tlast) =area under the drug concentration-time curve from time zero to time t last; Vss = apparent volume of distribution at steady state

**TABLE 3.**

| Parameter | Meropenem 1 g | | | | | | | | Meropenem 2 g | |
|---|---|---|---|---|---|---|---|---|---|---|
| | Alone | Compound I 250mg | Alone | Compound I 1000mg | Alone | Compound I 1500mg | Alone | Compound I 2000mg | Alone | Compound I 2000mg |
| | (N=24) | (N=8) | (N=9) | (N=5) | (N=13) | (N=7) | (N=14) | (N=7) | (N=14) | (N=8) |
| $C_{max}$(mg/L) | 16.35 ±3.04 | 17.17 ±4.81 | 18.93 ±3.65 | 20.16 ±3.97 | 20.75 ±2.23 | 20.76 ±4.53 | 17.31 ±2.45 | 18.21 ±2.06 | 42.54 ±15.24 | 48.83 ±5.88 |
| $AUC_{(0-\infty)}$ (mg·h/L) | 51.32 ±8.88 | 52.31 ±12.88 | 59.77 ±12.09 | 65.88 ±15.33 | 64.97 ±8.86 | 65.94 ±15.55 | 53.78 ±8.81 | 58.69 ±9.91 | 130.34 ±34.95 | 142.55 ±28.72 |
| Half-Life (h) | 0.98 ±0.18 | 0.91 ±0.14 | 0.96 ±0.11 | 1.15 ±0.21 | 0.89 ±0.08 | 1.03 ±0.19 | 0.96 ±0.09 | 1.01 ±0.31 | 1.14 ±0.36 | 1.51 ±0.98 |
| Vss (L) | 25.86 ±6.55 | 22.18 ±2.63 | 21.59 ±3.21 | 21.06 ±4.50 | 18.89 ±2.62 | 21.4 ±4.28 | 23.46 ±2.53 | 22.36 ±1.89 | 22.59 ±5.24 | 21.74 ±3.05 |
| Plasma Clearance (L/h) | 20.04 ±3.40 | 16.94 ±2.47 | 17.39 ±3.71 | 15.84 ±3.57 | 15.64 ±1.98 | 15.75 ±2.90 | 19.11 ±3.44 | 17.39 ±2.41 | 16.13 ±3.33 | 14.49 ±2.67 |

Cmax =maximum observed drug concentration; AUC(0-Tlast) =area under the drug concentration-time curve from time zero to time t last; Vss = apparent volume of distribution at steady state

Maximum concentrations of Compound I and meropenem were achieved at the end of the 3-hour infusions. Compound I and meropenem exposures (Cmax and AUC) increased proportionally with dose. The PK parameters of Compound I and meropenem following a single dose alone or in combination show no major changes in the PK properties of either drug (Tables 2 and 3). Meropenem PK alone and in combination with Compound I observed in this study is consistent with published literatures. *See,* for example, Lodise T.P. et al., "Penetration of meropenem into epithelial lining fluid of patients with ventilator-associated pneumonia," Antimicrob Agents Chemother. 2011; 55(4):1606-10 and Kuti J. L. et al., "Use of Monte Carlo simulation to design an optimized pharmacodynamics dosing strategy for meropenem," J Clin Pharmacol.2003; 43(10): 1116-23.

Table 4 summarizes the treatment emergent adverse events (AEs) observed in ≥ 3 subjects receiving the combination of Compound I and meropenem. No subjects discontinued due to AEs and no SAEs were observed. There was no evidence of increasing numbers or severity of AEs with increasing dose of either drug alone or in combination, and all AEs were mild or moderate in severity.

**TABLE 4.**

**Treatment Emergent Adverse Events Observed in ≥ 3 Subjects Receiving Compound I / Meropenem**

| N (%) | Pooled Placebo | Pooled Meropenem alone | Compound I 250 mg/ Meropenem 1g | Compound 11000 mg / Meropenem 1g | Compound I 1500 mg/ Meropenem 1g | Compound I 2000 mg/ Meropenem 1g | Compound I 2000 mg/ Meropenem 2g | Pooled Compound I / Meropenem |
|---|---|---|---|---|---|---|---|---|
| | (N=16) | (N=19) | (N=8) | (N=5) | (N=8) | (N=8) | (N=8) | (N=37) |
| Subjects with TEAEs | 12 (75%) | 18(95%) | 6(75%) | 5(100%) | 5(63%) | 4(50%) | 5 (63%) | 25(67%) |
| Headache | 2(12%) | 7(37%) | 3(37%) | 1 (20%) | 0 | 1 (12%) | 0 | 5(13%) |
| PK catheter site hematoma | 2(12%) | 4(21%) | 0 | 1 (20%) | 3(37%) | 0 | 1 (12%) | 5(13%) |
| Infusion site pain | 2(12%) | 2(10%) | 2(25%) | 0 | 0 | 2 (25%) | 0 | 4(11%) |
| PK catheter site pain | 3(19%) | 2(10%) | 0 | 0 | 1 (12%) | 1 (12%) | 2(25%) | 4(11%) |

Conclusion: Compound I alone and in combination with 1 or 2g meropenem was safe and well-tolerated at all doses tested. AUC and Cmax increased proportionally with dose and the pharmacokinetic parameters of Compound I and meropenem are similar. There were no effects of meropenem or Compound I on the PK of the other agent.

Example 3

Example 3 provides a summary of a clinical study of the safety, tolerability and pharmacokinetics of the beta-lactamase inhibitor Compound I alone, meropenem alone, and the combination of both following 7 days of TID (three times a day) in healthy adult subjects.

Methods: Eighty healthy subjects were enrolled into 1 of 5 cohorts in the single ascending dose phase (250 mg, 1000 mg, 1500 mg and 2000 mg Compound I in combination with 1 or 2 g of meropenem). Within each cohort subjects were administered either Compound I or meropenem on day 1, then were crossed over to Compound I or meropenem on day 3, then were administered both Compound I and meropenem in combination on day 7 followed by 7 days of TID dosing. All infusions were administered over 3 hours. Intensive plasma and urine PK sampling was obtained after dosing and assayed using validated HPLC/MS methods. Plasma pharmacokinetics of Compound I alone and in combination with meropenem after single and 7 days of TID dosing by 3-hour infusions in healthy subjects and plasma pharmacokinetics of meropenem alone and in combination with Compound I after single and 7 days of TID dosing by 3-hour infusions in healthy subjects are illustrated in **FIGs. 5** and **6** respectively.

Results: The pharmacokinetic parameters, derived using non-compartmental methods, for each drug alone and in combination in the Compound I/meropenem 1 g/1 g and 2 g/ 2 g cohorts are shown in Tables 5 and 6 below. Table 5 summarizes Compound I pharmacokinetic parameters (mean ±standard deviation) following a single dose alone (single) and single (first) followed by 7 days of TID dosing (last) of Compound I administered in combination with meropenem as 3-hour infusions to healthy subjects. Table 6 summarizes meropenem pharmacokinetic parameters (mean ±standard deviation) following a single dose alone (single) and single (first) followed by 7 days of TID dosing (last) of meropenem administered in combination with Compound I as 3-hour infusions to healthy subjects.

**TABLE 5.**

| Parameter | Compound I 250 mg | | | Compound I 1000 mg | | | Compound I 1500 mg | | | Compound I 2000 mg | | | Compound I 2000 mg | | |
|---|---|---|---|---|---|---|---|---|---|---|---|---|---|---|---|
| | Alone | Meropenem 1 g | | Alone | Meropenem 1 g | | Alone | Meropenem 1 g | | Alone | Meropenem 1 g | | Alone | Meropenem 1 g | |
| | Single (N=16) | First (N=8) | Last (N=8) | Single (N=5) | First (N=5) | Last (N=5) | Single (N=8) | First (N=7) | Last (N=7) | Single (N=8) | First (N=8) | Last (N=7) | Single (N=8) | First (N=8) | Last (N=8) |
| $C_{max}$(mg/L) | 5.20 ±0.92 | 5.34 ±0.78 | 4.61 ±0.70 | 21.98 ±3.54 | 23.68 ±4.38 | 19.96 ±1.67 | 37.23 ±5.33 | 37.14 ±4.70 | 32.74 ±3.28 | 39.20 ±4.29 | 41.44 ±4.38 | 34.93 ±3.96 | 51.44 ±16.16 | 51.66 ±7.26 | 55.61 ±10.96 |
| $AUC_{(0-\infty)}$ (mg·h/L) | 17.48 ±3.02 | 17.40 ±2.22 | 14.73 ±2.19 | 77.56 ±15.87 | 81.18 ±15.38 | 68.57 ±8.53 | 123.66 ±18.03 | 127.07 ±20.99 | 114.32 ±15.39 | 133.26 ±20.89 | 141.02 ±21.35 | 112.31 ±8.56 | 159.21 ±44.58 | 170.44 ±31.99 | 190.43 ±32.90 |
| Half-Life (h) | 1.18 ±0.35 | 1.08 ±0.21 | 1.17 ±0.17 | 1.56 ±0.67 | 1.53 ±0.32 | 1.09 ±0.16 | 1.21 ±0.24 | 1.35 ±0.22 | 1.08 ±0.09 | 1.31 ±0.32 | 1.43 ±0.22 | 1.19 ±0.21 | 1.39 ±0.20 | 1.98 ±0.81 | 1.37 ±0.24 |
| Vss (L) | 23.15 ±6.00 | 22.25 ±3.02 | 24.92 ±5.10 | 21.44 ±5.22 | 20.25 ±3.20 | 19.93 ±1.61 | 19.37 ±5.14 | 19.83 ±2.84 | 18.05 ±2.22 | 22.02 ±2.24 | 22.43 ±2.00 | 24.95 ±2.63 | 21.37 ±3.33 | 21.84 ±3.50 | 17.50 ±1.99 |
| Plasma Clearance (L/h) | 14.69 ±2.38 | 14.56 ±1.76 | 16.71 ±2.52 | 13.35 ±2.83 | 12.70 ±2.50 | 14.55 ±2.05 | 12.35 ±1.75 | 12.04 ±1.70 | 13.12 ±1.69 | 15.32 ±2.33 | 14.44 ±1.97 | 17.61 ±1.44 | 13.43 ±3.23 | 12.08 ±2.09 | 10.42 ±1.85 |

Cmax =maximum observed drug concentration; AUC(0-Tlast) =area under the drug concentration-time curve from time zero to time t last; Vss = apparent volume of distribution at steady state; First - First dose of TID dosing for 7 days; Last - Last Dose after 7 days of TID dosing

**TABLE 6.**

| Parameter | Meropenem 1g | | | | | | | | | | | | Meropenem 2 g | | |
|---|---|---|---|---|---|---|---|---|---|---|---|---|---|---|---|
| | Alone | Compound I 250mg | | Alone | Compound I 1000mg | | Alone | Compound I 1500mg | | Alone | Compound I 2000mg | | Alone | Compound I 2000mg | |
| | Single (N=16) | First (N=8) | Last (N=8) | Single (N=9) | First (N=5) | Last (N=5) | Single (N=13) | First (N=7) | Last (N=7) | Single (N=14) | First (N=7) | Last (N=7) | Single (N=14) | First (N=8) | Last (N=8) |
| $C_{max}$(mg/L) | 16.35 ±3.04 | 17.17 ±4.81 | 15.83 ±1.96 | 18.93 ±3.65 | 20.16 ±3.97 | 17.04 ±1.65 | 20.75 ±2.23 | 20.76 ±4.53 | 20.36 ±4.70 | 17.31 ±2.45 | 18.21 ±2.06 | 15.81 ±1.29 | 42.54 ±15.24 | 48.83 ±5.88 | 43.35 ±8.82 |
| $AUC_{(0-\infty)}$ (mg·h/L) | 51.32 ±8.88 | 52.31 ±12.88 | 47.64 ±4.91 | 59.77 ±12.09 | 65.88 ±15.33 | 54.52 ±6.96 | 64.97 ±8.86 | 65.94 ±15.55 | 66.09 ±14.41 | 53.78 ±8.81 | 58.69 ±9.91 | 48.06 ±2.01 | 130.34 ±34.95 | 142.55 ±28.72 | 137.71 ±26.37 |
| Half-Life (h) | 0.98 ±0.18 | 0.91 ±0.14 | 0.98 ±0.11 | 0.96 ±0.11 | 1.15 ±0.21 | 0.94 ±0.03 | 0.89 ±0.08 | 1.03 ±0.19 | 0.88 ±0.09 | 0.96 ±0.09 | 1.01 ±0.31 | 1.08 ± 0.15 | 1.14 ±0.36 | 1.51 ±0.98 | 1.07 ±0.16 |
| Vss (L) | 25.86 ±6.55 | 22.18 ±2.63 | 26.44 ±4.74 | 21.59 ±3.21 | 21.06 ±4.50 | 21.19 ±2.43 | 18.89 ±2.62 | 21.4 ±4.28 | 18.53 ±4.31 | 23.46 ±2.53 | 22.36 ±1.89 | 24.97 ±2.41 | 22.59 ±5.24 | 21.74 ±3.05 | 20.08 ±3.20 |
| Plasma Clearance (L/h) | 20.04 ±3.40 | 16.94 ±2.47 | 20.96 ±2.04 | 17.39 ±3.71 | 15.84 ±3.57 | 18.4 ±2.24 | 15.64 ±1.98 | 15.75 ±2.90 | 15.59 ±3.18 | 19.11 ±3.44 | 17.39 ±2.41 | 20.65 ±0.84 | 16.13 ±3.33 | 14.49 ±2.67 | 14.77 ±2.84 |

Cmax =maximum observed drug concentration; AUC(0-Tlast) =area under the drug concentration-time curve from time zero to time t last; Vss = apparent volume of distribution at steady state; First - First dose of TID dosing for 7 days; Last - Last Dose after 7 days of TID dosing

Maximum concentrations of Compound I and meropenem were achieved at the end of the 3-hour infusions. Compound I and meropenem exposures ($C_{max}$ and AUC) increased proportionally with dose. The PK parameters of Compound I and meropenem alone or in combination show no major changes in the PK properties of either drug (see Tables 5 and 6). There was no accumulation of either Compound I or meropenem observed after 7 days of TID dosing. Meropenem PK alone and in combination with Compound I observed in this study is consistent with published literatures.

Table 7 summarizes the number (%) of subjects with at least one treatment emergent AE and number of adverse events during the multiple dose phase. One subject who received meropenem 1 g/Compound I 2 g discontinued early due to an AE of thrombophlebitis. All AEs, except 2 were mild or moderate in severity. Mild nausea was observed only in the subjects who received meropenem 2 g, either alone or in combination. There is no evidence that the addition of Compound I changed the AE profile of meropenem.

**TABLE 7.**

**Number (%) of Subjects with at least one Treatment Emergent AE and Number of Events [ ] during the Multiple Dose Phase**

| N (%) [Number of AEs] | Pooled Placebo (N=18) | Cohort 1 250 mg Compound I and 1 g Meropenem (N=8) | Cohort 2 1 g Compound I and 1 g Meropenem (N=5) | Cohort 3 1.5 g Compound I and 1 g Meropenem (N=8) | Cohort 4 2g Compound I and 1 g Meropenem (N=8) | Cohort 5 2g Compound I and 2 g Meropenem (N=8) | Pooled 2g Meropenem (N=5) | Pooled Meropenem (1 and 2 g) (N=21) | All Compound I / Meropenem Combination (N=45) |
|---|---|---|---|---|---|---|---|---|---|
| AEs | 15(83%) [34] | 7(88%) [171 | 5(100%) [20] | 7(88%) [16] | 7(88%) [20] | 8(100%) [34] | 4(80%) [24] | 20 (95%) [67] | 41(91%) [155] |
| Moderate or Severe AEs | 2(11%) [2] | 2(25%) [2] | 0 | 1 (13%) [1] | 4(50%) [5] | 3(38%) [3] | 0 | 5(24%) [5] | 13(29%) [14] |
| SAEs | 0 | 0 | 0 | 0 | 0 | 0 | 0 | 0 | 0 |

Conclusion: Compound I alone and in combination with 1 g or 2 g meropenem was safe and well tolerated at all doses tested, with no evidence that the safety profile of meropenem was changed by the addition of Compound I. There was no accumulation of either Compound I or meropenem observed after 7 days of TID dosing. There were no effects of meropenem on the pharmacokinetics of Compound I or vice versa.

Example 4

Example 4 provides a summary of a preliminary study of the pharmacokinetics of the combination of Compound I (2 g) and meropenem (2 g) in healthy adult subjects by a 1-hour or 3-hour infusion.

Results: The pharmacokinetics of Compound I after 3-hour or 1-hour infusions (2 g Compound I alone and in combination with 2 g meropenem) in healthy subjects are illustrated in **FIGs. 7** and **8** respectively. The mean pharmacokinetics of Compound I after 1-hour or 3-hour infusions of 2 g Compound I in combination with 2 g meropenem in healthy subjects is summarized in **FIG. 9**. With respect to Compound I, no effects of meropenem on the pharmacokinetics of Compound I were observed with either infusion rate. In addition, there is no significant effect of infusion rate on Compound I exposure (p = 0.18).

The pharmacokinetics of meropenem after 3-hour or 1-hour infusions (2 g meropenem alone and in combination with 2 g Compound I) in healthy subjects are illustrated in **FIGs. 10** and **11** respectively. The mean pharmacokinetics of meropenem after 1-hour or 3-hour infusions of 2 g meropenem in combination with 2 g Compound I in healthy subjects is summarized in **FIG. 12**. The pharmacokinetics of meropenem open-lactam after 1-hour infusions of 2 g alone and in combination with 2 g Compound I and the mean pharmacokinetics of meropenem open-lactam after 1 or 3-hour infusions of 2 g meropenem in combination with 2 g Compound I are illustrated in **FIGs. 13** and **14.**

For meropenem, no effects of Compound I on the pharmacokinetics of meropenem were observed with either infusion rate. Meropenem exposure (AUC) after a 3 hour infusion of 2 g meropenem is consistent with published literatures. There was an increase in meropenem exposure (AUC) with 1-hour infusion compared to 3-hour infusion. Meropenem exposure (AUC) after a 1 hour infusion of 2 g meropenem is about 48% greater than that observed after a 3 hour infusion of 2 g meropenem (211 vs 142 mg*h/L). Meropenem weight adjusted clearance (Cl) after a 1 hour infusion of 2 g meropenem is about 25% slower than that observed after a 3 hour infusion (0.14 vs 0.19 l/h/kg; p = 0.015). Possible reasons for the difference observed in meropenem weight adjusted clearance may due to saturable renal clearance at 2 g dose due to high Cmax or longer infusion reduces the "dose" due to degradation (opening of the β-lactam ring results in formation of meropenem open-lactam).

Example 5

Example 5 provides a summary of an open-label study of the safety and pharmacokinetics of the combination of Compound I and meropenem in subjects with reduced renal function, including patients with standard hemodialysis.

The safety and pharmacokinetics of a single IV dose of 1 g meropenem plus 1 g Compound I, infused over 3 hours, was evaluated. Forty one subjects were enrolled in 5 groups based on their degree of renal insufficiency. The five cohorts included: patients with normal renal function (CrCl ≥ 90 ml/min), mild renal impairment (CrCl 60-89ml/min), moderate renal impairment (CrCl 30-<60ml/min), severe renal impairment (CrCl <30ml/min, and patients with end stage renal disease requiring hemodialysis. Patients on renal replacement therapy other than standard hemodialysis (including continuous veno-venous hemofiltration, continuous veno-venous hemodialysis and continuous renal replacement therapy) were not studied.

**FIG. 15** shows the relation between estimated GFR and meropenem or Compound I plasma clearance. The plasma clearance of both drugs remained similar throughout the range of renal function as evidenced by the clustering of values and the linear decline in clearance with decreasing renal function.

The removal of meropenem and Compound I during hemodialysis was studied in 9 patients with severe renal insufficiency on chronic hemodialysis. Patients received a single meropenem 1 g/ Compound I 1 g dose, followed by a hemodialysis session. Both meropenem and Compound I were removed from plasma by hemodialysis. These data indicate that maintenance doses of each drug (adjusted for degree of underlying endogenous renal function) should be administered after a dialysis session.

Determination of the combination of Compound I/meropenem dosage in patients with renal impairment

Dosage adjustment according to degree of renal impairment was determined by analysis of estimates of each subject's pharmacokinetics and determining exposures according to possible dosage regimens of meropenem or Compound I. The objective was to maintain exposures (as AUC) across the range of renal function to as consistent as possible across the spectrum of renal function. In view of PK-PD analyses in nonclinical models that show AUC is linked to efficacy for Compound I, AUC was the appropriate controller of efficacy for this agent. Since T>MIC is the PK-PD index important

of meropenem, different dosing intervals were evaluated to insure $T>MIC_{breakpoint}$ was above threshold values (T>MIC > 40%) for efficacy. For purposes of this analysis, the forecasted susceptibility breakpoint for meropenem based on the 2 gram dose and 3-hour infusion was 8 μg/ml. Free drug was considered for both meropenem and Compound I (plasma protein binding of 6% and 33%, respectively).

Meropenem

Table A shows meropenem AUC measured in each patient and PK-PD indices for three potential dosage regimens in each patient according to measured meropenem PK in each subject. Meropenem dosage regimens were identified for each of the strata of renal function that would meet or achieve target exposures (T>MIC of at least 40%) in all subjects (see shaded cells).

Table A summarizes the Analysis of different meropenem dosing regimens by individual subjects. The PK-PD target for meropenem is a T>MIC of at least 40% of the dosage interval where the MIC is 8 μg/mL. The shading in different creatinine clearance groups denotes the recommended meropenem dosing regimen.

**TABLE A.**

| Expected Meropenem Time, in hours per day, (% of dosing interval) Above MIC of 8μg/ml. According to Dosage Regimen | | | | | | |
|---|---|---|---|---|---|---|
| **Subject** | **Estimated Creatinine Clearance (ml/min)** | **2g q8h** | **1g q8h** | **1g q24h** | **500 mg q12h** | **500 mg q24h** |
| | **Normal** | **Mean 13.8 (58) Range 10.5-16.5 (44-69)** | | | | |
| **>50 mL/min group** | | | | | | |
| 4602 | 83 | 16.5 (69) | 13.5 (56) | 4.5(19) | 7(29) | 3.5 (15) |
| 5609 | 79 | 15.0 (63) | 12 (50) | 4(17) | 6(25) | 3(13) |
| 5607 | 77 | 16.5 (69) | 13.5 (56) | 4.5(19) | 7(29) | 3.5 (15) |
| 5618 | 77 | 16.5 (69) | 13.5 (56) | 4.5(19) | 7.6 (32) | 3.8(16) |
| 4601 | 71 | 20 (83) | 16.5 (69) | 5.5 (23) | 8(33) | 4(17) |
| 5605 | 67 | 16.5 (69) | 13.5 (56) | 4.5(19) | 7(29) | 3.5 (15) |
| 5606 | 56 | 20 (83) | 16.5 (69) | 5.5 (23) | 9(38) | 4.5(19) |
| 4613 | 55 | 20 (83) | 16.5 (69) | 5.5 (23) | 7.6 (32) | 3.8(16) |
| **30 - 49 ml/min group** | | | | | | |
| 5603 | 46 | 24 (100) | 18 (75) | 6(25) | 10(42) | 5(21) |
| 5608 | 44 | 24 (100) | 18 (75) | 6(25) | 9(38) | 4.5(19) |
| 5620 | 42 | 24 (100) | 16.5 (69) | 5.5 (23) | 8(33) | 4(17) |
| 5611 | 40 | 24 (100) | 24 (100) | 8(33) | 12(50) | 6(25) |
| 5610 | 38 | 24 (100) | 24(100) | 10(42) | 12(50) | 6(25) |
| 5614 | 32 | 24 (100) | 24(100) | 10(42) | 14(58) | 7(29) |
| **10 -19 ml/min group** | | | | | | |
| 5616 | 15 | 24 (100) | 24 (100) | 12(50) | 20 (83) | 10(42) |
| 5617 | 14 | 24 (100) | 24(100) | 12(50) | 16(67) | 8(33) |
| 4636 | 14 | 24 (100) | 24(100) | 10(42) | 16(67) | 8(33) |
| 5621 | 12 | 24 (100) | 24(100) | 12(50) | 20 (83) | 10(42) |
| 5615 | 11 | 24 (100) | 24(100) | 12(50) | 16(67) | 8(33) |
| 5612 | 10 | 24 (100) | 24 (100) | 14 (58) | 24 (100) | 12 (50) |

(continued)

| 5 - 9 ml/min group | | | | | | |
|---|---|---|---|---|---|---|
| 4640 | 8 | 24 (100) | 24 (100) | 24 (100) | 24 (100) | 12(50) |
| 5633 | 7 | 24 (100) | 24 (100) | 24 (100) | 24 (100) | 12 (50) |
| 5637 | 7 | 24 (100) | 24 (100) | 14 (58) | 20 (83) | 10(42) |
| 5642 | 6 | 24 (100) | 24 (100) | 24 (100) | 24 (100) | 12 (50) |
| 5634 | 6 | 24 (100) | 24 (100) | 24 (100) | 24 (100) | 12 (50) |
| 5641 | 5 | 24 (100) | 24(100) | 24(100) | 24 (100) | 24(100) |
| 5638 | 5 | 24 (100) | 24 (100) | 24 (100) | 24 (100) | 12(50) |

Compound I

Table B shows Compound I AUC measured in each patient and 24h AUC for three potential dosage regimens according to measured Compound I clearance in each subject. Since AUC is the target PK metric and Compound I clearance remained close to meropenem clearance, unit and 24 hr doses remained at a 1:1 ratio throughout the range of renal function.

Considerations for subjects with creatinine clearance < 10 ml/min

As noted in **FIG. 15**, as creatinine clearance falls below 10 ml/min, meropenem non-renal clearance assumes a greater proportion of total clearance. In contrast, Compound I has no measureable non-renal clearance. Thus, to maintain a 1:1 dose ratio to provide therapeutic exposures of each component and to avoid accumulation of Compound I, patients with a creatinine clearance <10 ml/min should receive hemodialysis about every 3 days (*i.e.*, twice weekly).

Table B provides a summary of the analysis of different Compound I dosing regimens by individual subjects enrolled the study. The shading in different creatinine clearance groups denotes the recommended meropenem dosing regimen.

**TABLE B. EXPECTED COMPOUND I FREE DRUG 24 H AUC (MG*HR/L)**

| **Expected Meropenem Time, in hours per day, (% of dosing interval) Above MIC of 8μg/ml. According to Dosage Regimen** | | | | | | | |
|---|---|---|---|---|---|---|---|
| **Subject** | **Estimated Creatinine Clearance (ml/min)** | **Observed $AUC_{0-inf}$ following 1g dose** | **2g q8h** | **1g q8h** | **1g q24h** | **500mg q12h** | **500mg q24h** |
| | **Normal** | | **Mean 358 Range 284 - 470** | | | | |
| >50 mL/min group | | | | | | | |
| 4602 | 83 | *70.0* | 420.0 | 210.0 | 70.0 | 70.0 | 35.0 |
| 5609 | 79 | *62.7* | 376.3 | 188.2 | 62.7 | 62.7 | 31.4 |
| 5607 | 77 | *69.2* | 415.0 | 207.5 | 69.2 | 69.2 | 34.6 |
| 5618 | 77 | *88.4* | 530.5 | 265.2 | 88.4 | 88.4 | 44.2 |
| 4601 | 71 | *70.7* | 424.2 | 212.1 | 70.7 | 70.7 | 35.4 |
| 5605 | 67 | *68.1* | 408.7 | 204.3 | 68.1 | 68.1 | 34.1 |
| 5606 | 56 | *108.4* | 650.6 | 325.3 | 108.4 | 108.4 | 54.2 |
| 4613 | 55 | *108.0* | 648.1 | 324.0 | 108.0 | 108.0 | 54.0 |
| 30-49 ml/min group | | | | | | | |
| 5603 | 46 | *119.3* | 715.7 | 357.8 | 119.3 | 119.3 | 59.6 |

(continued)

| | | | | | | | |
|---|---|---|---|---|---|---|---|
| 30-49 ml/min group | | | | | | | |
| 5608 | 44 | *129.1* | 774.5 | 387.2 | 129.1 | 129.1 | 64.5 |
| 5620 | 42 | *115.2* | 690.9 | 345.5 | 115.2 | 115.2 | 57.6 |
| 5611 | 40 | *228.1* | 1368.4 | 684.2 | 228.1 | 228.1 | 114.0 |
| 5610 | 38 | *251.1* | 1506.5 | 753.3 | 251.1 | 251.1 | 125.5 |
| 5614 | 32 | *310.6* | 1863.5 | 931.8 | 310.6 | 310.6 | 155.3 |
| | 10 - 19 ml/min group | | | | | | |
| 5616 | 15 | *505.1* | 3030.3 | 1515.2 | 505.1 | 505.1 | 252.5 |
| 5617 | 14 | *427.3* | 2563.8 | 1281.9 | 427.3 | 427.3 | 213.7 |
| 4636 | 14 | *493.6* | 2961.8 | 1480.9 | 493.6 | 493.6 | 246.8 |
| 5621 | 12 | *790.7* | 4744.3 | 2372.2 | 790.7 | 790.7 | 395.4 |
| 5615 | 11 | *830.3* | 4981.6 | 2490.8 | 830.3 | 830.3 | 415.1 |
| 5612 | 10 | *719.6* | 4317.6 | 2158.8 | 719.6 | 719.6 | 359.8 |
| 5 - 9 ml/min group | | | | | | | |
| 4640 | 8 | *8617.7* | 51706.2 | 25853.1 | 8617.7 | 8617.7 | 4308.9 |
| 5633 | 7 | *4189.5* | 25137.0 | 12568.5 | 4189.5 | 4189.5 | 2094.8 |
| 5637 | 7 | *794.5* | 4767.0 | 2383.5 | 794.5 | 794.5 | 397.3 |
| 5642 | 6 | *923.2* | 5539.8 | 2769.9 | 923.2 | 923.2 | 461.7 |
| 5634 | 6 | *840.0* | 5040.0 | 2520.0 | 840.0 | 840.0 | 420.0 |
| 5641 | 5 | *7581.7* | 45490.2 | 22745.1 | 7581.7 | 7581.7 | 3790.0 |
| 5638 | 5 | *2289.0* | 13734.0 | 3270.0 | 2289.0 | 2289.0 | 1144.5 |

Based on the above analysis, the Compound I/meropenem Combination dosage regimens in Table C can be used for subjects with impaired renal function.

**TABLE C: COMPOUND I/MEROPENEM COMBINATION DOSAGE ACCORDING TO RENAL FUNCTION**

| Estimated Creatinine Clearance (ml/min) | the Combination Dosage Regimen (All doses infused over 3 hrs) |
|---|---|
| ≥50 | Meropenem 2 g / Compound I 2g q8h |
| ≥30 - 49 | Meropenem 1 g / Compound I 1 q q8h |
| ≥20 - 29 | Meropenem 1 g / Compound I 1g q12h |
| ≥10-19 | Meropenem 500mg / Compound I 500mg q 12 h |
| <10 | Meropenem 500mg / Compound I 500mg every q 24h[1] |
| 1 Dosage regimen assumes patients receive hemodialysis at least twice per week. Maintenance doses of the Combination in these patients should be administered as soon as possible after the dialysis session. For example, if a subject is scheduled to receive the Combination at 18:00 but receives hemodialysis at 13:00, the planned 18:00 Combination dose should be given after the dialysis session is completed (rather than waiting until 18:00). | |

It is concluded that dose adjustment for renal function can be based on either meropenem or Compound I as both drugs are affected similarly as renal function declines. For subjects with creatinine clearance of equal or greater than 50 ml/min, there is no need for dose adjustment. The standard dosage of 2 g Compound 1/2 g meropenem TID (every 8 hours) can be used. For subjects with creatinine clearance of equal or greater than 30 ml/min and less than 50 ml/min, a reduced dosage of 1 g Compound I/1 g meropenem TID (every 8 hours) can be used and still achieve desired

effects. For subjects with creatinine clearance of equal or greater than 20 ml/min and less than 30 ml/min, a reduced dosage of 1 g Compound I/1 g meropenem administered every 12 hours can be used. For subjects with creatinine clearance of equal or greater than 10 ml/min and less than 20 ml/min, a reduced dosage of 500mg Compound I/500 mg meropenem administered every 12 hours can be used. For subjects with creatinine clearance of less than 10 ml/min, a reduced dosage of 500mg Compound I/500 mg meropenem every 24 hours can be used.

Example 6

Example 6 provides a summary of a randomized, open-label clinical study evaluating the plasma, epithelial lining fluid (ELF), and alveolar macrophage (AM) concentrations of the combination of 2 g Compound I/ 2 g meropenem ("the Combination") in healthy adult subjects.

For lower respiratory tract infections, epithelial lining fluid (ELF) and alveolar macrophages (AM) have been advocated as important infection sites for common extracellular and intracellular pathogens, respectively. Studies with bronchoscopy and bronchoalveolar lavage (BAL), which can reliably assess the intrapulmonary penetration of antibiotics into the ELF and AM, are needed. The primary objectives of this pharmacokinetic study are to determine and compare the plasma, ELF, and AM concentrations of Compound I and meropenem administered following multiple intravenous doses (2 g meropenem/ 2 g Compound I administered q8h for 3 doses) in healthy male and female adult subjects. A secondary objective of this study was to assess the safety and tolerability of intravenous administration of the Combination in healthy adult subjects.

Methods for Pharmacokinetic Analysis

*Study design and subjects.* A total of twenty-five (n=25) male and female subjects who met the study entry criteria and completed all phases of the pharmacokinetic study were included in this pharmacokinetic analysis. Each subject received the Combination (2 g of meropenem / 2 g of Compound I) administered every 8 hours for a total of three doses under direct observation at the study site. Blood samples were collected to measure drug concentrations in plasma prior to (time 0), and at 1.5, 2.95, 3.083, 3.25, 3.5, 4, 6, and 8 hours after the start of a 3-hour intravenous infusion of the third combination dose. Each subject had a single standardized bronchoscopy with BAL scheduled at a timed interval following the last dose of the Combination as indicated in the following table:

| | **BAL Sampling Times after Start of the Third Infusion of the Combination** | | | | |
|---|---|---|---|---|---|
| Sampling Time | 1.5 h | 3.25 h | 4h | 6h | 8h |
| Subjects (n) | 5 | 5 | 5 | 5 | 5 |

Urea has been commonly used as an endogenous marker to estimate the apparent volume of ELF. Blood samples to determine plasma urea concentrations were obtained just prior to scheduled bronchoscopy. Aliquots of BAL were obtained to determine urea concentrations in BAL and cell count with differential. The standardized bronchoscopy with BAL procedure for the collection of intrapulmonary samples has been previously described in the references listed below.

*Drug and Urea Assays.* Sample preparation procedures and assays for meropenem, Compound I, and meropenem open-lactam concentrations in plasma, ELF, and AM were performed with a high-performance liquid chromatography with mass spectrometric detection at MicroConstants, Inc., San Diego, CA (Reports MC14B-0013, MC14B-0015, MC14I-011, and MC14I-0012). The urea concentrations in plasma and BAL were performed with a microplate-based method with an O-phthalaldehyde chromogenic solution at MicroConstants, Inc., San Diego, CA.

*Pharmacokinetic Calculations of Plasma Concentrations.* Noncompartmental methods were used to generate pharmacokinetic parameters for meropenem, Compound I, and meropenem open-lactam in plasma. Peak plasma concentration ($C_{max}$) and time to $C_{max}$ ($T_{max}$) were read from the observed plasma concentration-time profile after the start of the intravenous infusion of the third Combination dose. Area under the plasma concentration-time curve over 8 hours ($AUC_{0-8}$) after the third dose was calculated with the linear-log trapezoidal rule (WinNonlin®, version 6.3, Pharsight Corporation, Cary, North Carolina). The elimination rate constant (β) was determined by nonlinear least-squares regression. Elimination half-life ($t_{1/2}$) was calculated by dividing β into the natural logarithm of two. For meropenem and Compound I, the apparent clearance (CL) and volume of distribution terms ($V_{ss}$) were calculated with the standard noncompartmental equations embedded in the WinNonlin® prorgram.

*Calculations of ELF Volume and Antibiotic Concentrations in ELF and AM* The calculations of ELF volume and drug concentrations in ELF and AM were performed with BAL supernatant and pulmonary (alveolar) cells ("cell pellet") from aspirates recovered from the 2nd, 3rd, and 4th instillations (BAL2). The concentration of drug ($ABX_{ELF}$) in the epithelial

lining fluid (ELF) was determined as follows:

$$ABX_{ELF} = ABX_{BAL}\ x\ (V_{BAL} / V_{ELF})$$

where $ABX_{BAL}$ is the measured concentration of meropenem, Compound I or meropenem open-lactam in BAL fluid, $V_{BAL}$ is the volume of aspirated BAL fluid, and $V_{ELF}$ is the volume of ELF sampled by the BAL. $V_{ELF}$ is derived from the following:

$$V_{ELF} = V_{BAL}\ x\ Urea_{BAL} / Urea_{P}$$

where $Urea_{BAL}$ is the concentration of urea in BAL fluid and $Urea_{P}$ is the concentration of urea in plasma.

The concentration of drug ($ABX_{AM}$) in the alveolar cells (AC) was determined as follows:

$$ABX_{AM} = ABX_{M} / V_{AC}$$

where $ABX_{M}$ is the measured concentration of meropenem, Compound I or meropenem open-lactam in the 1-ml cell suspension, and $V_{AC}$ is the volume of alveolar cells in the 1-ml cell suspension. Differential cell count was performed to determine the number of macrophages present. A mean macrophage cell volume of 2.42 μl/106 cells was used in the calculations for volume of alveolar cells in the pellet suspension.

The concentration ratios of ELF and AM to the simultaneous plasma concentrations were calculated for each subject and summarized for each group at each sampling time. The mean and median concentrations of meropenem and Compound I from the bronchopulmonary sampling times (e.g., 1.5, 3.25, 4, 6, and 8 hours) were used to estimate the $AUC_{0-8}$ of plasma, ELF, and AM. The 8-hour sampling time was also used as a value at time zero for determining the area term of plasma, ELF, and AM. The $AUC_{0-8}$ for each matrix was determined with the linear trapezoidal method. The ratio of $AUC_{0-8}$ of ELF to plasma and AM to plasma were calculated.

Results

Twenty-six (26) healthy adult subjects were enrolled into this study. One subject was discontinued from the study due to adverse events and pharmacokinetic phases (e.g., blood sample collection to measure drug concentrations in plasma and a bronchoscopy with BAL at the scheduled sampling time [4-hour]) were not performed. The characteristics of the 25 study subjects receiving the Combination for three doses and completing all phases of the pharmacokinetic study are reported in Table 8.

Mean (±SD) plasma concentrations of meropenem before and after the start of the intravenous infusion of the third Combination dose are displayed in **FIG. 16**. The mean (± SD) $C_{max}$ and $AUC_{0-8}$ for plasma meropenem concentrations were 58.2 ± 10.8 μg/mL and 185.5 ± 33.6 μg·h/mL, respectively. The mean (±SD) pharmacokinetic parameters of meropenem in plasma are summarized in Table 9. Mean (±SD) plasma concentrations of Compound I before and after the start of the intravenous infusion of the third Combination dose are displayed in **FIG. 17**. The mean (± SD) $C_{max}$ and $AUC_{0-8}$ for plasma Compound I concentrations were 59.0 ± 8.4 μg/mL and 204.2 ± 34.6 (μg·h/mL, respectively. The mean (±SD) pharmacokinetic parameters of Compound I in plasma are summarized in Table 10.

The mean (±SD) concentrations of meropenem in plasma and ELF at the bronchopulmonary sampling times are illustrated in **FIG. 18**. The mean concentrations of meropenem in plasma and ELF ranged from 1.36 to 41.2 μg/mL and 2.51 to 28.3 (μg/mL, respectively. The mean (±SD) concentrations of meropenem after the last dose in plasma, ELF, and AM at the five bronchopulmonary sampling times are reported in Table 11. The concentrations of meropenem in the alveolar cells were below the quantifiable limit for all samples.

The mean (±SD) concentrations of Compound I in plasma, ELF, and AM at the bronchopulmonary sampling times are illustrated in **FIG. 19**. The mean concentrations of Compound I in plasma and ELF ranged from 2.74 to 51.1 μg/mL and 2.61 to 26.1 (μg/mL, respectively. **FIGs. 20** and **21** illustrate the similar magnitude and time course of concentrations for meropenem and Compound I in plasma and ELF. The mean (±SD) concentrations of Compound I after the last dose in plasma, ELF, and AM at the five bronchopulmonary sampling times are reported in Table 12. Alveolar macrophage concentrations of Compound I were measurable for all samples and ranged from 1.26 to 93.9 μg/mL.

The mean (±SD) ratios of ELF to the simultaneous plasma concentrations for meropenem are reported in Table 13. The mean ratios of ELF to simultaneous plasma concentrations for meropenem during the 8-hour period after drug administration ranged from 0.525 to 2.13. The $AUC_{0-8}$ values based on mean and median ELF concentrations were 111.7 and 102.4 μg·h/mL, respectively. The ratio of ELF to total plasma meropenem concentrations based on the mean

and median $AUC_{0-8}$ values were 0.63 and 0.58, respectively. The ratios of ELF to unbound plasma meropenem concentrations (protein binding = 2%) based on the mean and median $AUC_{0-8}$ values were 0.65 and 0.59, respectively.

The mean (±SD) ratios of ELF and AM to the simultaneous plasma concentrations for Compound I are reported in Table 14. The mean ratios of ELF and AM to simultaneous plasma concentration for Compound I during the 8-hour period after drug administration ranged from 0.45 to 1.01 and 0.062 to 2.58, respectively. The $AUC_{0-8}$ values based on mean and median ELF concentrations were 105.1 and 96.7 μg·hr/mL, respectively. The ratio of ELF to total plasma Compound I concentrations based on the mean and median $AUC_{0-8}$ values were 0.53 and 0.48, respectively. The ratios of ELF to unbound plasma Compound I concentrations (protein binding = 33%) based on the mean and median $AUC_{0-8}$ values were 0.79 and 0.72, respectively.

Summary

The Combination (2 g meropenem / 2 g Compound I) administered every 8 hours, as 3-hour IV infusions, achieved a similar time course and magnitude of meropenem and Compound I concentrations in plasma and ELF. The intrapulmonary penetration of meropenem and Compound I based on $AUC_{0-8}$ values of ELF and total plasma concentrations were approximately 63% and 53%, respectively. When unbound plasma concentrations were considered, penetration was 65% and 79% for meropenem and Compound I, respectively. Results from this study lend support to exploring the meropenem 2g/Compound I 2g combination as a potential antimicrobial agent for the treatment of lower respiratory tract bacterial infections caused by susceptible pathogens.

The concentrations of meropenem in the alveolar cells were below the quantifiable limit for all samples. In contrast, concentrations of Compound I were measurable for all alveolar cell samples and AM concentrations ranged from 1.26 to 93.9 μg/mL. It is worth noting that two subjects of the 6-hour sampling time had the highest reported concentrations of Compound I in AM (35.4 and 93.9 (μg/mL) which consequently inflated the mean ratio of AM to plasma concentration (2.58 ± 3.57, Table 14). Both of these subjects had extremely high concentrations of red blood cells in their BAL fluid (176,000 and 226,250 cells/$mm^3$) which may have contributed to such high measurements of AM concentrations.

The ratio of systematic exposure of meropenem open-lactam to meropenem was approximately 11% and 15% based on comparison of maximum plasma concentration and $AUC_{0-8}$ values, respectively. The mean ELF concentrations of meropenem open-lactam ranged from only 1.81 to 2.69 μg/mL during the first 6 hours after meropenem administration, and all ELF concentrations of meropenem open-lactam were below the quantifiable limit at the 8-hour sampling time. Only three AM concentrations of meropenem open-lactam were measurable and ranged from 1.91 to 8.46 μg/mL.

Conte *et al.* administered meropenem at a dose of 500 mg, 1 gram or 2 gram every 8 hours, as 30-minute IV infusions, for a total of four doses. The mean meropenem ELF concentrations at 1, 2, 3, 5, and 8 hours were 5.3, 2.7, 1.9, 0.7, and 0.2 μg/mL for the 500 mg dose and 7.7, 4.0, 1.7, 0.8, and 0.03 μg/mL for the 1 gram dose. The ratios of ELF concentrations to total plasma concentrations at the sampling times ranged from 0.49 to 2.3 for the 500 mg dose and 0.32 to 0.53 for the 1 gram dose. The intrapulmonary penetration of meropenem based on $AUC_{0-8}$ values of ELF and total plasma concentrations were approximately 43% and 28% for the 500 mg and 1 gram doses, respectively. For the 2 gram dose, the mean meropenem ELF concentrations and penetration ratios at 1- and 3-hour sampling times were 2.9 and 2.8 μg/mL, and 0.05 and 0.22, respectively. For the 2 gram dose, the number of observations were limited (n=8) and calculations of $AUC_{0-8}$ value for ELF was not possible.

The meropenem findings in this study are not directly comparable to those of Conte *et al* due to differences in study design. This study evaluated a 2 gram dose of meropenem administered as a prolonged infusion of 3 hours and in combination with Compound I. In addition, this study included more extensive collection of ELF concentrations (n=30) during the 8-hour dosing interval which allowed an accurate estimation of $AUC_{0-8}$ value. Higher mean concentrations of meropenem in plasma and ELF after 2 gram administration with prolonged infusions (range: 1.36 to 41.2 μg/mL and 2.51 to 28.3 μg/mL, respectively) was observed. It is also possible that more prolonged infusions of carbapenems may provide higher penetration into ELF, as has been reported previously for biapenem (Kikuchi *et al*). The mean ratios of ELF to simultaneous plasma concentrations for meropenem during the 8-hour period ranged from 0.525 to 2.13. The $AUC_{0-8}$ values based on mean and median ELF concentrations were 111.7 and 102.4 (μg·h/mL, respectively. The ratio of ELF to total plasma meropenem concentrations based on the mean and median $AUC_{0-8}$ values were 0.63 and 0.58, respectively. These data support further study of the Compound I/meropenem combination for treatment of pulmonary infections.

**TABLE 8.** CHARACTERISTICS OF STUDY SUBJECTS RECEIVING THE COMBINATION EVERY 8 HOURS FOR 3 DOSES

| BAL Sampling Time | Sex | Age (years) | Height (cm) | Weight (kilograms) | BMI ($kg/m^2$) | Total Cell Count in BAL Fluid ($mm^3$) | Macrophages (%) |
|---|---|---|---|---|---|---|---|
| 1.5-hour | 5 M | 32 ± 9 | 181 ± 7 | 83.2 ± 5.5 | 25.5 ± 3.3 | 114±46 | 89 ± 7 |
| 3.25-hour | 3 M, 2 F | 40 ± 12 | 174 ± 10 | 80.5 ± 11.9 | 26.6 ± 1.6 | 92 ± 52 | 83 ± 13 |
| 4-hour | 5 M | 40 ± 9 | 179 ± 10 | 80.5 ± 13.0 | 25.2 ± 2.3 | 173 ± 80 | 91 ±4 |
| 6-hour | 3 M, 2 F | 43 ± 8 | 169 ± 9 | 80.9 ± 8.3 | 28.5 ± 0.7 | 197 ± 186 | 80 ± 10 |
| 8-hour | 2 M, 3 F | 40 ±12 | 168 ±5 | 76.2±9.2 | 26.9±2.1 | 130±76 | 85±8 |

Data are expressed as mean ± SD except for sex
M = males; F = females
BMI = body mass index = weight [kg] ÷ (height [m])$^2$

**TABLE 9**. NONCOMPARTMENTAL PHARMACOKINETICS PARAMETERS IN PLASMA OF MEROPENEM 2 G EVERY 8 HOURS FOR 3 DOSES

| | $C_{max}$ (μg/mL) | $T_{max}$ (hours) | $AUC_{0-8}$ (μg·hr/mL) | $t_{1/2}$ (hours) | $V_{ss}$ (Liters) | CL (L/hr) |
|---|---|---|---|---|---|---|
| All Subjects[a] | 58.2 ± 10.8 | 2.98 ± 0.06 | 185.5 ± 33.6 | 1.03 ± 0.15 | 16.3 ± 2.6 | 11.1 ± 2.1 |
| 1.5-hour BAL Sampling Group[b] | 56.9 ± 19.3 | 2.95 ± 0.01 | 167.8 ± 41.7 | 0.98 ± 0.05 | 17.5 ± 2.5 | 12.5 ± 2.8 |
| 3.25-hour BAL Sampling Group[b] | 57.9 ± 7.5 | 3.00 ± 0.07 | 183.8 ± 29.7 | 1.04 ± 0.13 | 16.7 ± 2.6 | 11.1 ± 1.8 |
| 4-hour BAL Sampling Group[b] | 59.6 ± 7.4 | 2.98 ± 0.06 | 196.2 ± 33.5 | 1.07 ±0.15 | 15.3 ± 2.1 | 10.5 ± 1.9 |
| 6-hour BAL Sampling Group[b] | 59.4 ± 11.5 | 2.98 ± 0.06 | 197.4 ± 38.7 | 1.12 ± 0.24 | 16.1 ± 3.4 | 10.4 ± 2.0 |
| 8-hour BAL Sampling Group[b] | 57.3 ± 9.0 | 2.98 ± 0.06 | 182.4 ± 28.7 | 0.96 ± 0.13 | 15.6 ± 2.8 | 11.2 ± 1.9 |

Data are expressed as mean ± SD.
a. 25 subjects per parameter estimate
b. 5 subjects per parameter estimate

**TABLE** 10. NONCOMPARTMENTAL PHARMACOKINETICS PARAMETERS IN PLASMA OF COMPOUND I 2 G EVERY 8 HOURS FOR 3 DOSES

| | $C_{max}$ (μg/mL) | $T_{max}$ (hours) | $AUC_{0-8}$ (μg·hr/mL) | $t_{1/2}$ (hours) | $V_{ss}$ (Liters) | CL (L/hr) |
|---|---|---|---|---|---|---|
| All Subjects[a] | 59.0 ± 8.4 | 2.98 ± 0.06 | 204.2 ± 34.6 | 1.27 ± 0.21 | 17.6 ± 2.6 | 10.1 ± 1.9 |
| 1.5-hour BAL Sampling Group[b] | 56.1 ± 13.0 | 2.95 ± 0.01 | 183.6 ± 38.6 | 1.18 ± 0.08 | 18.6 ± 2.3 | 11.3 ± 2.6 |
| 3.25-hour BAL Sampling Group[b] | 59.7 ± 5.8 | 3.00 ± 0.07 | 210.5 ± 32.2 | 1.26 ± 0.23 | 17.3 ± 2.3 | 9.7 ± 1.6 |

(continued)

| | $C_{max}$ (μg/mL) | $T_{max}$ (hours) | $AUC_{0-8}$ (μg·hr/mL) | $t_{1/2}$ (hours) | $V_{ss}$ (Liters) | CL (L/hr) |
|---|---|---|---|---|---|---|
| 4-hour BAL Sampling Group[b] | 60.1 ± 5.7 | 2.98 ± 0.06 | 213.7 ± 35.4 | 1.34 ± 0.26 | 16.9 ± 0.9 | 9.5 ± 1.3 |
| 6-hour BAL Sampling Group[b] | 60.9 ± 9.7 | 3.00 ± 0.07 | 215.8 ± 33.7 | 1.37 ± 0.27 | 18.1 ± 3.9 | 9.5 ± 1.6 |
| 8-hour BAL Sampling Group[b] | 57.9 ± 8.8 | 2.98 ± 0.06 | 197.5 ± 36.6 | 1.18 ± 0.16 | 17.0 ± 3.3 | 10.4 ± 2.0 |

Data are expressed as mean ± SD.
a. 25 subjects per parameter estimate
b. 5 subjects per parameter estimate

**TABLE 11.** MEROPENEM CONCENTRATIONS IN PLASMA, ELF, AND AM AT TIME OF BRONCHOSCOPY AND BAL

| BAL Sampling Time | Plasma (μg/mL) | ELF (μg/mL) | AM (μg/mL) |
|---|---|---|---|
| 1.5-hour | 41.2 ± 5.0 | 21.4 ± 4.0 | BQL |
| 3.25-hour | 47.7 ± 7.3 | 28.3 ± 6.7 | BQL |
| 4-hour | 23.8 ± 4.3 | 16.1 ± 4.8 | BQL |
| 6-hour | 7.24 ± 2.79 | 7.52 ± 5.29 | BQL |
| 8-hour | 1.36 ± 0.51 | 2.51 ± 1.13 | BQL |

Data are expressed as mean ± SD
5 subjects per sampling period
BQL = below quantifiable limit

**TABLE 12.** COMPOUND I CONCENTRATIONS IN PLASMA, ELF, AND AM AT TIME OF BRONCHOSCOPY AND BAL

| BAL Sampling Time | Plasma (μg/mL) | ELF (μg/mL) | AM (μg/mL) |
|---|---|---|---|
| 1.5-hour | 42.1 ± 5.0 | 18.6 ± 3.8 | 2.71 ± 1.44 |
| 3.25-hour | 51.1 ± 6.8 | 26.1 ± 1.1 | 8.79 ± 9.43 |
| 4-hour | 28.2 ± 5.3 | 15.7 ± 3.4 | 5.51 ± 3.15 |
| 6-hour | 10.8 ± 2.8 | 8.03 ± 5.80 | 27.6 ± 39.6 |
| 8-hour | 2.74 ± 1.12 | 2.61 ± 1.35 | 4.40 ± 4.10 |

Data are expressed as mean ± SD
5 subjects per sampling period

**TABLE 13.** RATIOS OF ELF TO TOTAL PLASMA CONCENTRATIONS OF MEROPENEM

| BAL Sampling Time | ELF to Plasma |
|---|---|
| 1.5-hour | 0.525 ± 0.107 |
| 3.25-hour | 0.590 ± 0.079 |
| 4-hour | 0.705 ± 0.302 |
| 6-hour | 1.037 ± 0.475 |
| 8-hour | 2.133 ± 1.366 |

Data are expressed as mean ± SD
5 subjects per sampling period

**TABLE 14.** RATIOS OF ELF AND AM TO TOTAL PLASMA CONCENTRATIONS OF COMPOUND I

| BAL Sampling Time | ELF to Plasma | AM to Plasma |
|---|---|---|
| 1.5-hour | 0.450 ± 0.123 | 0.062 ± 0.029 |
| 3.25-hour | 0.508 ± 0.096 | 0.165 ± 0.163 |
| 4-hour | 0.570 ± 0.159 | 0.191 ± 0.101 |
| 6-hour | 0.705 ± 0.329 | 2.58 ± 3.57 |
| 8-hour | 1.009 ± 0.391 | 1.603 ± 1.103 |

Data are expressed as mean ± SD
5 subjects per sampling period

Example 7

Example 7 provides a summary of a Hollow-Fiber Model study of the pharmacokinetic profiles of the combination of Compound I and meropenem in two different dosing regimens (2g meropenem/2g Compound I and 1g meropenem/1g Compound I) given every 8 hours by 3-hour infusion. The combination is highly active against gram-negative pathogens, including KPC-producing, carbapenem-resistant Enterobacteriaceae *K. pneumonia* and *P. aeruginosa*. The objective of this study was to demonstrate the efficacy of meropenem in combination with Compound I against clinical isolates of *P. aeruginosa* using simulated human exposures in an in vitro hollow fiber model. The pharmacokinetics simulation was based on data from the clinical study disclosed in Example 2.

Methods: Three *P. aeruginosa* strains were tested. The minimal inhibitory concentrations (MICs) were determined by broth microdilution assay using to CLSI reference methods and are shown in Table D.

| Table D. Bacterial Strains Used In These Studies | | |
|---|---|---|
| Strain | Meropenem MIC (mg/L) | Meropenem (w/ 8 mg/L Compound I) MIC (mg/L) |
| *P. aeruginosa* PAM3210 | 2 | 2 |
| *P. aeruginosa* PAM3377 | 4-8 | 4-8 |
| *P. aeruginosa* PAM3353 | 8 | 8 |

In Vitro PK-PD Model: Six medium sized hollow-fiber cartridges (FiberCell Systems) were used per experiment. Three strains studied in duplicate were used for each experiment. Log-phase cells were inoculated and incubated for 2 hours prior to the start of treatment to achieve about $10^8$ CFU/mL. Target PK parameters are listed in Tables E and F. The exposures were based on the published literatures disclosed in Example 2. Samples were collected from the central compartment for the determination of drug concentrations over a 32 hour period and were analyzed using an LC-MS/MS method.

| Table E. Meropenem Pharmacokinetic Parameters | | |
|---|---|---|
| **PK Parameters** | **Meropenem Target** | **Average Meropenem Actual** |
| Half-Life (hrs) | 1.33 | 1.3 |
| Cmax (mg/L) | 39 | 33.9 |
| AUC (mg*h/L) | 140 | 129.0 |

| Table F. The Combination Compound I/Meropenem Pharmacokinetic Parameters | | | | |
|---|---|---|---|---|
| **PK Parameters** | **Meropenem Target** | **Average Meropenem Actual** | **Compound I Target** | **Average Compound I Actual** |
| Half-Life (hrs) | 1.33 | 1.4 | 1.52 | 1.5 |
| Cmax (mg/L) | 39 | 33.5 | 30 | 26.4 |

(continued)

| Table F. The Combination Compound I/Meropenem Pharmacokinetic Parameters | | | | |
|---|---|---|---|---|
| **PK Parameters** | **Meropenem Target** | **Average Meropenem Actual** | **Compound I Target** | **Average Compound I Actual** |
| AUC (mg*h/L) | 140 | 131.5 | 106 | 105.3 |

Klebsiella pneumoniae carbapenemase (KPC)-producing strains of *Enterobacteriaceae* with meropenem alone MIC ranging from 8 to 512 μg/ml and with meropenem/Compound I (wherein Compound I was administered at fixed concentration of 8 μg/ml with meropenem, the MIC meropenem ranges from ≤0.06 to 8 μg/ml) as well as *P. aeruginosa* strains with meropenem and meropenem/Compound I MIC 2-8 μg/ml were used.

Results:

Exposure from the combination of 1g meropenem and 1g Compound I dosing regimen was associated with effective killing and no regrowth at 32 hours of KPC-producing strains of *K. pneumonia* with meropenem alone (MIC ranging from 8 to 64 μg/ml) and with the combination of meropenem and Compound I (where Compound I was administered at the fixed concentration of 4 μg/ml with meropenem, the MIC of meropenem ranges from from ≤0.06 to 2 μg/ml) (*see* **FIG. 22** and **FIG. 23).** Several clones of the strains KP1061, KP1087, KP1004 and KP1074 that survived at 32 hours were tested for susceptibility to meropenem and meropenem/Compound I combination and were found to be indistinguishable from the pre-exposed strains.

On the other hand, less killing was observed for the strain KP1099 with meropenem alone (MIC is 128 μg/ml) and the combination of meropenem and Compound I (when Compound I was administered at the fixed concentration of 4 μg/ml, the MIC of meropenem reduced to 4 μg/ml). *See* **FIG. 23.** Regrowth was observed after 16 hours from the start of treatment. When colonies of KP1099 that survived exposure to three doses of 1g meropenem/1 g Compound I were investigated, their susceptibility to meropenem/Compound I was reduced 16-32-fold indicating selection of resistance under the conditions of inadequate exposure.

Importantly, exposure from 2g meropenem/2g Compound I dosing regimen was associated with efficient killing and no regrowth/resistance development using strains with meropenem alone and meropenem/Compound I. For the strain KP1094, MIC for meropenem alone was as high as 512 μg/ml. However, when Compound I was administered at the fixed concentration of 8 μg/ml with meropenem, the observed MIC of meropenem was reduced to 8 μg/ml (*see* **FIG. 24).**

Exposure from 1g meropenem/1g Compound I dosing regimen resulted in effective killing and no regrowth at 32 hours due to resistance development for the strain of *P. aeruginosa* PAM3210 with meropenem and meropenem/Compound I (when Compound I was administered at the fixed concentration of 4 μg/ml or 8 μg/ml, the MIC of meropenem remains 2 μg/ml. However, regrowth and resistance development occurred in the strains PAM3353 and PAM3377with an MIC of 8 μg/ml for meropenem (*see* **FIG. 25).**

For the efficacy of simulated human exposures of meropenem compared to the combination of Compound I 2g/meropenem 2g against *Pseudomonas aeruginosa* in the in vitro hollow fiber model, it was observed that the model effectively simulated human exposures of both meropenem and Compound I. (*See* **FIG. 26).** Antibacterial activity of meropenem in the model is shown in **FIG. 27.** Meropenem 2g q8h by 3 hour infusion produced over 4 logs of bacterial killing against the strain with an MIC of 2 mg/L, almost 4 logs of killing against the strain with an MIC of 4 - 8 mg/L. Resistance developed in the strain with an MIC of 8 mg/L. Antibacterial activity of the combination of 2g meropenem/2g Compound I in the model is shown in **FIG. 28.** The combination produced over 4 logs of bacterial killing against all strains tested with no regrowth or resistance development over the 32 hour test period. 2g meropenem/2g Compound I dosing regimen was efficacious against all three strains. No resistant mutants were identified among surviving bacterial (*see* **FIG. 28).** The results are summarized in Table G below.

| Table G. | | | |
|---|---|---|---|
| | **MIC μg/mL** | **Human Equivalent Dosage Regimen** | **Change in Log CFU over 32 hours** |
| *P. aeruginosa* PAM3210 | | | |
| Meropenem | 2 | 2g q8h by 3 hour infusion | >4 |

(continued)

| Table G. | | | |
|---|---|---|---|
| | MIC μg/mL | Human Equivalent Dosage Regimen | Change in Log CFU over 32 hours |
| Meropenem/Compound I | 2 | 2g/2g q8h by 3 hour infusion | >4 |
| *P. aeruginosa* PAM3377 | | | |
| Meropenem | 4-8 | 2g q8h by 3 hour infusion | 3.7 |
| Meropenem/Compound I | 4-8 | 2g/2g q8h by 3 hour infusion | >4 |
| *P. aeruginosa* PAM3353 | | | |
| Meropenem | 8 | 2g q8h by 3 hour infusion | 1.3* |
| Meropenem/Compound I | 8 | 2g/2g q8h by 3 hour infusion | >4 |
| * Resistance Developed | | | |

In conclusion, the PK/PD studies in *in vitro* models of infections demonstrate that the human exposures from 2g/2g combination of meropenem/Compound I are associated with extensive killing of target pathogens and prevention of resistance for the strains with Compound I at fixed 8 μg/ml and meropenem MIC less or equal to 8 μg/ml. In addition, the 2g/2g dose combination reduced exposures that are associated with resistance development.

In addition, the combination of Compound I 2g/meropenem 2g administered every 8 hours by three hour infusion was highly efficacious in this in vitro model against *P. aeruginosa* strains with MICs as high as 8 mg/L, with no regrowth and no resistance development over the course of the 32 hour study. Meropenem 2g q8h by 3 hour infusion was effective against 2 out of 3 strains, but resistance developed in the third strain with an MIC of 8 mg/L.

**Claims**

1. Compound I or a pharmaceutically acceptable salt thereof and meropenem for use in a method of treating or ameliorating a bacterial infection in a subject suffering from reduced renal function:

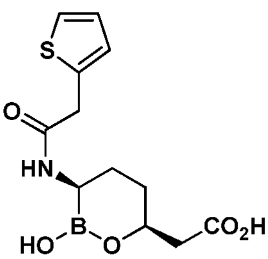 (Compound I),

wherein:

Compound I or the pharmaceutically acceptable salt thereof and meropenem are administered after a dialysis session and wherein:

(i) the subject has a creatinine clearance level of equal or greater than 30 ml/min and less than 50 ml/min, and wherein the amount of Compound I or the pharmaceutically acceptable salt thereof is about 1.0 g and the amount of meropenem is about 1.0 g, and wherein Compound I or the pharmaceutically acceptable salt thereof and meropenem are administered every 8 hours (q8h); or,

(ii) the subject has a creatinine clearance level of equal or greater than 20 ml/min and less than 30 ml/min, and wherein the amount of Compound I or the pharmaceutically acceptable salt thereof is about 1.0 g and the amount of meropenem is about 1.0 g, and wherein Compound I or the pharmaceutically acceptable salt thereof

and meropenem are administered every 12 hours (q12h); or

(iii) the subject has a creatinine clearance of equal or greater than 10 ml/min and less than 20 ml/min, and wherein the amount of Compound I or the pharmaceutically acceptable salt thereof is about 0.5 g and the amount of meropenem is about 0.5 g, and wherein Compound I or the pharmaceutically acceptable salt thereof and meropenem are administered every 12 hours (q12h); or

(iv) the subject has a creatinine clearance of less than 10 ml/min, and wherein the amount of Compound I or the pharmaceutically acceptable salt thereof is about 0.5 g and the amount of meropenem is about 0.5 g, and wherein Compound I or the pharmaceutically acceptable salt thereof and meropenem are administered every 24 hours (q24h).

**2.** Compound I or a pharmaceutically acceptable salt thereof and meropenem for use according to claim 1, wherein the composition is administered by intravenous infusion; and optionally,
wherein the intravenous infusion is completed in about 3 hours.

**3.** Compound I or a pharmaceutically acceptable salt thereof and meropenem for use according to any one of claims 1 to 2, wherein Compound I or the pharmaceutically acceptable salt thereof is administered prior or subsequent to meropenem.

**4.** Compound I or a pharmaceutically acceptable salt thereof and meropenem for use according to any one of claims 1 to 3, wherein Compound I or the pharmaceutically acceptable salt thereof and meropenem are:

(i) formulated into a pharmaceutical composition; or

(ii) in a single dosage form, optionally, wherein the single dosage form further comprises a pharmaceutically acceptable excipient, diluent, or carrier.

**5.** Compound I or a pharmaceutically acceptable salt thereof and meropenem for use according to any one of claims 1 to 4, wherein said method further comprises administering an additional medicament selected from an antibacterial agent, an antifungal agent, an antiviral agent, an anti-inflammatory agent, or an anti-allergic agent.

**6.** Compound I or a pharmaceutically acceptable salt thereof and meropenem for use according to any one of claims 1 to 5, wherein the subject is suffering from a lower-respiratory tract infection.

**7.** Compound I or a pharmaceutically acceptable salt thereof and meropenem for use according to any one of claims 1 to 6, wherein the infection is caused by carbapenem-resistant *enterobacteriaceae.*

**8.** Compound I or a pharmaceutically acceptable salt thereof and meropenem for use according to claim 7, wherein the bacterial infection is caused by *Klebsiella pneumoniae, Escherichia coli,* or *Enterobacter cloacae,* or combinations thereof.

## Patentansprüche

**1.** Verbindung I oder ein pharmazeutisch unbedenkliches Salz davon und Meropenem zur Verwendung bei einem Verfahren zur Behandlung oder Besserung einer bakteriellen Infektion bei einem Individuum, das an reduzierter Nierenfunktion leidet:

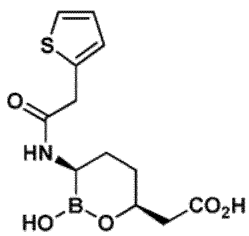

(Verbindung I),

wobei:
Verbindung I oder das pharmazeutisch unbedenkliche Salz davon und Meropenem nach einer Dialysesitzung verabreicht werden und wobei:

(i) das Individuum ein Kreatinin-Clearance-Niveau von gleich oder größer als 30 ml/min und weniger als 50 ml/min aufweist und wobei die Menge an Verbindung I oder dem pharmazeutisch unbedenklichen Salz davon etwa 1,0 g beträgt und die Menge an Meropenem etwa 1,0 g beträgt und wobei Verbindung I oder das pharmazeutisch unbedenkliche Salz davon und Meropenem alle 8 Stunden (q8h) verabreicht werden oder
(ii) das Individuum ein Kreatinin-Clearance-Niveau von gleich oder größer als 20 ml/min und weniger als 30 ml/min aufweist und wobei die Menge an Verbindung I oder dem pharmazeutisch unbedenklichen Salz davon etwa 1,0 g beträgt und die Menge an Meropenem etwa 1,0 g beträgt und wobei Verbindung I oder das pharmazeutisch unbedenkliche Salz davon und Meropenem alle 12 Stunden (q12h) verabreicht werden oder
(iii) das Individuum ein Kreatinin-Clearance-Niveau von gleich oder größer als 10 ml/min und weniger als 20 ml/min aufweist und wobei die Menge an Verbindung I oder dem pharmazeutisch unbedenklichen Salz davon etwa 0,5 g beträgt und die Menge an Meropenem etwa 0,5 g beträgt und wobei Verbindung I oder das pharmazeutisch unbedenkliche Salz davon und Meropenem alle 12 Stunden (q12h) verabreicht werden oder
(iv) das Individuum ein Kreatinin-Clearance-Niveau von weniger als 10 ml/min aufweist und wobei die Menge an Verbindung I oder dem pharmazeutisch unbedenklichen Salz davon etwa 0,5 g beträgt und die Menge an Meropenem etwa 0,5 g beträgt und wobei Verbindung I oder das pharmazeutisch unbedenkliche Salz davon und Meropenem alle 24 Stunden (q24h) verabreicht werden.

**2.** Verbindung I oder ein pharmazeutisch unbedenkliches Salz davon und Meropenem zur Verwendung nach Anspruch 1, wobei die Zusammensetzung durch intravenöse Infusion verabreicht wird; und gegebenenfalls
wobei die intravenöse Infusion in etwa 3 Stunden abgeschlossen ist.

**3.** Verbindung I oder ein pharmazeutisch unbedenkliches Salz davon und Meropenem zur Verwendung nach einem der Ansprüche 1 bis 2, wobei Verbindung I oder das pharmazeutisch unbedenkliche Salz davon vor oder nach Meropenem verabreicht wird.

**4.** Verbindung I oder ein pharmazeutisch unbedenkliches Salz davon und Meropenem zur Verwendung nach einem der Ansprüche 1 bis 3, wobei Verbindung I oder das pharmazeutisch unbedenkliche Salz davon und Meropenem:

(i) in einer pharmazeutischen Zusammensetzung formuliert sind oder
(ii) in einer Einzeldosierungsform formuliert sind, gegebenenfalls wobei die Einzeldosierungsform einen pharmazeutisch unbedenklichen Hilfsstoff, ein pharmazeutisch unbedenkliches Verdünnungsmittel oder einen pharmazeutisch unbedenklichen Träger umfasst.

**5.** Verbindung I oder ein pharmazeutisch unbedenkliches Salz davon und Meropenem zur Verwendung nach einem der Ansprüche 1 bis 4, wobei das Verfahren ferner das Verabreichen eines zusätzlichen Medikaments, das aus einem antibakteriellen Mittel, einem Antimykotikum, einem antiviralen Mittel, einem entzündungshemmenden Mittel oder einem Antiallergikum ausgewählt ist, umfasst.

**6.** Verbindung I oder ein pharmazeutisch unbedenkliches Salz davon und Meropenem zur Verwendung nach einem der Ansprüche 1 bis 5, wobei das Individuum an einer Infektion der unteren Atemwege leidet.

**7.** Verbindung I oder ein pharmazeutisch unbedenkliches Salz davon und Meropenem zur Verwendung nach einem der Ansprüche 1 bis 6, wobei die Infektion durch Carbapenem-resistente *Enterobacteriaceae* verursacht wird.

**8.** Verbindung I oder ein pharmazeutisch unbedenkliches Salz davon und Meropenem zur Verwendung nach Anspruch 7, wobei die bakterielle Infektion durch *Klebsiella pneumoniae, Escherichia coli* oder *Enterobacter cloacae* oder Kombinationen davon verursacht wird.

## Revendications

**1.** Composé I ou sel pharmaceutiquement acceptable correspondant et méropénem pour une utilisation dans un procédé de traitement ou d’amélioration d’une infection bactérienne chez un sujet souffrant d’une fonction rénale réduite :

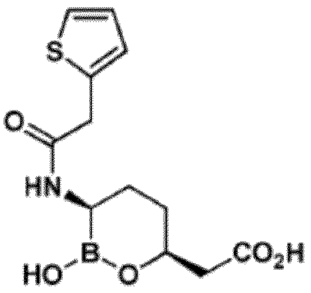

(composé I),

le composé I ou le sel pharmaceutiquement acceptable correspondant et le méropénem étant administrés après une session de dialyse et :

(i) le sujet présentant un taux de clairance de créatinine égal ou supérieur à 30 ml/min et inférieur à 50 ml/min, et la quantité de composé I ou du sel pharmaceutiquement acceptable correspondant étant d'environ 1,0 g et la quantité de méropénem étant d'environ 1,0 g et le composé I ou le sel pharmaceutiquement acceptable correspondant et le méropénem étant administrés toutes les 8 heures (q8h) ; ou,
(ii) le sujet présentant un taux de clairance de créatinine égal ou supérieur à 20 ml/min et inférieur à 30 ml/min, et la quantité de composé I ou du sel pharmaceutiquement acceptable correspondant étant d'environ 1,0 g et la quantité de méropénem étant d'environ 1,0 g et le composé I ou le sel pharmaceutiquement acceptable correspondant et le méropénem étant administrés toutes les 12 heures (q12h) ; ou
(iii) le sujet présentant un taux de clairance de créatinine égal ou supérieur à 10 ml/min et inférieur à 20 ml/min, et la quantité de composé I ou du sel pharmaceutiquement acceptable correspondant étant d'environ 0,5 g et la quantité de méropénem étant d'environ 0,5 g et le composé I ou le sel pharmaceutiquement acceptable correspondant et le méropénem étant administrés toutes les 12 heures (q12h) ; ou
(iv) le sujet présentant un taux de clairance de créatinine inférieur à 10 ml/min, et la quantité de composé I ou du sel pharmaceutiquement acceptable correspondant étant d'environ 0,5 g et la quantité de méropénem étant d'environ 0,5 g et le composé I ou le sel pharmaceutiquement acceptable correspondant et le méropénem étant administrés toutes les 24 heures (q24h).

**2.** Composé I ou sel pharmaceutiquement acceptable correspondant et méropénem pour une utilisation selon la revendication 1, la composition étant administrée par perfusion intraveineuse ; et éventuellement,
la perfusion intraveineuse étant terminée en environ 3 heures.

**3.** Composé I ou sel pharmaceutiquement acceptable correspondant et méropénem pour une utilisation selon l'une quelconque des revendications 1 à 2, le composé I ou le sel pharmaceutiquement acceptable correspondant étant administré avant ou après le méropénem.

**4.** Composé I ou sel pharmaceutiquement acceptable correspondant et méropénem pour une utilisation selon l'une quelconque des revendications 1 à 3, le composé I ou le sel pharmaceutiquement acceptable correspondant et le méropénem étant :

(i) formulés en une composition pharmaceutique ; ou
(ii) en une forme de dosage unique, éventuellement, la forme de dosage unique comprenant en outre un excipient, diluant ou support pharmaceutiquement acceptable.

**5.** Composé I ou sel pharmaceutiquement acceptable correspondant et méropénem pour une utilisation selon l'une quelconque des revendications 1 à 4, ledit procédé comprenant en outre l'administration d'un médicament supplémentaire choisi parmi un agent antibactérien, un agent antifongique, un agent antiviral, un agent anti-inflammatoire ou un agent antiallergique.

**6.** Composé I ou sel pharmaceutiquement acceptable correspondant et méropénem pour une utilisation selon l'une quelconque des revendications 1 à 5, le sujet souffrant d'une infection des voies respiratoires inférieures.

**7.** Composé I ou sel pharmaceutiquement acceptable correspondant et méropénem pour une utilisation selon l'une quelconque des revendications 1 à 6, l'infection étant causée par des *enterobacteriaceae* résistantes aux carbapénèmes.

**8.** Composé I ou sel pharmaceutiquement acceptable correspondant et méropénem pour une utilisation selon la revendication 7, l'infection bactérienne étant causée par *Klebsiella pneumoniae, Escherichia coli,* ou *Enterobacter cloacae,* ou des combinaisons correspondantes.

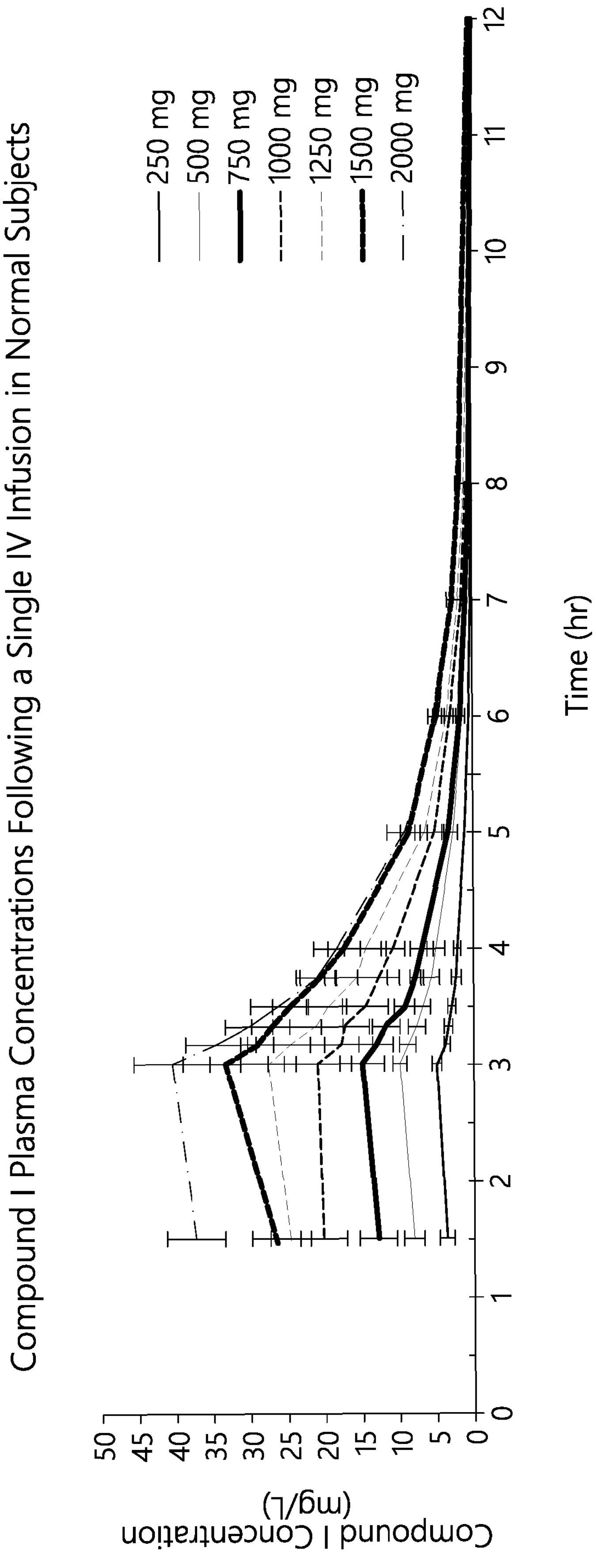
Compound I Plasma Concentrations Following a Single IV Infusion in Normal Subjects
250 mg
500 mg
750 mg
1000 mg
1250 mg
1500 mg
2000 mg
Compound I Concentration (mg/L)
0
5
10
15
20
25
30
35
40
45
50
Time (hr)
0
1
2
3
4
5
6
7
8
9
10
11
12

FIG. 1

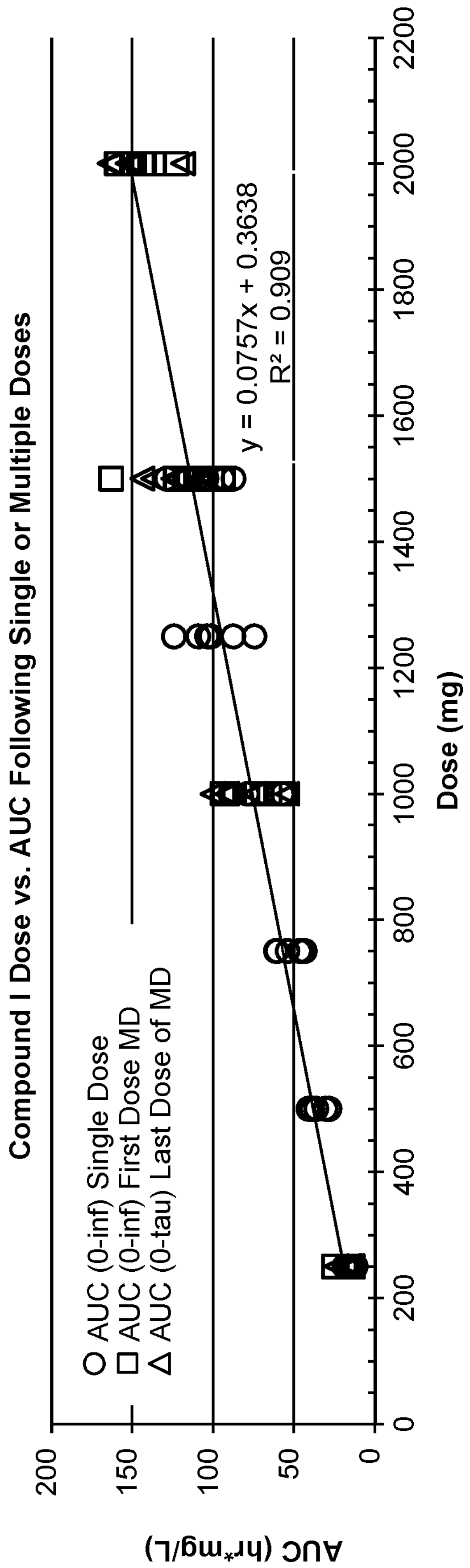
Compound I Dose vs. AUC Following Single or Multiple Doses
○ AUC (0-inf) Single Dose
□ AUC (0-inf) First Dose MD
Δ AUC (0-tau) Last Dose of MD
y = 0.0757x + 0.3638
R² = 0.909
AUC (hr*mg/L)
200
150
100
50
0
Dose (mg)
0
200
400
600
800
1000
1200
1400
1600
1800
2000
2200

FIG. 2

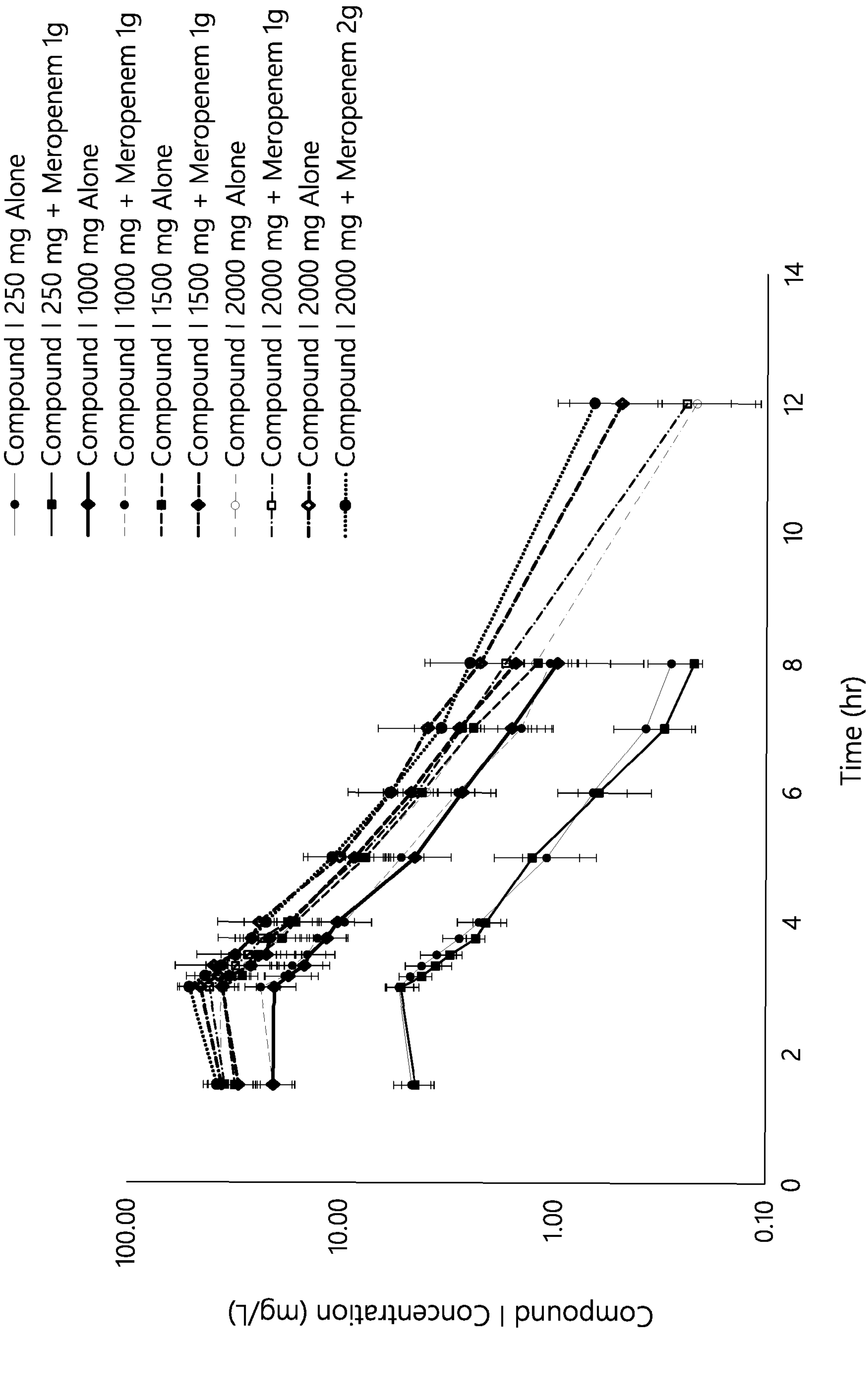
Compound I 250 mg Alone
Compound I 250 mg + Meropenem 1g
Compound I 1000 mg Alone
Compound I 1000 mg + Meropenem 1g
Compound I 1500 mg Alone
Compound I 1500 mg + Meropenem 1g
Compound I 2000 mg Alone
Compound I 2000 mg + Meropenem 1g
Compound I 2000 mg Alone
Compound I 2000 mg + Meropenem 2g
Compound I Concentration (mg/L)
100.00
10.00
1.00
0.10
0
2
4
6
8
10
12
14
Time (hr)

FIG. 3

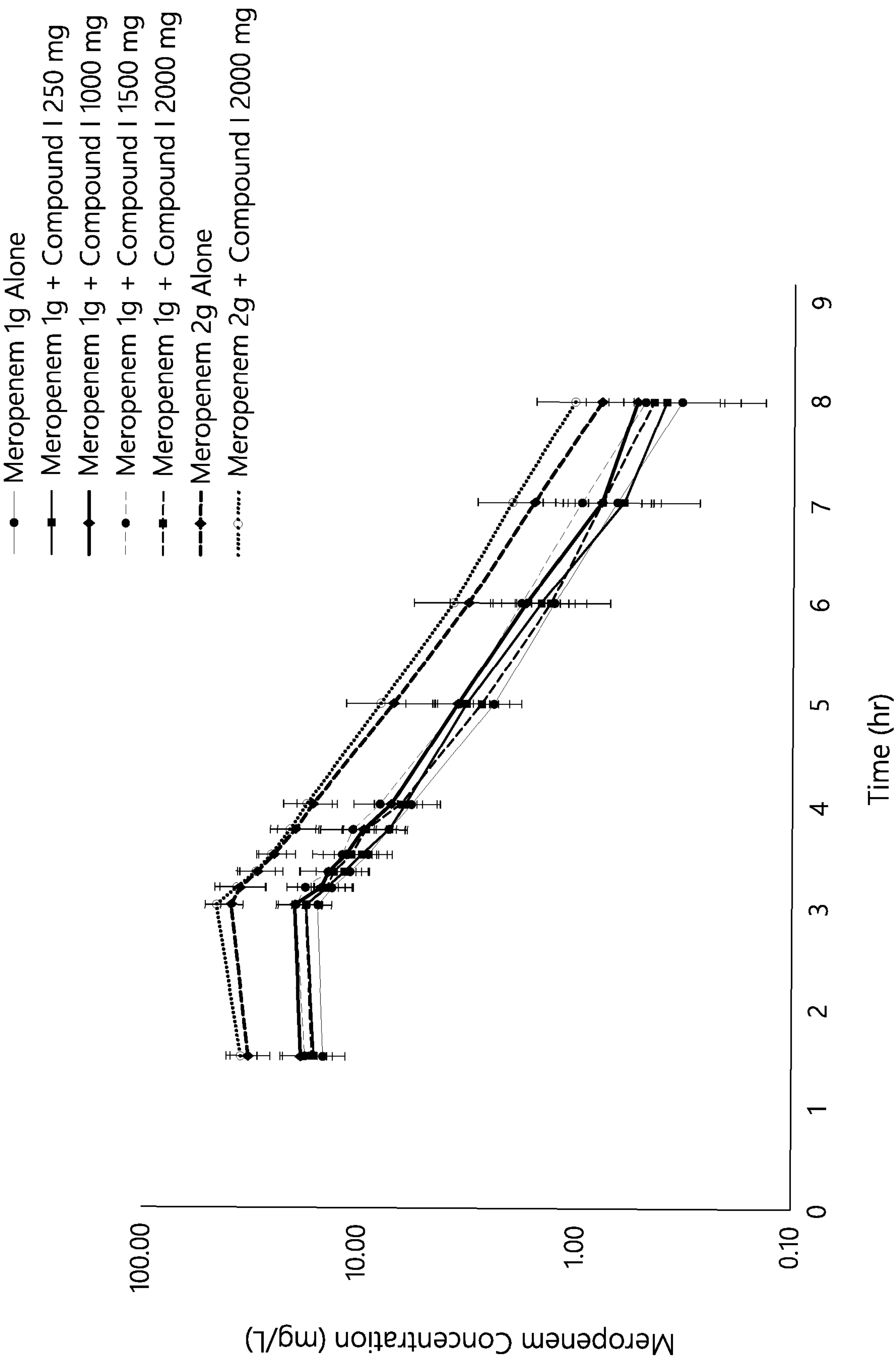
Meropenem 1g Alone
Meropenem 1g + Compound I 250 mg
Meropenem 1g + Compound I 1000 mg
Meropenem 1g + Compound I 1500 mg
Meropenem 1g + Compound I 2000 mg
Meropenem 2g Alone
Meropenem 2g + Compound I 2000 mg
Meropenem Concentration (mg/L)
100.00
10.00
1.00
0.10
0
1
2
3
4
5
6
7
8
9
Time (hr)

FIG. 4

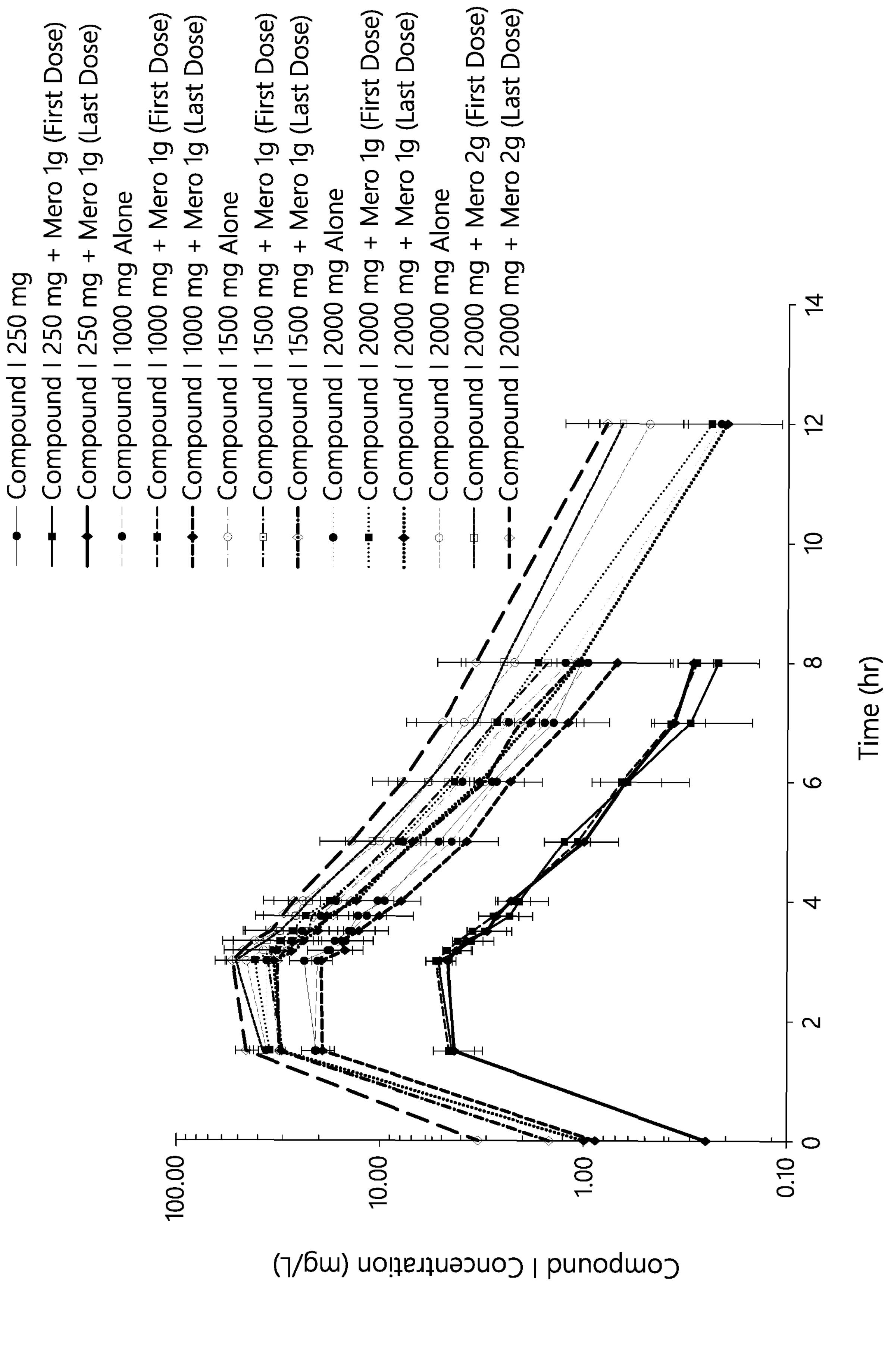
Compound I 250 mg
Compound I 250 mg + Mero 1g (First Dose)
Compound I 250 mg + Mero 1g (Last Dose)
Compound I 1000 mg Alone
Compound I 1000 mg + Mero 1g (First Dose)
Compound I 1000 mg + Mero 1g (Last Dose)
Compound I 1500 mg Alone
Compound I 1500 mg + Mero 1g (First Dose)
Compound I 1500 mg + Mero 1g (Last Dose)
Compound I 2000 mg Alone
Compound I 2000 mg + Mero 1g (First Dose)
Compound I 2000 mg + Mero 1g (Last Dose)
Compound I 2000 mg Alone
Compound I 2000 mg + Mero 2g (First Dose)
Compound I 2000 mg + Mero 2g (Last Dose)
Compound I Concentration (mg/L)
100.00
10.00
1.00
0.10
0
2
4
6
8
10
12
14
Time (hr)

FIG. 5

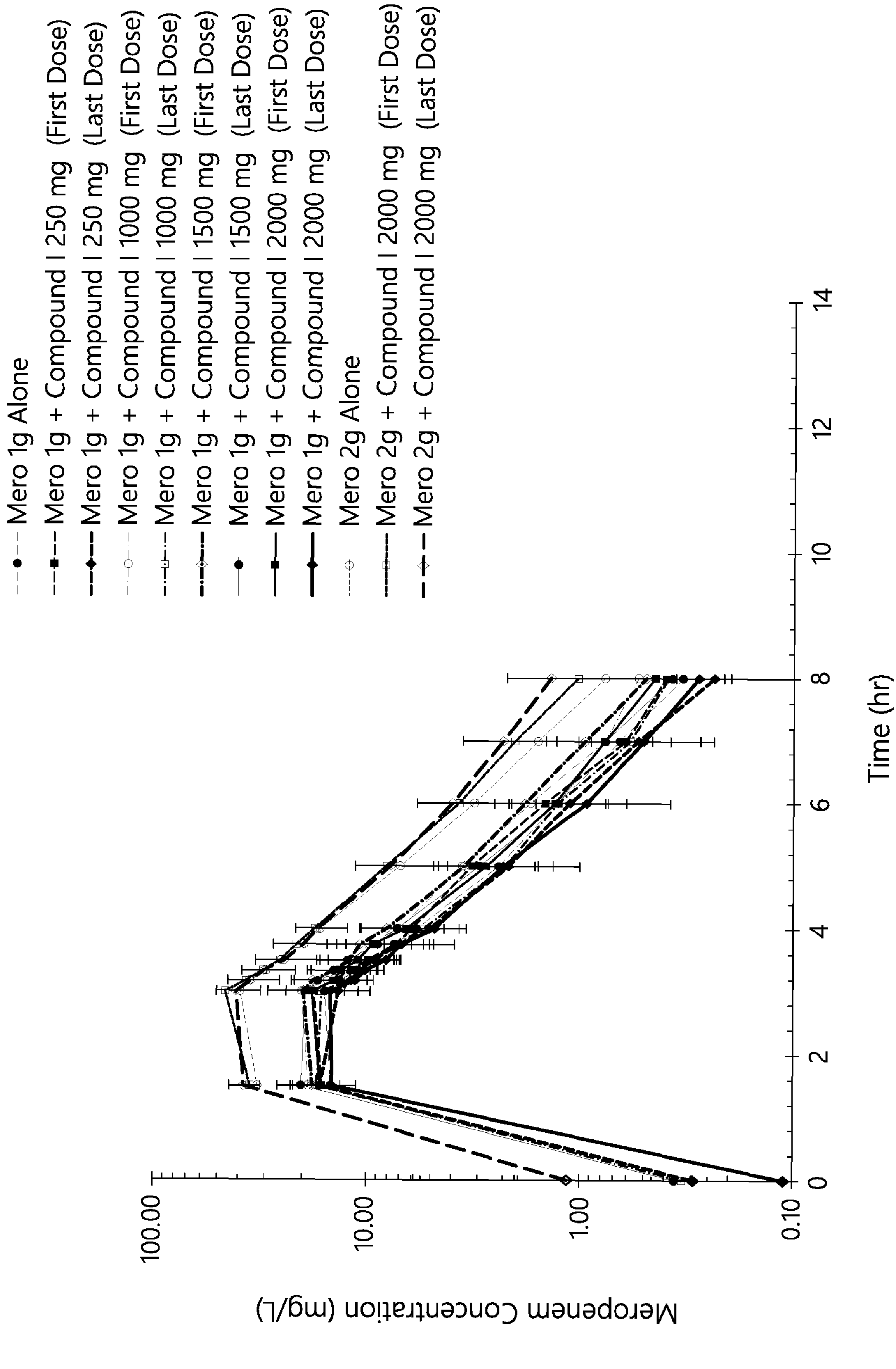
Mero 1g Alone
Mero 1g + Compound I 250 mg (First Dose)
Mero 1g + Compound I 250 mg (Last Dose)
Mero 1g + Compound I 1000 mg (First Dose)
Mero 1g + Compound I 1000 mg (Last Dose)
Mero 1g + Compound I 1500 mg (First Dose)
Mero 1g + Compound I 1500 mg (Last Dose)
Mero 1g + Compound I 2000 mg (First Dose)
Mero 1g + Compound I 2000 mg (Last Dose)
Mero 2g Alone
Mero 2g + Compound I 2000 mg (First Dose)
Mero 2g + Compound I 2000 mg (Last Dose)
Meropenem Concentration (mg/L)
100.00
10.00
1.00
0.10
0
2
4
6
8
10
12
14
Time (hr)

FIG. 6

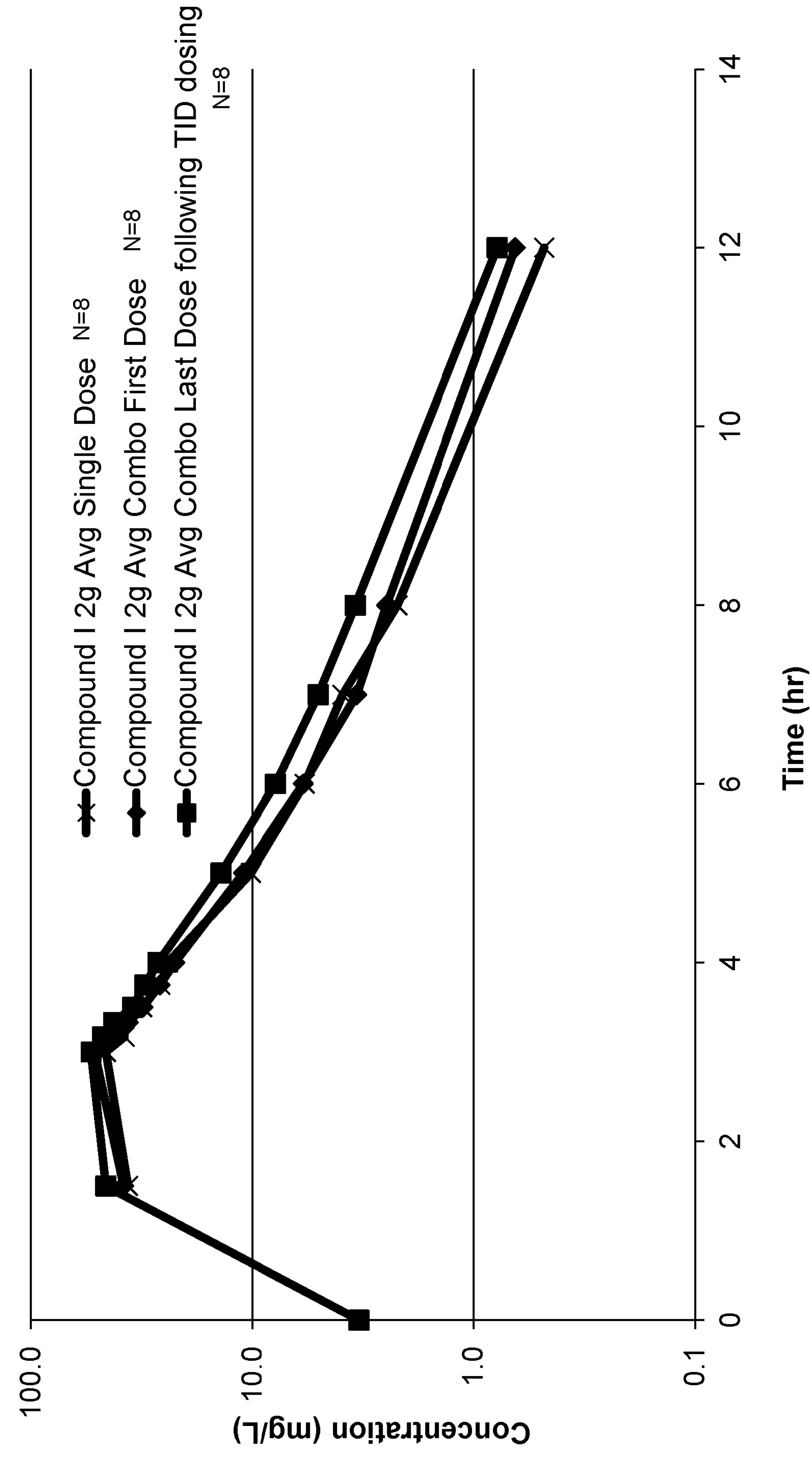
Compound I 2g Avg Single Dose N=8
Compound I 2g Avg Combo First Dose N=8
Compound I 2g Avg Combo Last Dose following TID dosing N=8
Concentration (mg/L)
100.0
10.0
1.0
0.1
Time (hr)
0
2
4
6
8
10
12
14

FIG. 7

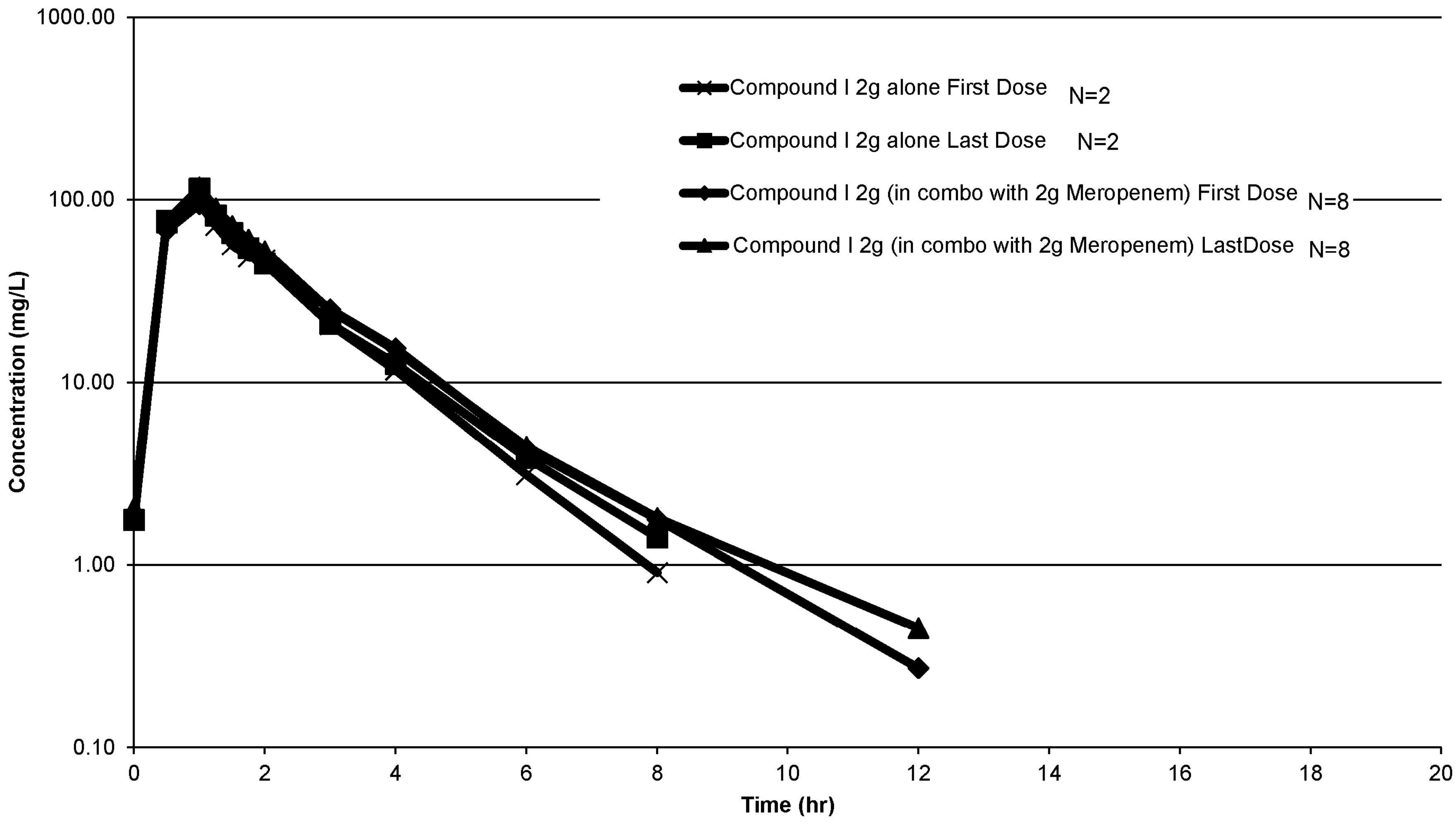

Compound I 2g alone First Dose N=2
Compound I 2g alone Last Dose N=2
Compound I 2g (in combo with 2g Meropenem) First Dose N=8
Compound I 2g (in combo with 2g Meropenem) LastDose N=8
Concentration (mg/L)
1000.00
100.00
10.00
1.00
0.10
0
2
4
6
8
10
12
14
16
18
20
Time (hr)


FIG. 8

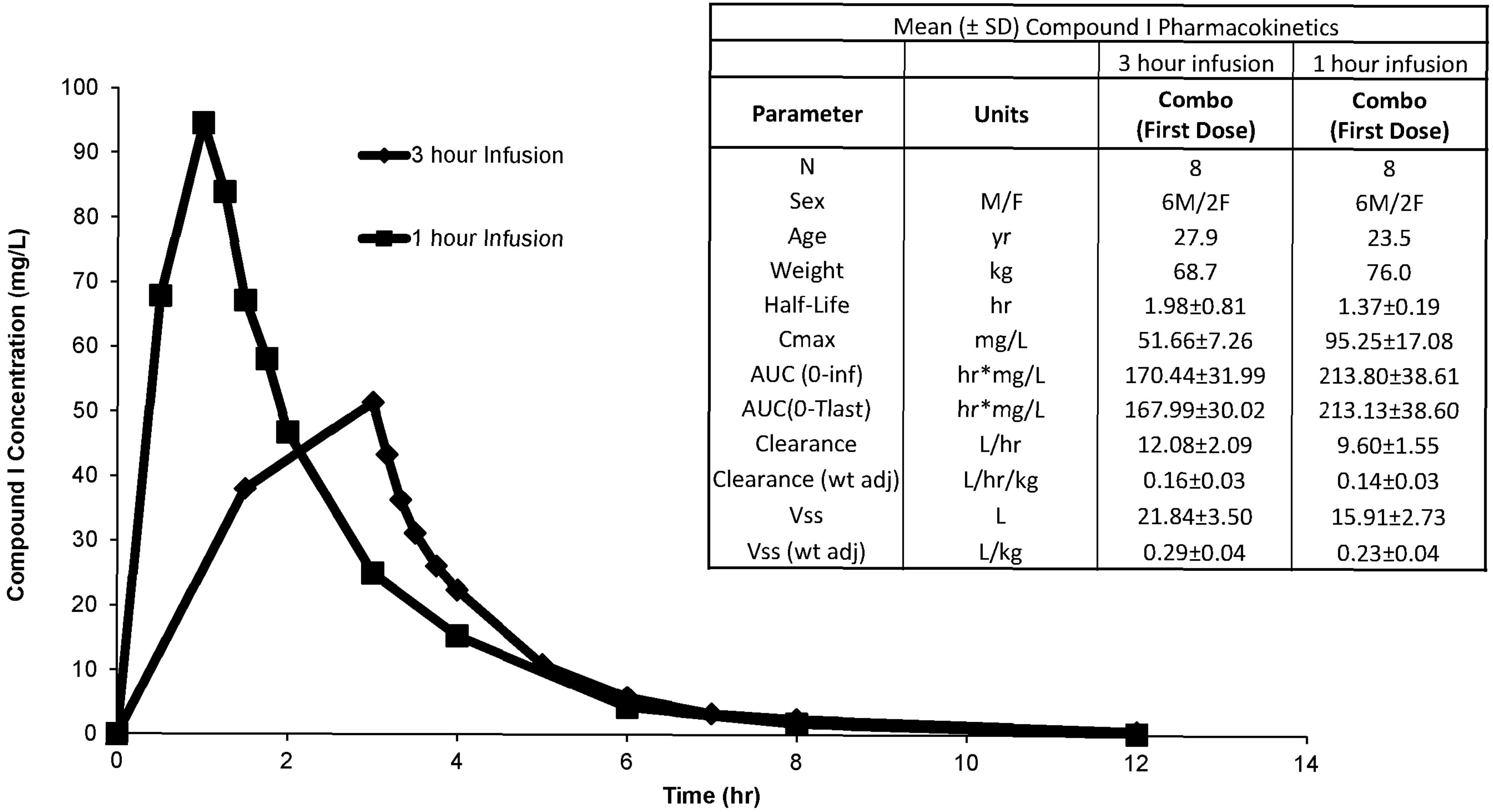


| Mean (± SD) Compound I Pharmacokinetics | | | |
|---|---|---|---|
| | | 3 hour infusion | 1 hour infusion |
| **Parameter** | **Units** | **Combo (First Dose)** | **Combo (First Dose)** |
| N | | 8 | 8 |
| Sex | M/F | 6M/2F | 6M/2F |
| Age | yr | 27.9 | 23.5 |
| Weight | kg | 68.7 | 76.0 |
| Half-Life | hr | 1.98±0.81 | 1.37±0.19 |
| Cmax | mg/L | 51.66±7.26 | 95.25±17.08 |
| AUC (0-inf) | hr*mg/L | 170.44±31.99 | 213.80±38.61 |
| AUC(0-Tlast) | hr*mg/L | 167.99±30.02 | 213.13±38.60 |
| Clearance | L/hr | 12.08±2.09 | 9.60±1.55 |
| Clearance (wt adj) | L/hr/kg | 0.16±0.03 | 0.14±0.03 |
| Vss | L | 21.84±3.50 | 15.91±2.73 |
| Vss (wt adj) | L/kg | 0.29±0.04 | 0.23±0.04 |

FIG. 9

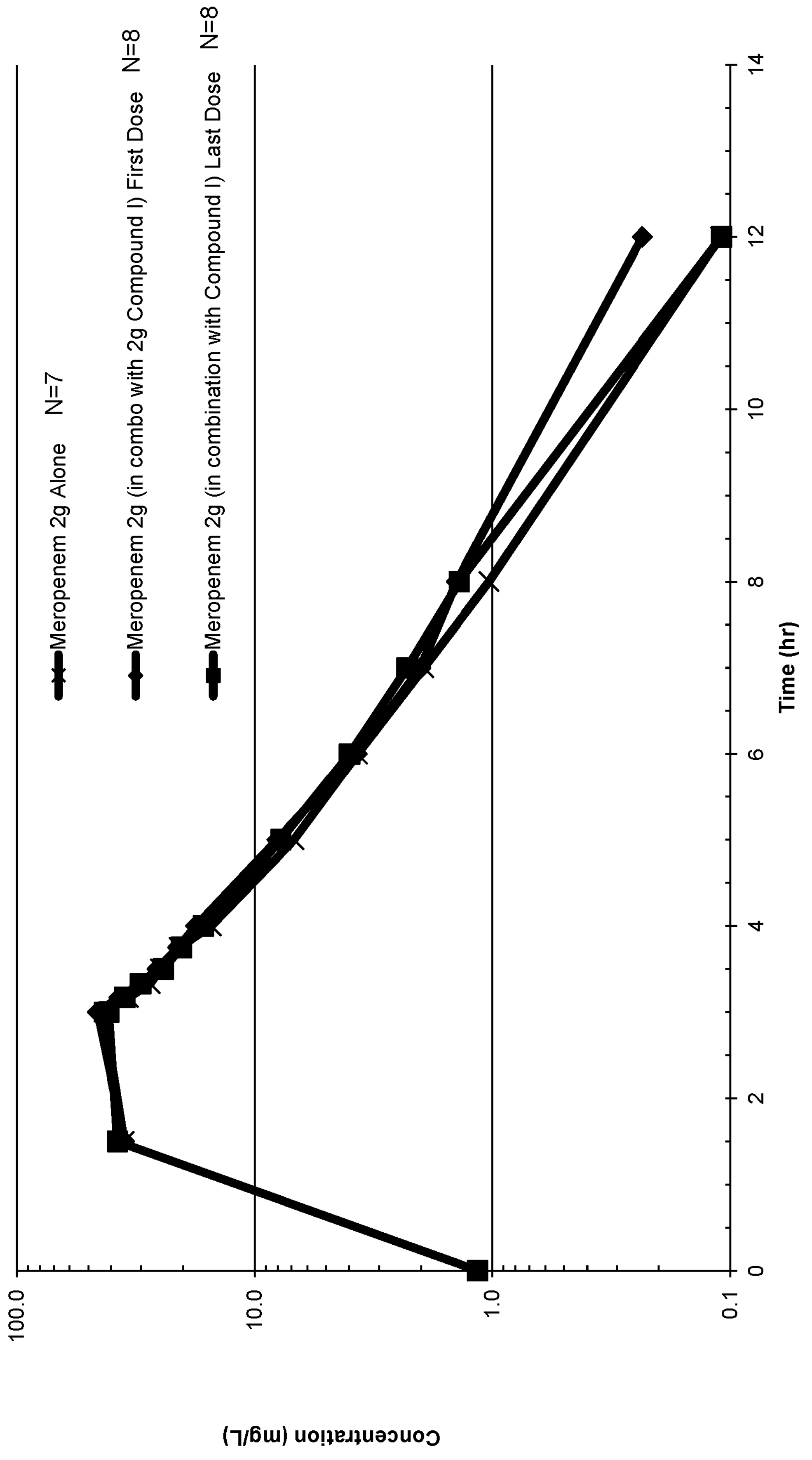
Meropenem 2g Alone N=7
Meropenem 2g (in combo with 2g Compound I) First Dose N=8
Meropenem 2g (in combination with Compound I) Last Dose N=8
Concentration (mg/L)
100.0
10.0
1.0
0.1
Time (hr)
0
2
4
6
8
10
12
14

FIG. 10

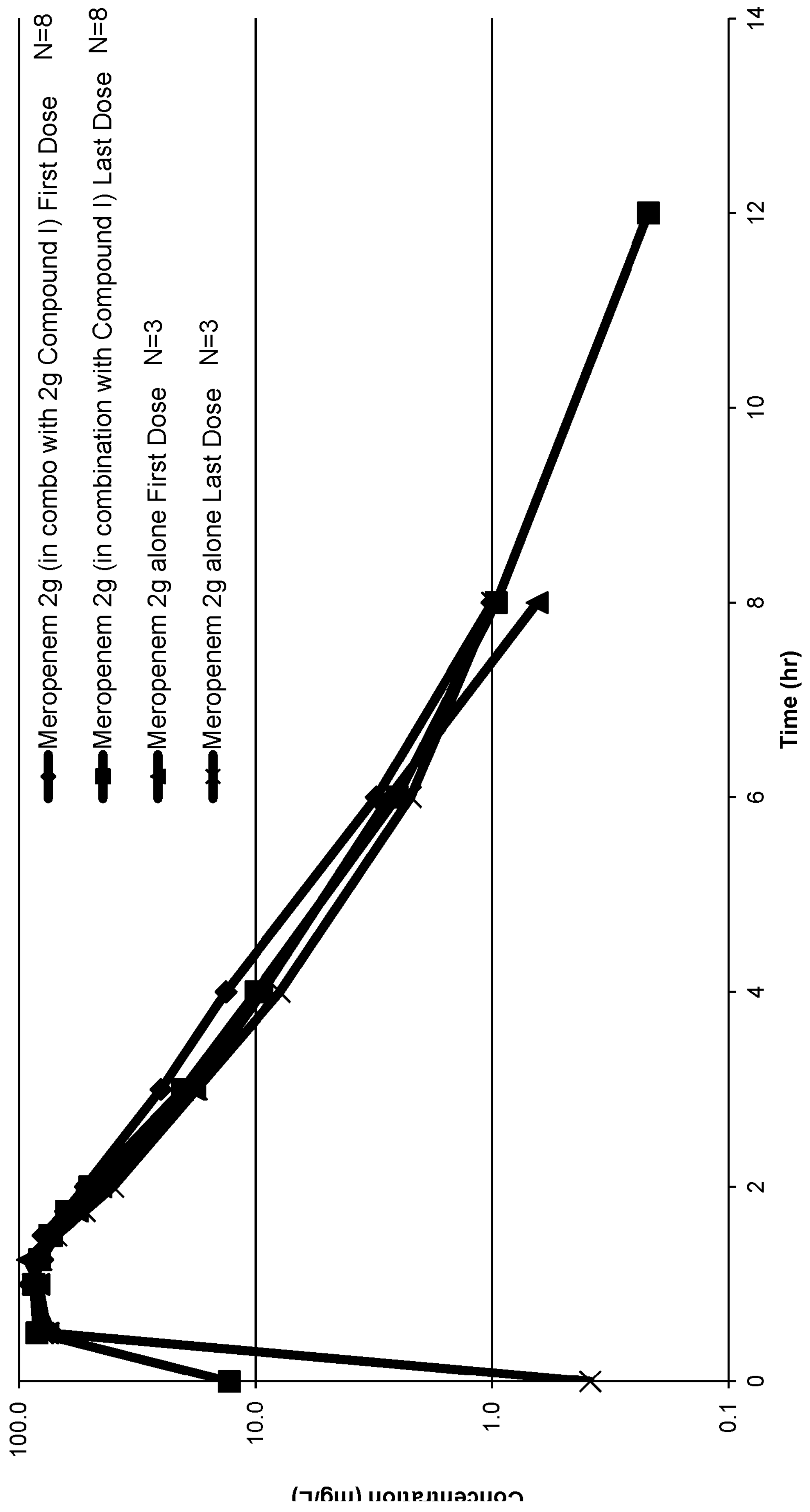
Meropenem 2g (in combo with 2g Compound I) First Dose N=8
Meropenem 2g (in combination with Compound I) Last Dose N=8
Meropenem 2g alone First Dose N=3
Meropenem 2g alone Last Dose N=3
Concentration (mg/L)
100.0
10.0
1.0
0.1
Time (hr)
0
2
4
6
8
10
12
14

FIG. 11

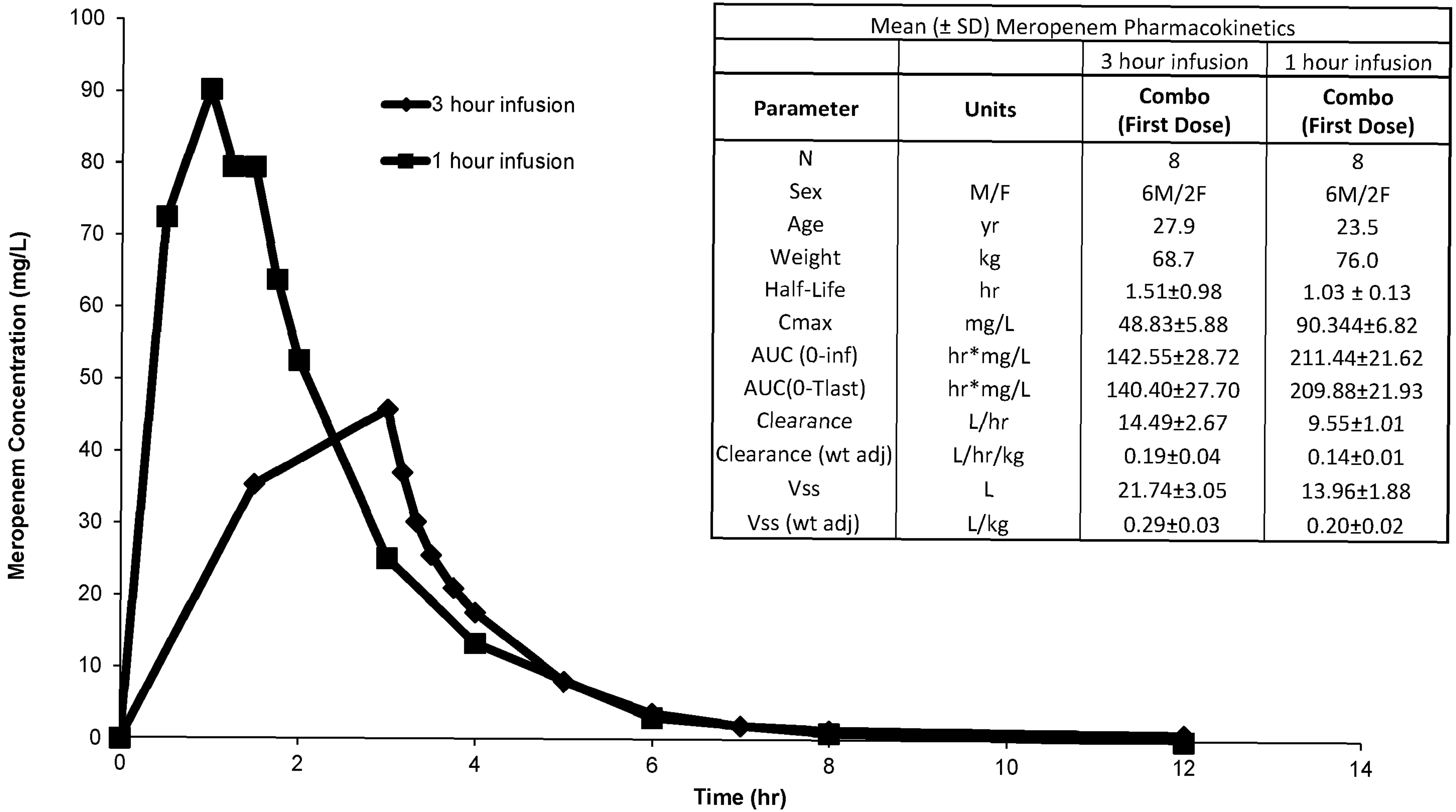


| Mean (± SD) Meropenem Pharmacokinetics | | | |
|---|---|---|---|
| | | 3 hour infusion | 1 hour infusion |
| **Parameter** | **Units** | **Combo (First Dose)** | **Combo (First Dose)** |
| N | | 8 | 8 |
| Sex | M/F | 6M/2F | 6M/2F |
| Age | yr | 27.9 | 23.5 |
| Weight | kg | 68.7 | 76.0 |
| Half-Life | hr | 1.51±0.98 | 1.03 ± 0.13 |
| Cmax | mg/L | 48.83±5.88 | 90.344±6.82 |
| AUC (0-inf) | hr*mg/L | 142.55±28.72 | 211.44±21.62 |
| AUC(0-Tlast) | hr*mg/L | 140.40±27.70 | 209.88±21.93 |
| Clearance | L/hr | 14.49±2.67 | 9.55±1.01 |
| Clearance (wt adj) | L/hr/kg | 0.19±0.04 | 0.14±0.01 |
| Vss | L | 21.74±3.05 | 13.96±1.88 |
| Vss (wt adj) | L/kg | 0.29±0.03 | 0.20±0.02 |

FIG. 12

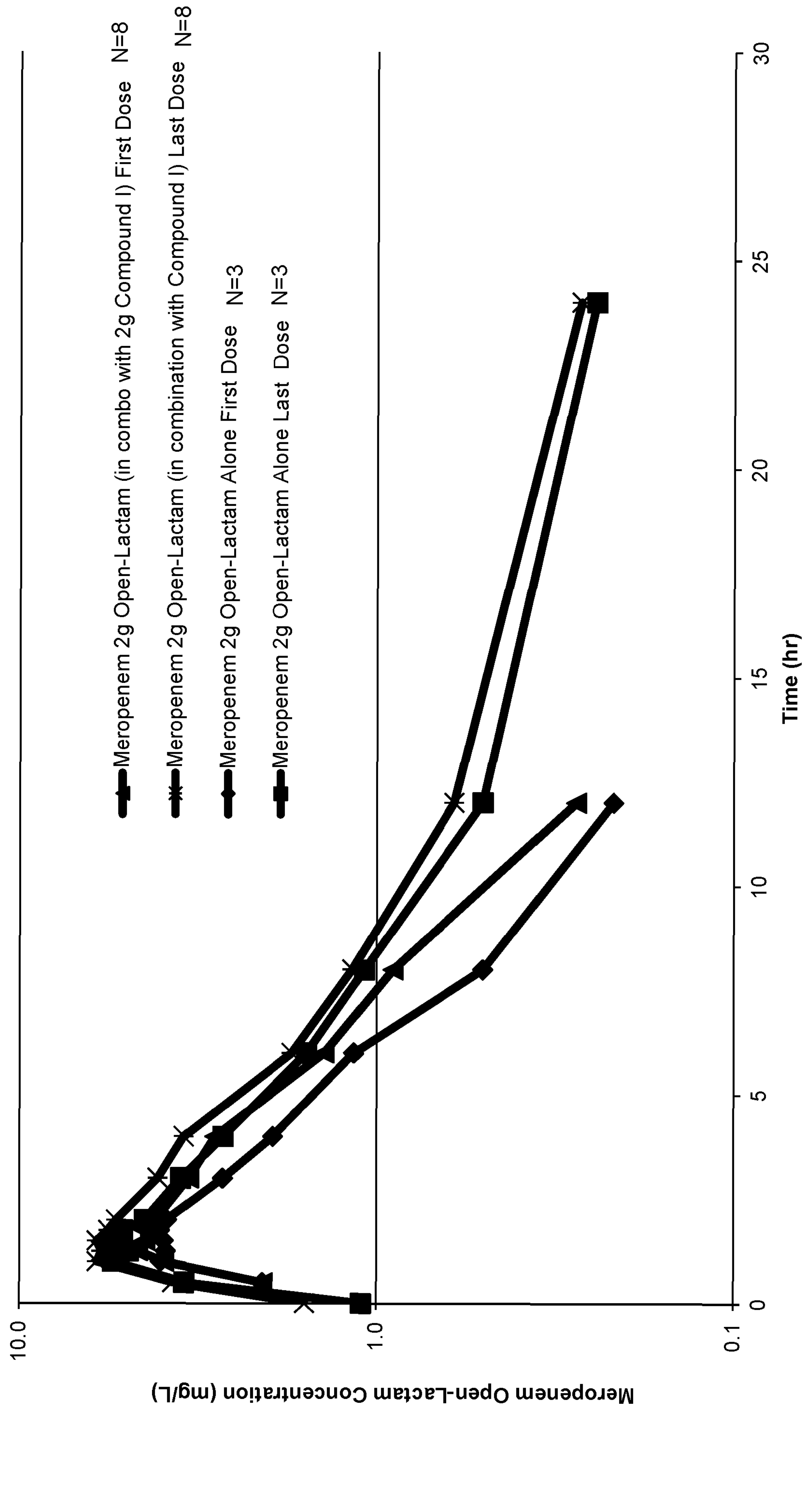
Meropenem 2g Open-Lactam (in combo with 2g Compound I) First Dose N=8
Meropenem 2g Open-Lactam (in combination with Compound I) Last Dose N=8
Meropenem 2g Open-Lactam Alone First Dose N=3
Meropenem 2g Open-Lactam Alone Last Dose N=3
Meropenem Open-Lactam Concentration (mg/L)
10.0
1.0
0.1
0
5
10
15
20
25
30
Time (hr)

FIG. 13

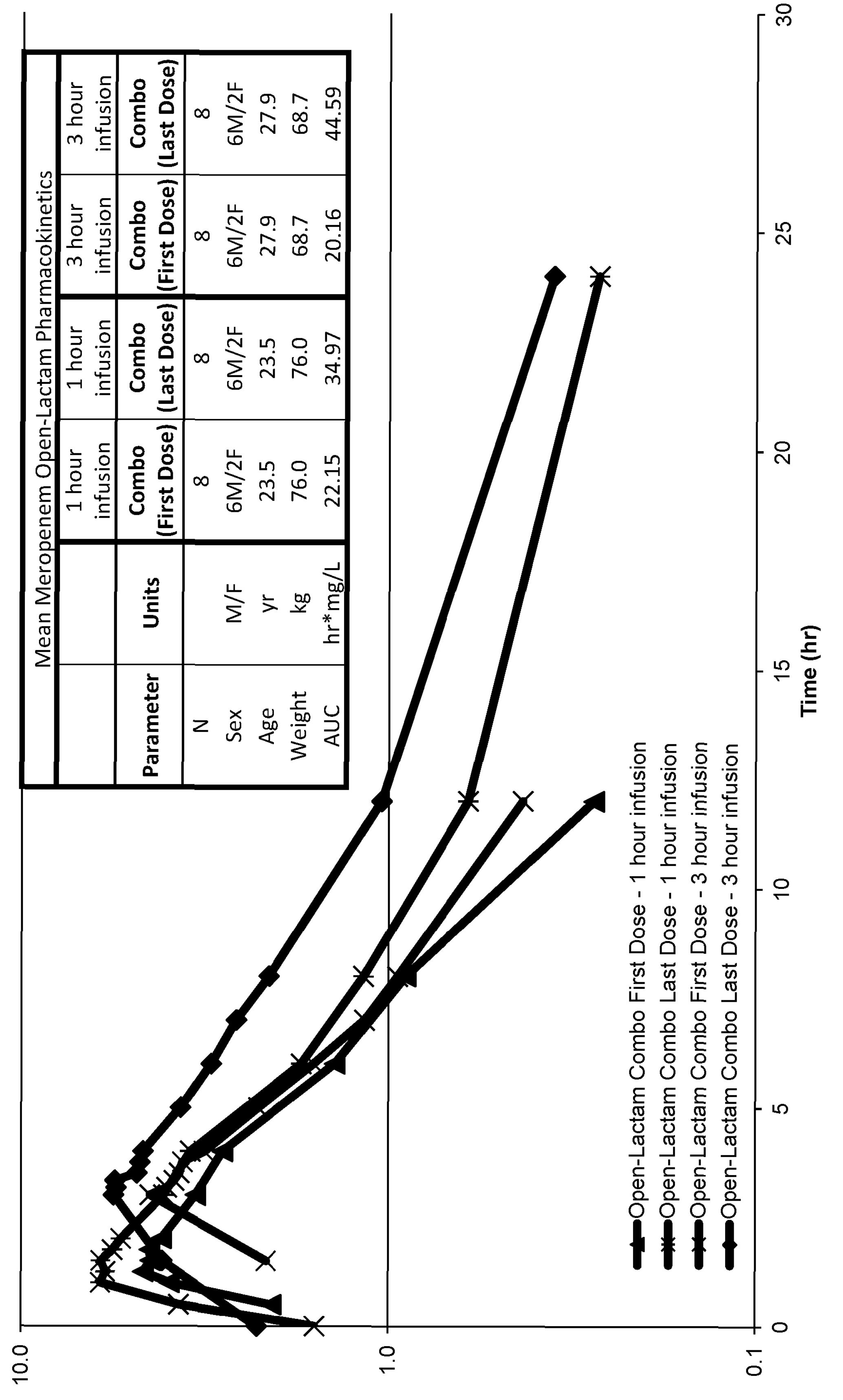


| | | 1 hour infusion | 1 hour infusion | 3 hour infusion | 3 hour infusion |
|---|---|---|---|---|---|
| Parameter | Units | Combo (First Dose) | Combo (Last Dose) | Combo (First Dose) | Combo (Last Dose) |
| N | | 8 | 8 | 8 | 8 |
| Sex | M/F | 6M/2F | 6M/2F | 6M/2F | 6M/2F |
| Age | yr | 23.5 | 23.5 | 27.9 | 27.9 |
| Weight | kg | 76.0 | 76.0 | 68.7 | 68.7 |
| AUC | hr*mg/L | 22.15 | 34.97 | 20.16 | 44.59 |

FIG. 14

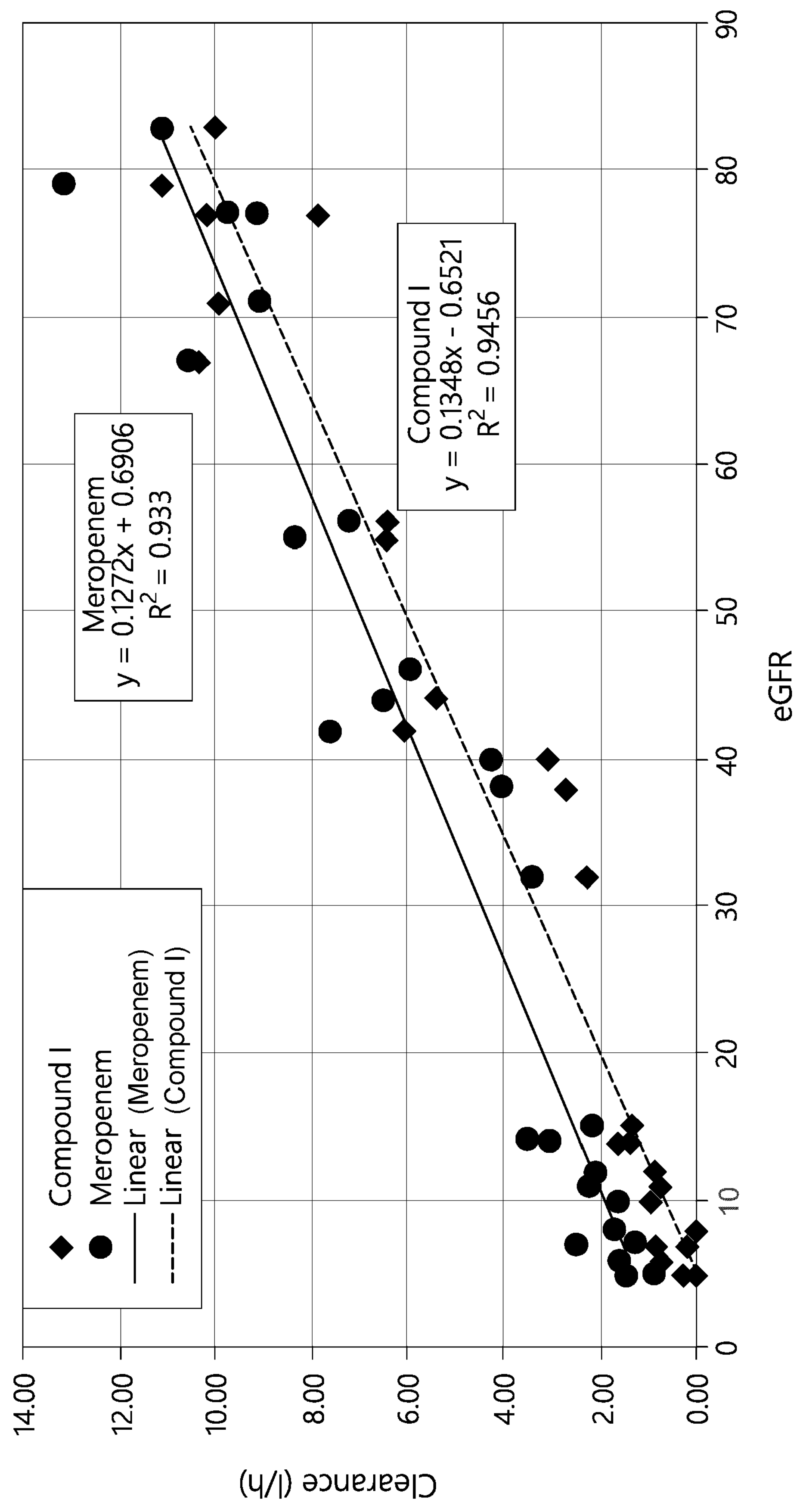
Compound I
Meropenem
Linear (Meropenem)
Linear (Compound I)
Meropenem
y = 0.1272x + 0.6906
$R^2$ = 0.933
Compound I
y = 0.1348x - 0.6521
$R^2$ = 0.9456
Clearance (l/h)
0.00
2.00
4.00
6.00
8.00
10.00
12.00
14.00
eGFR
0
10
20
30
40
50
60
70
80
90

FIG. 15

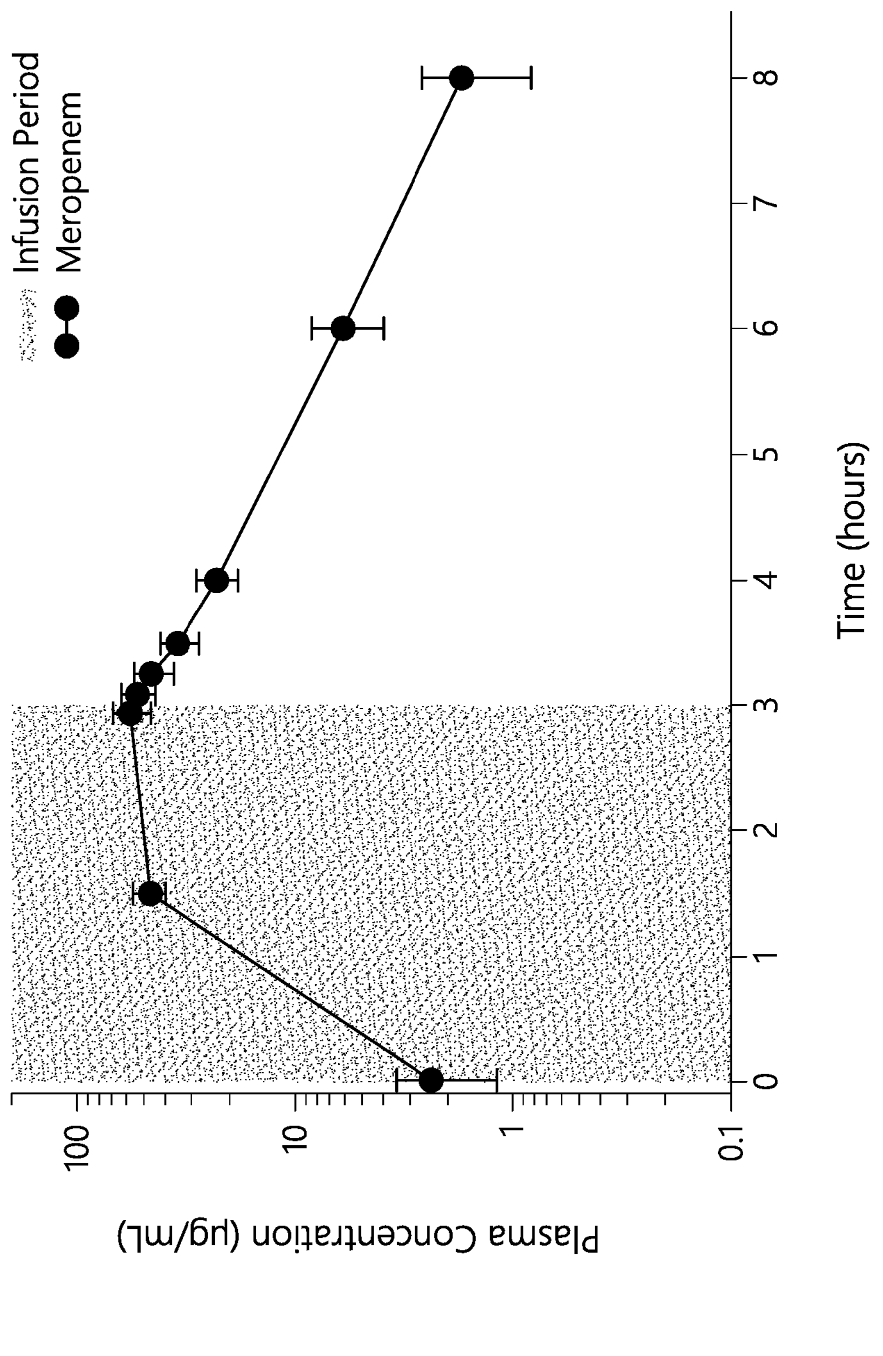
Infusion Period
Meropenem
Plasma Concentration (µg/mL)
100
10
1
0.1
0
1
2
3
4
5
6
7
8
Time (hours)

FIG. 16

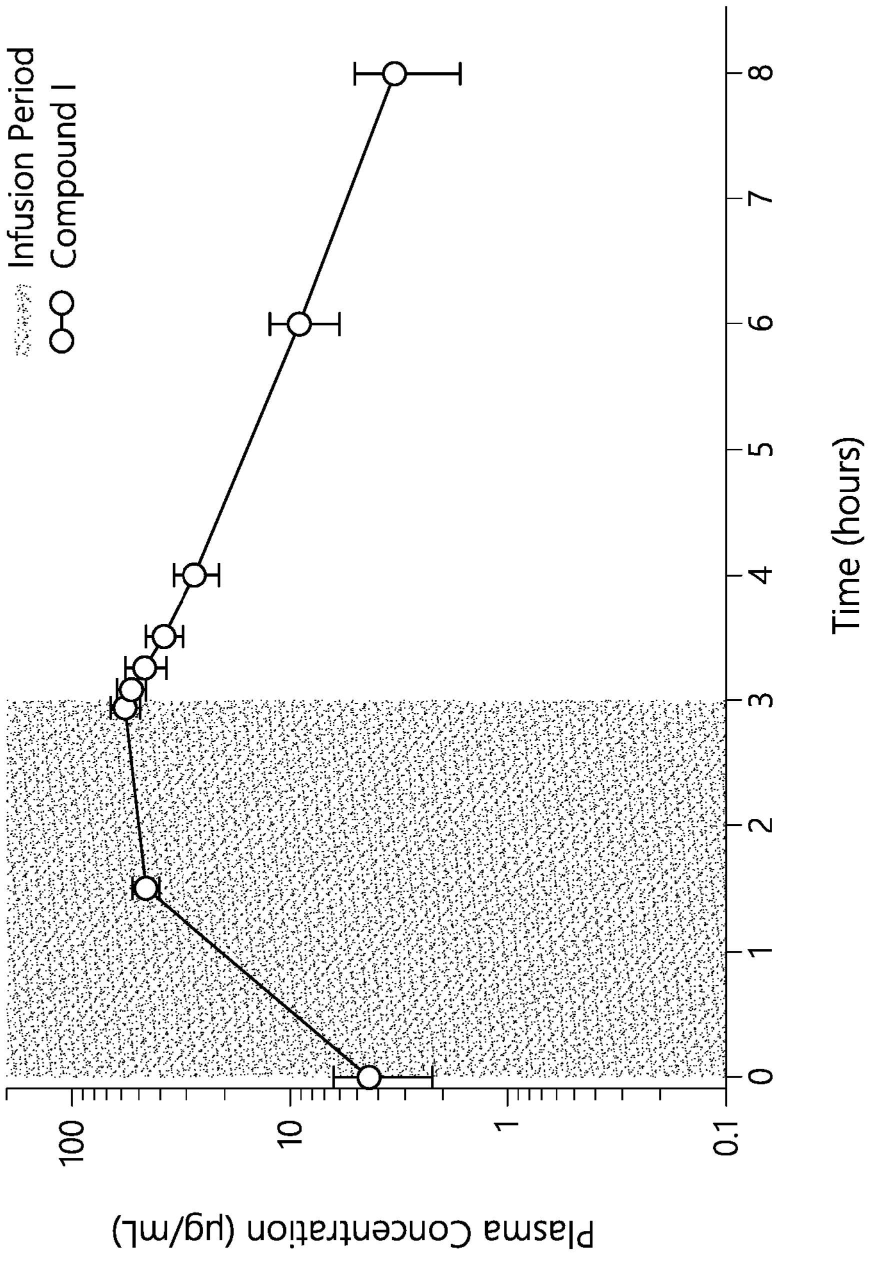
Infusion Period
Compound I
Plasma Concentration (μg/mL)
100
10
1
0.1
0
1
2
3
4
5
6
7
8
Time (hours)

FIG. 17

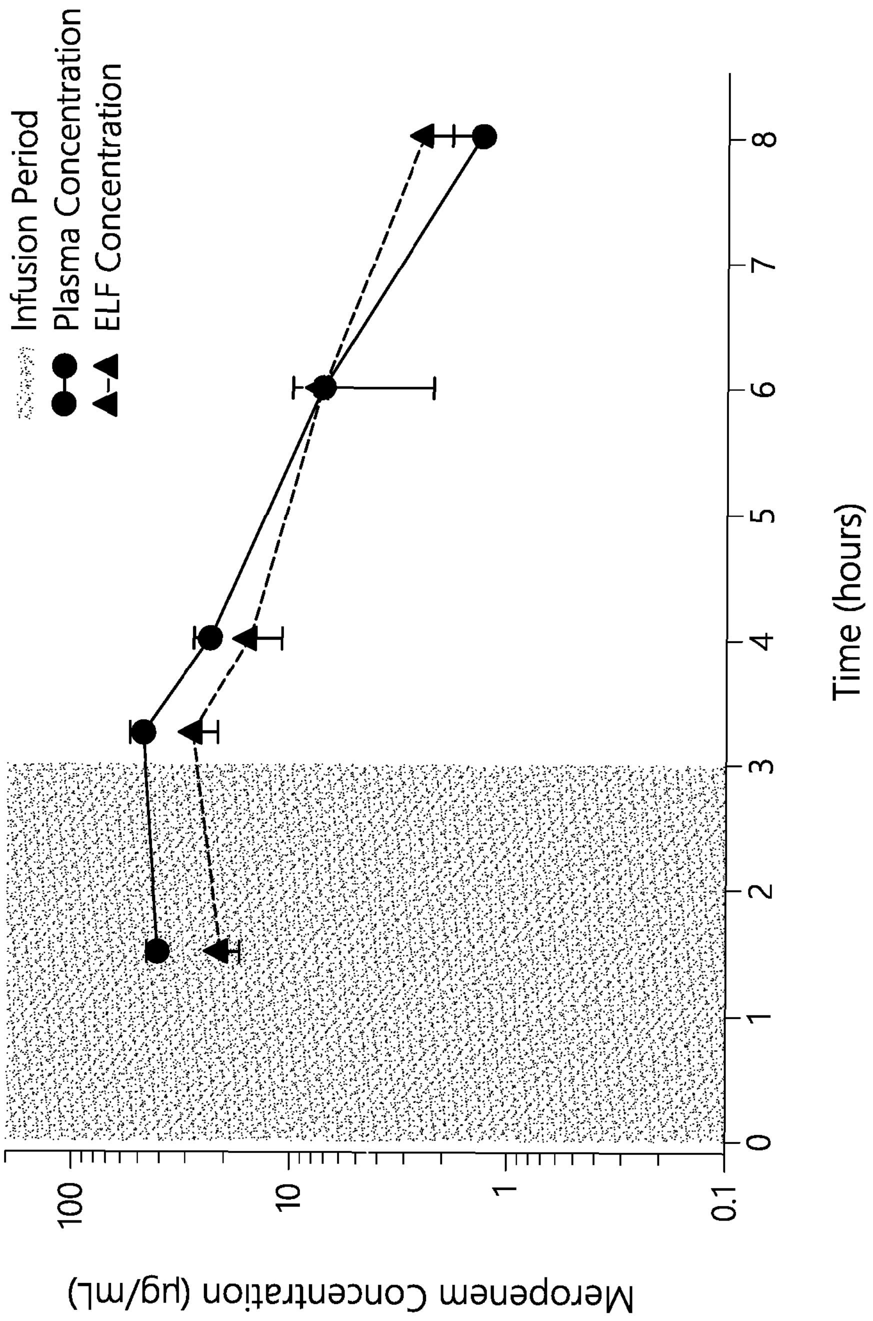
Infusion Period
Plasma Concentration
ELF Concentration
Meropenem Concentration (µg/mL)
100
10
1
0.1
Time (hours)
0
1
2
3
4
5
6
7
8

FIG. 18

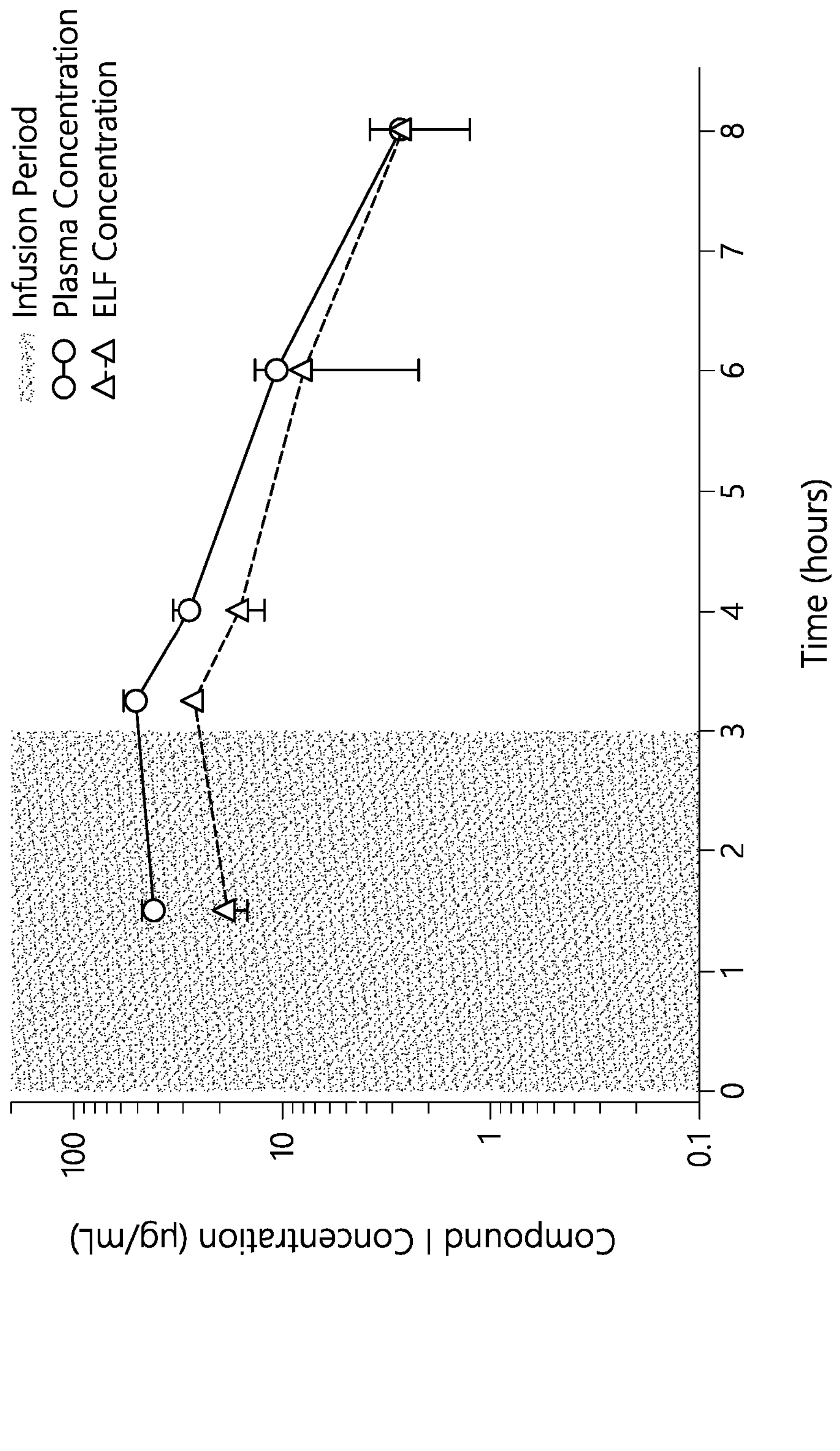
Infusion Period
Plasma Concentration
ELF Concentration
Compound I Concentration (µg/mL)
100
10
1
0.1
0
1
2
3
4
5
6
7
8
Time (hours)

FIG. 19

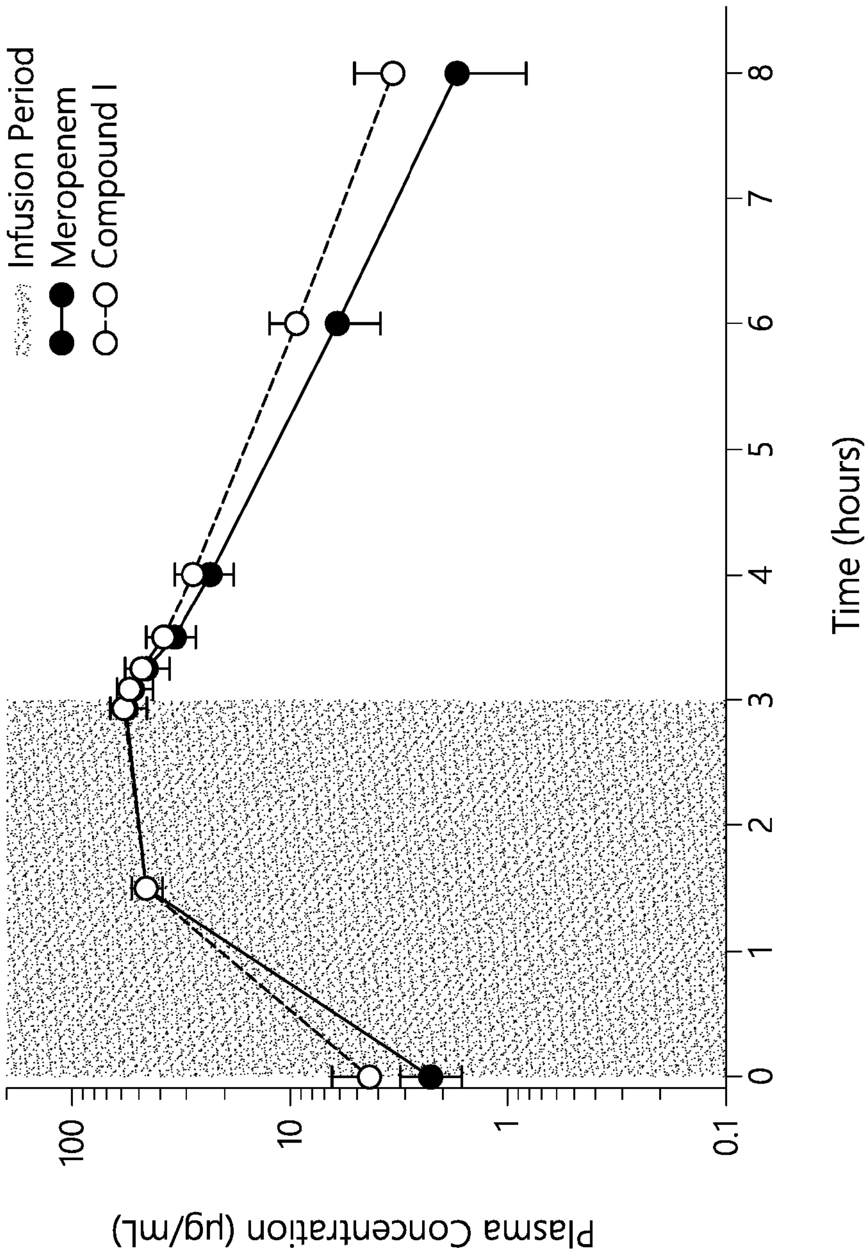
Infusion Period
Meropenem
Compound I
Plasma Concentration (µg/mL)
100
10
1
0.1
Time (hours)
0
1
2
3
4
5
6
7
8

FIG. 20

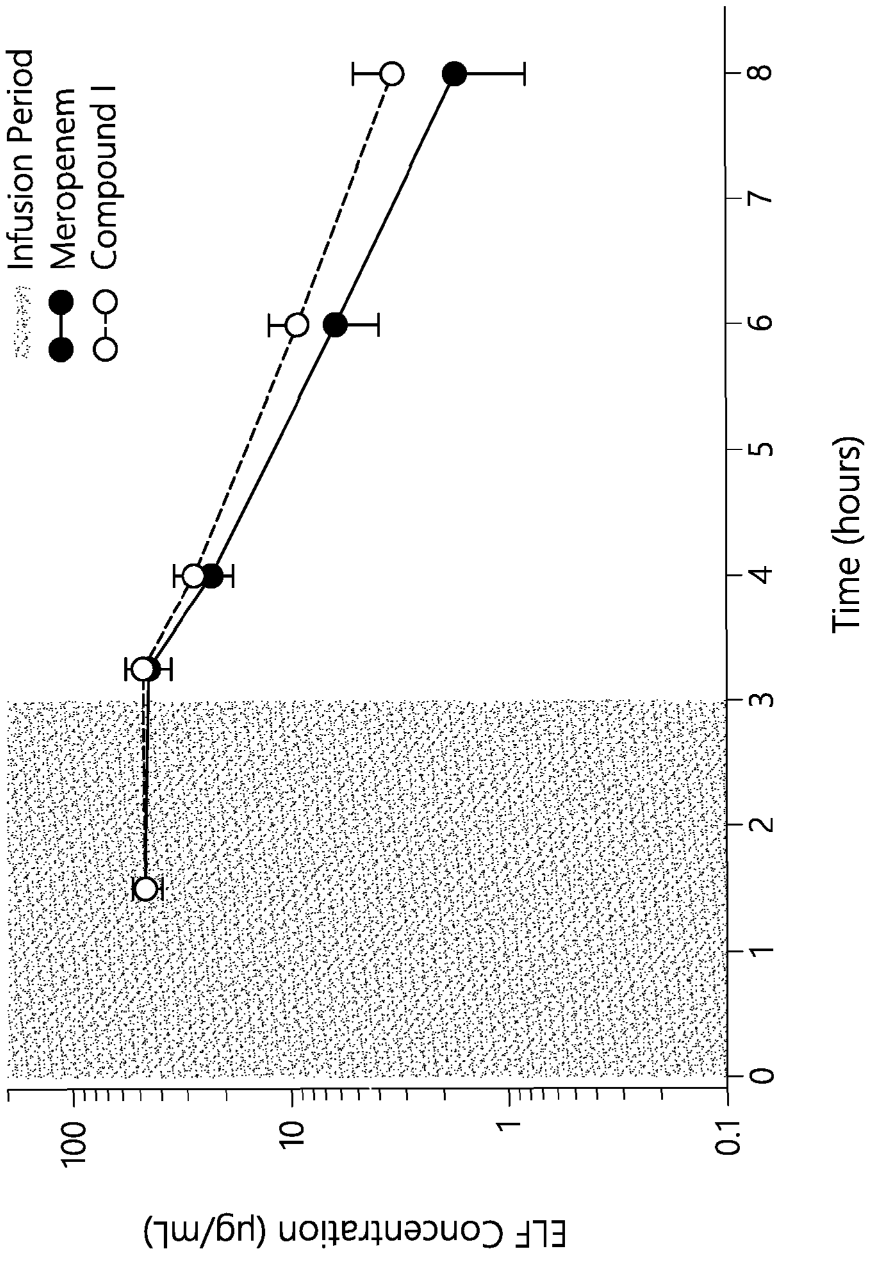
Infusion Period
Meropenem
Compound I
ELF Concentration (μg/mL)
100
10
1
0.1
Time (hours)
0
1
2
3
4
5
6
7
8

FIG. 2I

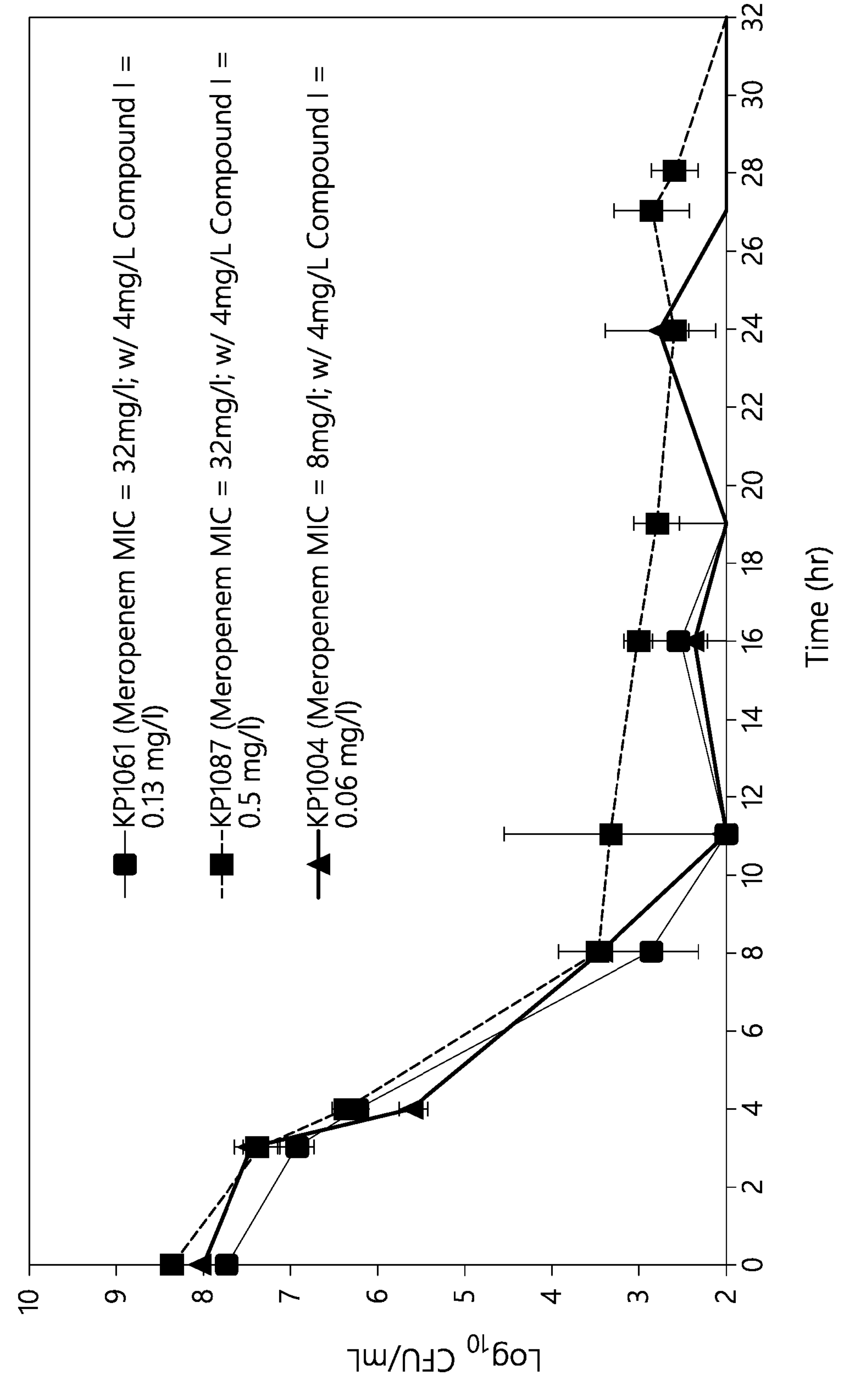
KP1061 (Meropenem MIC = 32mg/l; w/ 4mg/L Compound I = 0.13 mg/l)
KP1087 (Meropenem MIC = 32mg/l; w/ 4mg/L Compound I = 0.5 mg/l)
KP1004 (Meropenem MIC = 8mg/l; w/ 4mg/L Compound I = 0.06 mg/l)
Log10 CFU/mL
10
9
8
7
6
5
4
3
2
Time (hr)
0
2
4
6
8
10
12
14
16
18
20
22
24
26
28
30
32

FIG. 22

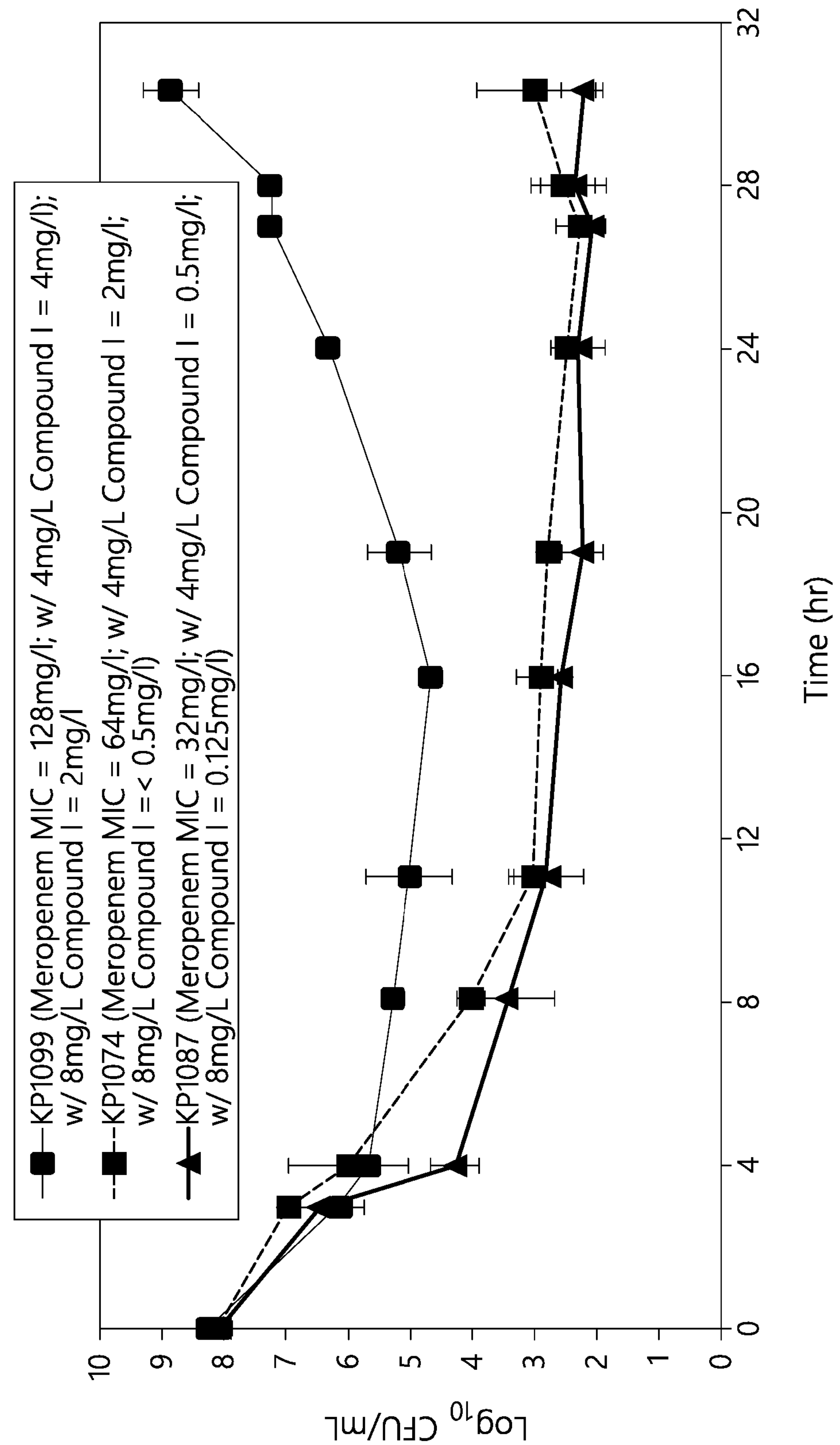
KP1099 (Meropenem MIC = 128mg/l; w/ 4mg/L Compound I = 4mg/l); w/ 8mg/L Compound I = 2mg/l
KP1074 (Meropenem MIC = 64mg/l; w/ 4mg/L Compound I = 2mg/l; w/ 8mg/L Compound I =< 0.5mg/l)
KP1087 (Meropenem MIC = 32mg/l; w/ 4mg/L Compound I = 0.5mg/l; w/ 8mg/L Compound I = 0.125mg/l)
Log10 CFU/mL
Time (hr)
0
1
2
3
4
5
6
7
8
9
10
0
4
8
12
16
20
24
28
32

FIG. 23

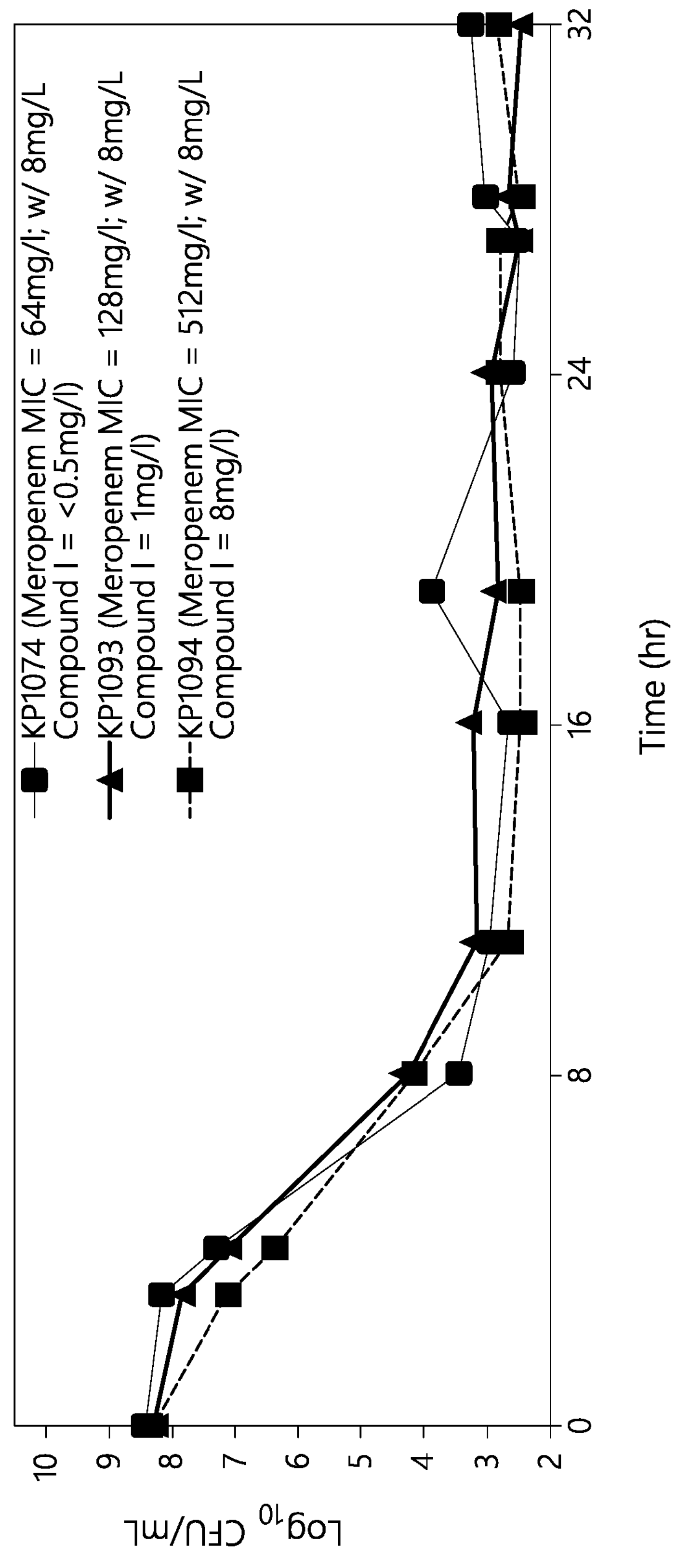
KP1074 (Meropenem MIC = 64mg/l; w/ 8mg/L Compound I = <0.5mg/l)
KP1093 (Meropenem MIC = 128mg/l; w/ 8mg/L Compound I = 1mg/l)
KP1094 (Meropenem MIC = 512mg/l; w/ 8mg/L Compound I = 8mg/l)
Log10 CFU/mL
2 3 4 5 6 7 8 9 10
Time (hr)
0 8 16 24 32

FIG. 24

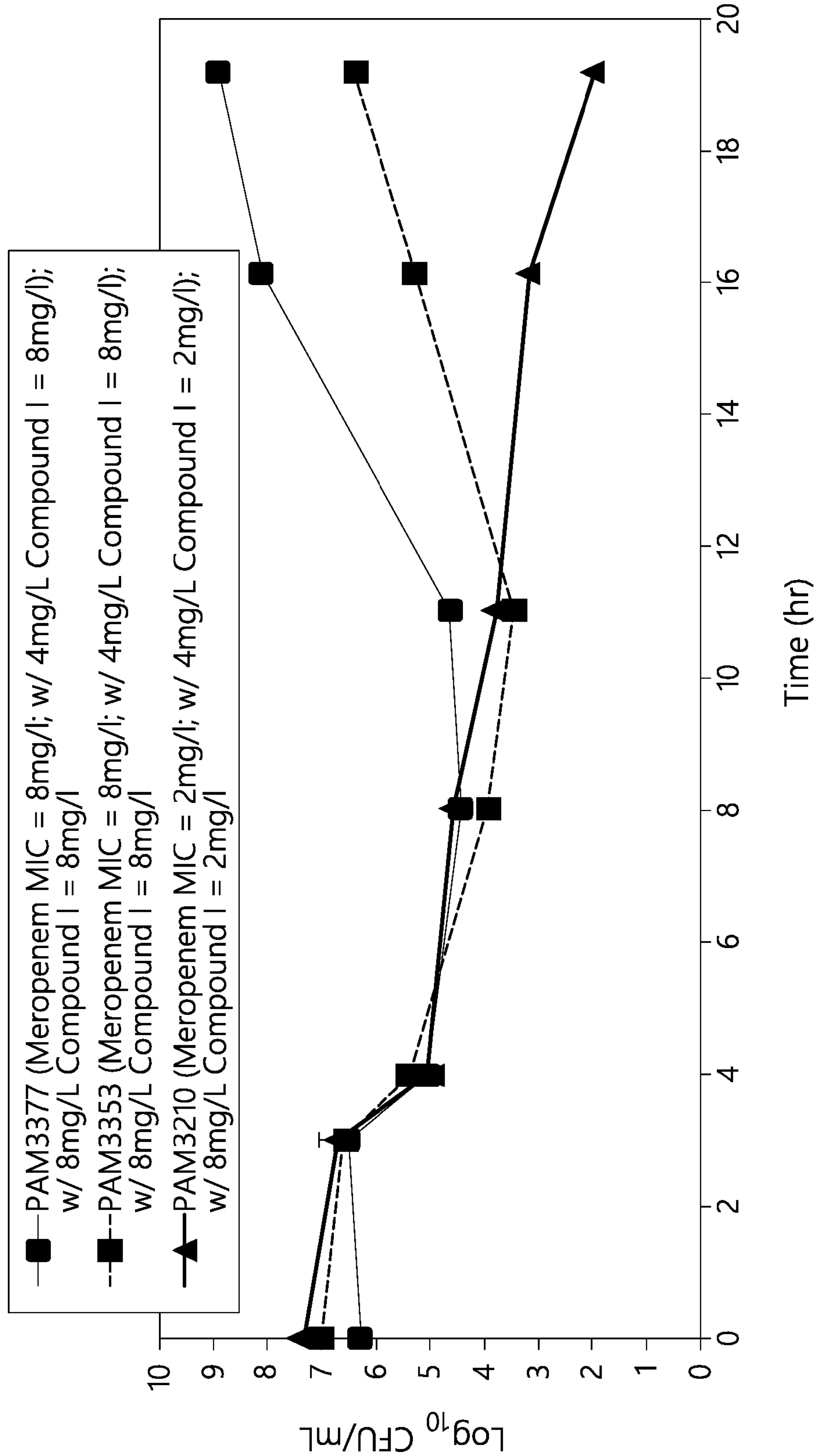
PAM3377 (Meropenem MIC = 8mg/l; w/ 4mg/L Compound I = 8mg/l); w/ 8mg/L Compound I = 8mg/l
PAM3353 (Meropenem MIC = 8mg/l; w/ 4mg/L Compound I = 8mg/l); w/ 8mg/L Compound I = 8mg/l
PAM3210 (Meropenem MIC = 2mg/l; w/ 4mg/L Compound I = 2mg/l); w/ 8mg/L Compound I = 2mg/l
Log10 CFU/mL
0
1
2
3
4
5
6
7
8
9
10
0
2
4
6
8
10
12
14
16
18
20
Time (hr)

FIG. 25

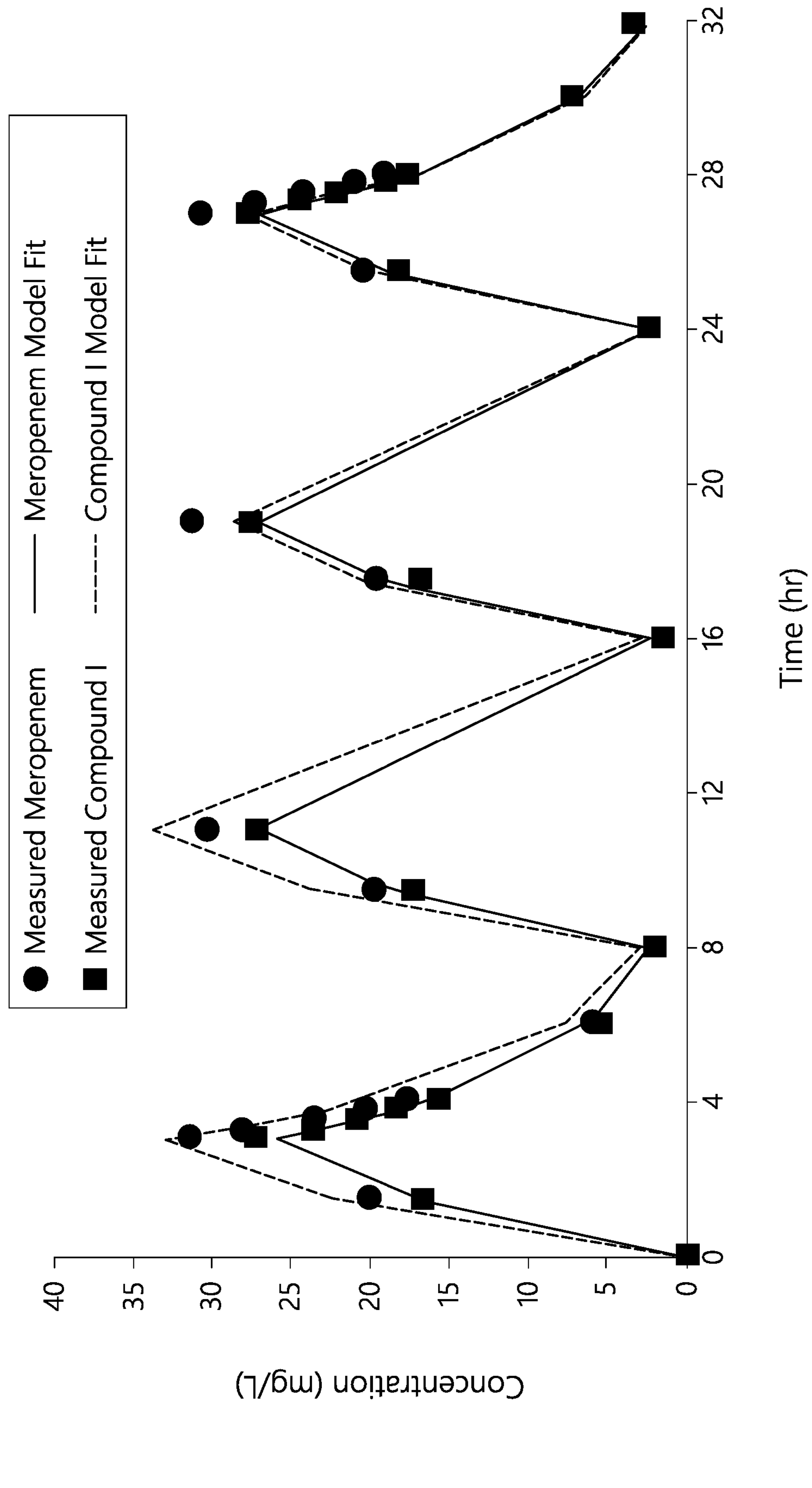
Measured Meropenem
Measured Compound I
Meropenem Model Fit
Compound I Model Fit
Concentration (mg/L)
0
5
10
15
20
25
30
35
40
Time (hr)
0
4
8
12
16
20
24
28
32

FIG. 26

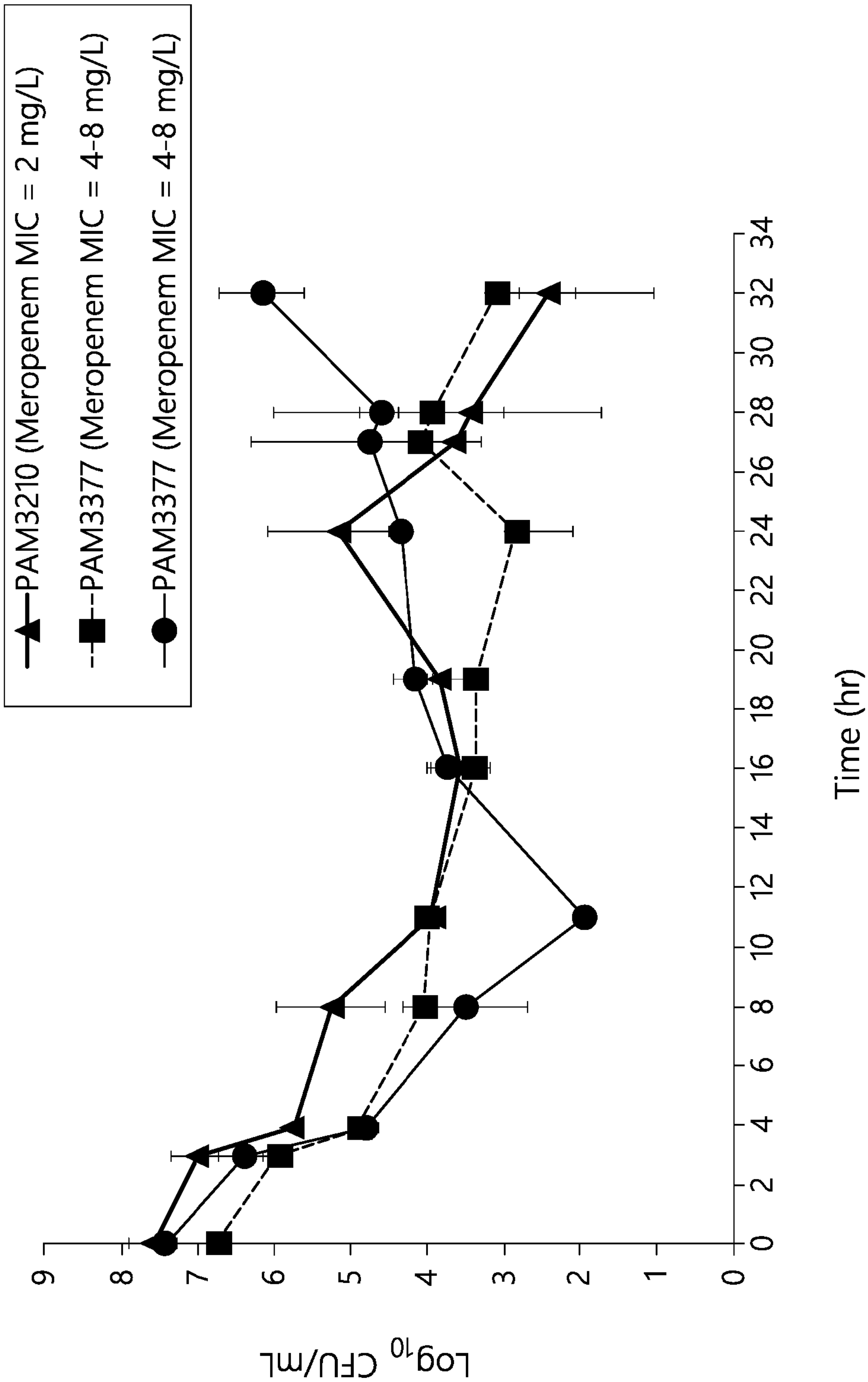
PAM3210 (Meropenem MIC = 2 mg/L)
PAM3377 (Meropenem MIC = 4-8 mg/L)
PAM3377 (Meropenem MIC = 4-8 mg/L)
Log10 CFU/mL
Time (hr)

FIG. 27

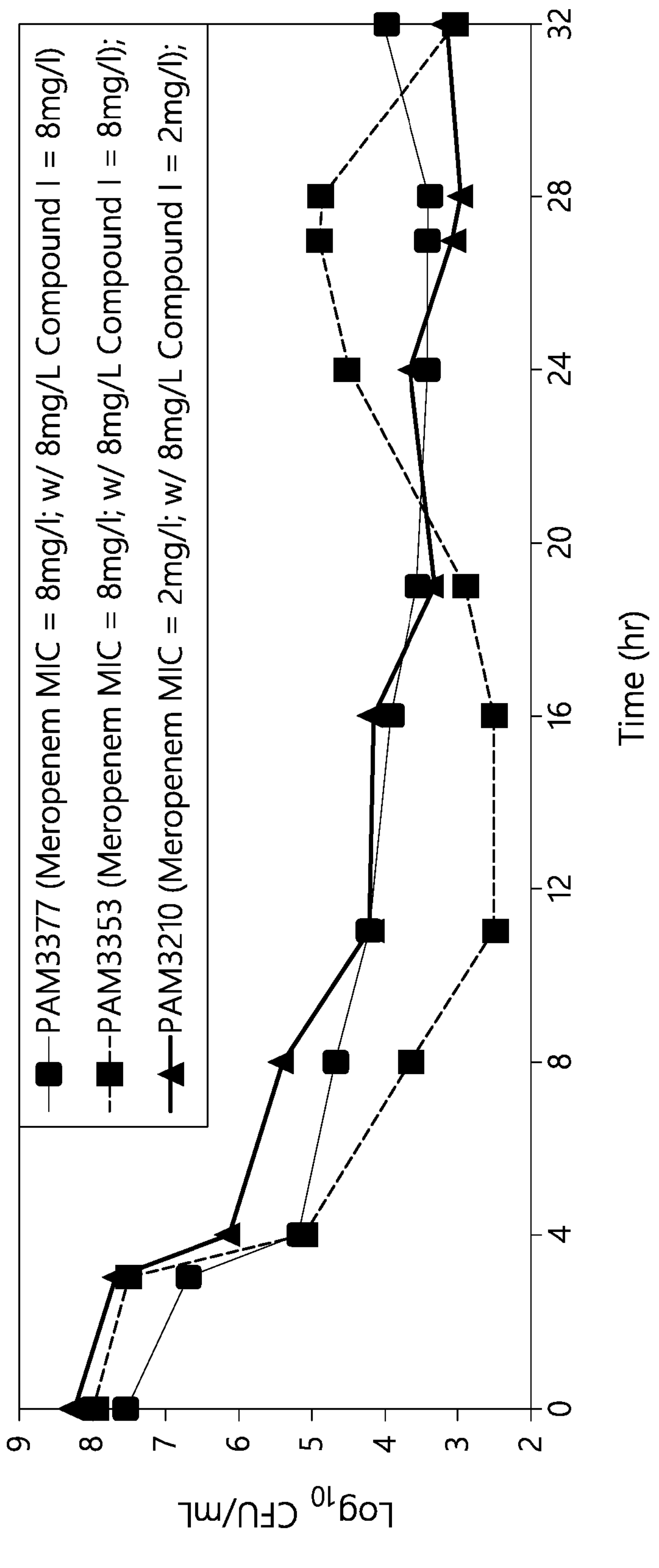
PAM3377 (Meropenem MIC = 8mg/l; w/ 8mg/L Compound I = 8mg/l)
PAM3353 (Meropenem MIC = 8mg/l; w/ 8mg/L Compound I = 8mg/l);
PAM3210 (Meropenem MIC = 2mg/l; w/ 8mg/L Compound I = 2mg/l);
Log10 CFU/mL
9
8
7
6
5
4
3
2
0
4
8
12
16
20
24
28
32
Time (hr)

FIG. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8705297 A, Johnston
- US 8680136 B


### Non-patent literature cited in the description


- **MONTEFOUR, K. et al.** *Crit. Care Nurse,* 2008, vol. 28, 15
- **PEREZ, F. et al.** *Expert Rev. Anti Infect. Ther.,* 2008, vol. 6, 269
- **BOU, G. ; MARTINEZ-BELTRAN.** *J. Antimicrob. Agents Chemother.,* 2000, vol. 40, 428
- **BOU, G. et al.** *J. Antimicrob. Agents Chemother.,* 2000, vol. 44, 1556
- **GRIFFITH D et al.** A Phase 1 Study of the Safety, Tolerability, and Pharmacokinetics of the Beta-lactamase inhibitor RPX7009 Alone, Meropenem Alone, and both in Combination (Carbavance) TID for 7 days in Healthy Adult Subjects. *54th Interscience Conference on Antimicrobial Agents and Chemotherapy (ICAAC); Washington, DC, USA,* 2014
- **KUTI J L et al.** Optimal cefepime and meropenem dosing for ventilator-associated pneumonia patients with reduced renal function: An update to our clinical pathway. *J. of Critical Care,* 2010, vol. 25, 155-156
- **YAGI YUSUKE et al.** Outcome evaluation of an intervention to improve the effective and safe use of meropenem. *J. Clin. Pharm.,* 2014, vol. 36, 648-656
- Goodman and Gilman's: The Pharmacological Basis of Therapeutics. Pergamon Press, 1990
- Remington's The Science and Practice of Pharmacy. Lippincott Williams & Wilkins, 2005
- Modern Pharmaceutics. 2002
- **LIEBERMAN et al.** *Pharmaceutical Dosage Forms: Tablets,* 1989
- **ANSEL.** Introduction to Pharmaceutical Dosage Forms. 2004
- **POWELL et al.** Compendium of Excipients for Parenteral Formulations. *PDA J Pharm Sci and Tech,* 1998, vol. 52, 238-311
- **NEMA et al.** Excipients and Their Role in Approved Injectable Products: Current Usage and Future Directions. *PDA J Pharm Sci and Tech,* 2011, vol. 65, 287-332
- **LODISE T.P. et al.** Penetration of meropenem into epithelial lining fluid of patients with ventilator-associated pneumonia. *Antimicrob Agents Chemother.,* 2011, vol. 55 (4), 1606-10
- **KUTI J. L. et al.** Use of Monte Carlo simulation to design an optimized pharmacodynamics dosing strategy for meropenem. *J Clin Pharmacol.,* 2003, vol. 43 (10), 1116-23